# EUROPEAN PATENT APPLICATION

(11) **EP 2 463 796 A2**
(43) Date of publication of application: **13.06.2012**
(21) Application number: 11192032.8
(22) Date of filing: 06.12.2011
(51) Int. Cl.: G06F 19/00

(54) **Health care device, method and graphical user interface for health care**

(30) Priority: 07.12.2010 US 420425 P; 11.03.2011 KR 20110021865
(71) Applicant: Samsung Electronics Co., Ltd., Suwon-si, Gyeonggi-do 442-742 (KR)
(72) Inventor: Lee, Sung Hwa, Gyeonggi-Do (KR)
(74) Representative: D'Halleweyn, Nele Veerle Trees Gertrudis

(57) **Abstract**

A health care management method and device which display information on a health state of a patient related to first bio-information and second bio-information of the patient, display information that requests the patient to obtain a second measurement of the first bio-information or the second bio-information based on a schedule, and display a bio-information measurement view to assist the patient in obtaining the second measurement.

## Description

### BACKGROUND

### 1. Field

Methods and apparatuses consistent with the exemplary embodiments relate to a health care device that assists users in maintaining their health, a health care method using the same, and a graphical user interface using the same.

### 2. Description of the Related Art

A home health care, or u-health care service, is a service through which patients measure bio-information using various measurement devices at home, without visiting hospitals, and receive medical advice based on the measurement results in a remote fashion.

For patients to receive medical advice in a remote fashion, health care devices, which are terminals for patients, collect bio-information of the patients at home and transmit the collected information to a hospital server or a care server, such a health care center.

Many users of the health care devices are persons of advanced age who require long-term health care. Conventional health care devices are designed to have user interfaces, through which basic functions of the health care devices may be easily used. In consideration of characteristics of a health care service which must be continuously provided, however, such user interfaces designed based on ease of use may not motivate patients who wish to continuously measure bio-information.

### SUMMARY

Exemplary embodiments provide a user interface to provide information on a health state of a patient, a health care device that provides information on the health state of the patient in response to user input through the user interface, a health care method of the health care device, and a computer-readable storage medium that stores a computer program to execute the health care method.

In accordance with an aspect of an exemplary embodiment, there is provided a health care management method including: displaying, by a health care device, information on a health state of a patient related to a first measurement of first bio-information and a first measurement of second bio-information of the patient, displaying information that requests the patient to obtain a second measurement of one of the first bio-information and the second bio-information based on a schedule of the patient in which the second measurement of the first bio-information and the second measurement of the second bio-information are to be obtained, and displaying a bio-information measurement view to acquire the second measurement through measuring the one of the first bio-information and the second bio-information in response to a user input to obtain the second measurement.

The displaying information on the health state may include determining an image that represents the patient based on at least one of the first measurement value of the first bio-information and the first measurement value of the second bio-information and displaying the determined image.

The displaying information on the health state may include identifying a section of a display of the health care device in which the first measurement value of the first bio-information is displayed and displaying information on the health state corresponding to the first measurement value of the first bio-information in the identified section. The health care method may further include determining a motivation message to obtain the second measurement and displaying the determined motivation message. The health care method may further include determining a plurality of motivation messages and selecting the motivation message from among the plurality of motivation messages based on at least one of header information of the motivation message, a transmission mode of the motivation message, a sender of the motivation message, and a content of the motivation message.

The motivation message may include a plurality of motivation messages, and the determining the motivation message may include selecting at least one motivation message from among the plurality of motivation messages based on the health state of the patient.

The health care method may further include receiving a new message and displaying the new message so that at least a portion of a region where the new message is displayed includes at least a portion of a region where the motivation message is displayed.

The health care method may further include displaying a user interface connected to an external device external to the health care device and providing information on the health state of the patient stored in the external in response to user input through the user interface.

The health care method may further include displaying information on the schedule to measure at least one of the first bio-information and the second bio-information of the patient or a dosage schedule of the patient and displaying the schedule to measure the at least one of the first bio-information and the second bio-information of the patient or the dosage schedule of the patient in response to user input through a user interface.

The displaying the schedule to measure the at least one of the first bio-information and the second bio-information of the patient or the dosage schedule of the patient may include, if the schedule comprises a plurality of schedules, providing a visual effect to at least one of the schedules so that the schedules are displayed according to priority.

The health care method may further include displaying information on a message related to the health state of the patient and displaying the message related to the health state of the patient in response to user input through a user interface.

In accordance with an aspect of another exemplary embodiment, there is provided a health care management method including: displaying, by a health care device, information on a health state of a patient related to a first measurement of first bio-information and a first measurement of second bio-information of the patient, displaying a motivation message for the patient to obtain a second measurement of one of the first bio-information and the second bio-information, and displaying a bio-information measurement view to acquire the second measurement through measuring the one of the first bio-information and the second bio-information in response to a user input to obtain the second measurement.

In accordance with aspect of another exemplary embodiment, there is provided a health care device including a display that displays information on a health state of a patient related to a first measurement of first bio-information and a first measurement of second bio-information of a patient and information that requests the patient to obtain a second measurement of one of the first bio-information and the second bio-information based on a schedule of the patient in which the second measurement of the first bio-information and the second measurement of the second bio-information are to be obtained, an input unit that accepts user input to obtain the second measurement, and a processor that controls the display to display a bio-information measurement view to acquire the second measurement through measuring the one of the first bio-information and the second bio-information in response to the user input.

The display may display an image that represents the patient decided based on at least one of the first measurement value of the first bio-information and the first measurement value of the second bio-information.

The processor may determine a motivation message to obtain the second measurement and controls the display to display the determined motivation message.

The processor may determine a plurality of motivation messages and selects the motivation message from among the plurality of messages based on of at least one of header information of the motivation message, a transmission mode of the motivation message, a sender of the motivation message, and a content of the motivation message.

The display may display a user interface connected to an external device external to the health care device, and the processor may control the display to display information on the health state of the patient stored in the external in response to user input through the user interface.

The display may display information on the schedule to measure at least one of the first bio-information and the second bio-information of the patient or a dosage schedule of the patient, and the processor may control the display to display the schedule to measure the at least one of the first bio-information and the second bio-information of the patient or the dosage schedule of the patient in response to user input through a user interface.

The display may display information on a message to care for health of the patient, and the processor may control the display to display the message to care for health of the patient in response to user input through a user interface.

In accordance with an aspect of another exemplary embodiment, there is provided a computer-readable recording medium contains a program to execute a health care management method including displaying information on a health state of a patient related to a first measurement of first bio-information and a first measurement of second bio-information of the patient, displaying information that requests the patient to obtain a second measurement of one of the first bio-information and the second bio-information based on a schedule of the patient in which the second measurement of the first bio-information and the second measurement of the second bio-information are to be obtained, and displaying a bio-information measurement view to acquire the second measurement through measuring the one of the first bio-information and the second bio-information in response to a user input to obtain the second measurement.

### BRIEF DESCRIPTION OF THE DRAWINGS

These and/or other aspects of the exemplary embodiments will become apparent and more readily appreciated from the following description of the exemplary embodiments, taken in conjunction with reference to the accompanying drawings of which:
FIG. 1 is a block diagram showing a health care system including a health care device according to an exemplary embodiment;
FIG. 2A shows a user selection view of the device according to an exemplary embodiment;
FIG. 2B is a flow chart showing the operation of a user in the user selection view of the device according to an exemplary embodiment;
FIG. 2C shows a user selection view of the device according to another embodiment;
FIG. 3 shows a password input view of the device according to an exemplary embodiment;
FIG. 4A shows a main view of the device according to an exemplary embodiment;
FIG. 4B is a flow chart showing the operation of a user in the main view of the device according to an exemplary embodiment ;
FIG. 5 shows a main view of the device according to an exemplary embodiment;
FIG. 6A shows a message list view of the device according to an exemplary embodiment;
FIG. 6B shows a message list view of the device according to an exemplary embodiment;
FIG. 7 shows a message detail view according to an exemplary embodiment;
FIG. 8 shows message measurement value views according to an exemplary embodiment;
FIG. 9 shows a schedule list view according to an exemplary embodiment;
FIG. 10 shows a schedule setting view according to an exemplary embodiment;
FIG. 11 shows a day schedule setting view according to an exemplary embodiment;
FIG. 12 shows a measurement schedule setting view according to an exemplary embodiment;
FIG. 13A shows a schedule addition view according to an exemplary embodiment;
FIG. 13B shows a schedule addition view according to an exemplary embodiment ;
FIG. 14 shows a schedule deletion view according to an exemplary embodiment;
FIG. 15 shows a measurement schedule request view according to an exemplary embodiment;
FIG. 16 shows a schedule deletion view according to an exemplary embodiment ;
FIG. 17 shows a dosage schedule view according to an exemplary embodiment;
FIG. 18A shows a schedule addition view according to an exemplary embodiment;
FIG. 18B shows a schedule addition view according to an exemplary embodiment ;
FIG. 19 shows a schedule deletion view according to an exemplary embodiment;
FIG. 20 shows a dosage schedule request view according to an exemplary embodiment;
FIGS. 21A to 21F show bio-information measurement views according to an exemplary embodiment;
FIGS. 22A to 22C show immediate measurement views according to an exemplary embodiment;
FIGS. 23A to 23F show measurement guidance views according to an exemplary embodiment;
FIGS. 24A to 24F show measurement guidance views according to an exemplary embodiment;
FIGS. 25A to 25E show measurement guidance views according to an exemplary embodiment;
FIGS. 26A to 26D show measurement result views according to an exemplary embodiment;
FIGS. 27A to 27C show untransmitted measurement value list views according to an exemplary embodiment;
FIGS. 28A to 28C show untransmitted measurement value views according to an exemplary embodiment;
FIGS. 29A to 29C show recent measurement value list views according to an exemplary embodiment;
FIGS. 30A to 30C show recent measurement value views according to an exemplary embodiment;
FIG. 31 shows an environment setting view according to an exemplary embodiment;
FIGS. 32A to 32C show general setting views according to an exemplary embodiment;
FIGS. 33A to 34 show individual user information setting views according to an exemplary embodiment;
FIGS. 35A to 35C show individual user information setting views according to an exemplary embodiment ;
FIGS. 36A and 36B show password setting views according to an exemplary embodiment;
FIG. 37 shows a measurement value transmission mode setting view according to an exemplary embodiment;
FIG. 38 shows an automatic area code setting view according to an exemplary embodiment;
FIG. 39 shows a transmission restriction list setting view according to an exemplary embodiment;
FIGS. 40A to 40C show reception restriction list setting views according to an exemplary embodiment;
FIGS. 41A and 41B show administrator password input views according to an exemplary embodiment;
FIGS. 42A and 42B show administrator mode views according to an exemplary embodiment;
FIGS. 43A and 43B show user password setting views according to an exemplary embodiment;
FIGS. 44A and 44B show bio-information measurement sensor registration views according to an exemplary embodiment;
FIGS. 45A and 45B show bio-information measurement sensor change views according to an exemplary embodiment ; and
FIGS. 46A to 46W show health care notification views according to an exemplary embodiment.

### DETAILED DESCRIPTION

Reference will now be made in detail to exemplary embodiments , which are illustrated in the accompanying drawings, wherein like reference numerals refer to like elements throughout.

In connection with terms 'embodiment', 'example', 'aspect', 'illustration', etc. used in this specification, these terms must not be interpreted that an aspect or design is better or more advantageous than other aspects or designs.

Terms 'unit', 'component', 'module', 'system', 'interface', etc., which will be used hereinafter, generally mean computer-related entities. For example, such terms may mean hardware, such as a processor or circuit, software and combinations of hardware and software.

Also, a term 'or' does not mean 'exclusive or' but 'inclusive or'. That is, expression 'x uses a or b' means any one of the natural inclusive permutations as far as it is not differently mentioned or is not clear from context.

Also, a singular expression ('a' or 'an') used in this specification and claims must be interpreted to generally mean 'one or more' as far as it is not differently mentioned or is not clear from context.

Also, a term 'and/or' used in this specification must be understood to indicate and include all possible combinations of one or more items selected from among listed related items.

Also, term 'includes' and/or 'including' means that a corresponding characteristic, process, operation, module, element and/or component exists. However, such term must be understood not to exclude existence or addition of one or more other characteristics, processes, operations, modules, elements, components and/or groups thereof.

Also, terms 'first', 'second', etc. may be used to describe various elements in this specification; however, such elements are not limited by such terms. Such terms are used merely to distinguish two or more elements from one another and thus must not be interpreted as indicating order or priority.

Also, an expression 'a process of doing y in response to x' used in this specification may be interpreted as meaning 'a process of responding to x and a process of doing y'. One or more processes may be included between 'a process of responding to x' and 'a process of doing y'.

Hereinafter, embodiments of a health care device, a health care method for the health care device and a user interface for the health care device will be described.

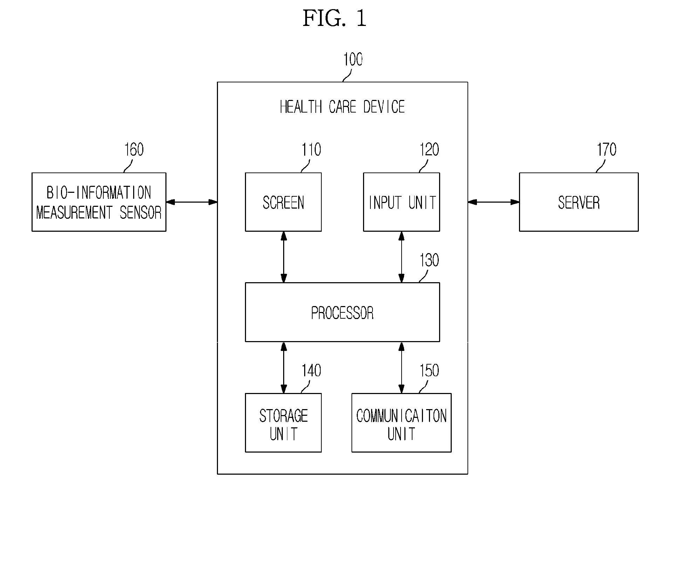

FIG. 1 is a block diagram showing a health care system including a health care device 100 according to an exemplary embodiment.

Referring to FIG. 1, a health care system may include a health care device 100, a bio-information measurement sensor 160, and a server 170. However, the health care system may include more or fewer elements than those disclosed herein. Two or more elements may be coupled, or the health care system may have other structures or arrangements.

The health care system may provide a health care service to a user using the above-mentioned elements. For example, the user may access the health care system through a log-in process. While accessing the health care system, the user may measure his/her bio-information using a bio-information measurement sensor 160. The health care device 100 may transmit a bio-information measurement value to the server 170, receive advice as to the measurement value from a doctor, and provide the advice to the user through a screen 110.

The health care device 100, which provides a health care service to a user, may be called a patient terminal or gateway.

An element of the device 100 includes one more signal processing circuits or customized semiconductors, which are embodied using hardware, software or combinations thereof. For example, the device 100 may include at least one selected from among a display integrated device, a set top box type device including an IP-TV and cable TV, a smart phone type device, a mobile phone type device, a WiBro terminal type device, Wi-Fi wireless router type device, a PC type device including a tablet PC, and a medical equipment integrated device.

The device 100 may provide various kinds of health care services to a user. The user may measure various kinds of bio-information using the bio-information measurement sensor 160 according to the provided health care services. For example, the device 100 may receive a bio-information measurement value from the bio-information measurement sensor 160 and transmit the bio-information measurement value to the server 160 so that a doctor may provide medical advice. At this time, the user of the device 100 is a patient who suffers from various diseases. The user may be a person who does not suffer from any disease but uses the device 100 for health care or to operate the device 100. In the following description, a patient will be cited as an example of the user.

A health care service is related to patient health care provided to a user through the device 100 and may include information necessary for patient health care. Information necessary for patient health care may include, without being limited to, at least one selected from among information on patient health, kinds of bio-information to be measured, a history (date, time, frequency, etc.) of measuring bio-information, measurement time zone of bio-information, a method of using the bio-information measurement sensor 160, kinds of medicines for a patient to take, a dosage (amount, frequency, timing, etc.) to take medicines, dosage time zone of medicines, a method of taking medicines, a menu for a patient including a recommended food list and a list of food avoid, and an exercise plan to improve patient health.

The bio-information measurement sensor 160 measures bio-information of a user. The bio-information measurement sensor 160 may include, without being limited to, at least one selected from among a sphygmomanometer, a blood glucose meter, a blood tester, an electronic stethoscope, a peak flow meter, an exercise quantity meter, a menu analyzer, a lifestyle analyzer, a camera, scales, a body fat analyzer, medical imaging equipment and a urine analyzer. The bio-information is medical information on a user. The bio-information may include, without being limited to, blood pressure, blood glucose, exercise quantity, blood, peak flow, lifestyle, a menu, weight, body fat and dosage time. The user may acquire a bio-information measurement value using the bio-information measurement sensor 160. The bio-information measurement sensor 160 may transmit the acquired bio-information measurement value to the device 100.

The server 170 receives information necessary for user health care, such as user bio-information, and provides such information to a doctor so that the doctor cares for the health of the user in a remote fashion. For example, the server 170 may be a hospital server or a care server, such a health care center. The server 170 may include a database of users who use a health care service. The user database may include, without being limited to, personal information of users, user diagnosis information, health care items prepared based on diagnosis information, bio-information measurement materials, and analysis results of bio-information measurement materials.

Also, the server 170 may diagnose a user and provide a prescription using a clinical decision support system (CDSS). CDSS is a system useful in medical decision-making. CDSS may accurately analyze user bio-information and provide a prescription in place of diagnosis by a doctor. For example, CDSS may provide customized guidance on a per value basis of bio-information measurement values or may determine an emergency situation on a per patient disease basis and provide a procedure or detailed guidance in such an emergency situation.

Referring to FIG. 1, the device 100 may include a display screen 110, an input unit 120, a processor 130, a storage unit 140 and a communication unit 150.

The screen 110 may provide information on a health care service to achieve patient health care. A plasma display panel (PDP), an electronic paper display (EPD), a liquid crystal display (LCD), a light emitting polymer display (LPD), an organic light-emitting diode (OLED) or an active-matrix organic light-emitting diode (AMOLED) may be used as the screen 110. Also, other well-known technology or technology under development may be applied to the screen 110.

The input unit 120 allows a user to perform an input operation. For example, the input unit 120 may include a touch sensor, a mouse, a keyboard, a joystick, a button and a slider switch. The touch sensor is a device to sense user touch input. Capacitive technology, resistive technology, infrared technology and surface acoustic wave technology may be applied to the touch sensor. Also, other well-known technology or technology under development may be applied to the touch sensor. The touch sensor may be coupled to the screen 110 to operate as a touch screen. The touch screen may transmit information on a user gesture input through the screen 110 to the processor 130. The user gesture information may include a click gesture, a scroll gesture, a flick gesture, a drag gesture and a swipe gesture.

The storage unit 140 may store programs necessary to provide a health care service and content corresponding to digital images or various other data. The storage unit 140 may include a volatile memory and a nonvolatile memory. For example, the storage unit 140 may include, without being limited to, a high-speed random access memory (RAM), magnetic disk, SRAM, DRAM and read-only memory (ROM). Also, the storage unit 140 may be detachably mounted in the device 100. For example, the storage unit 140 may include, without being limited to, a compact flash (CF) card, secure digital (SD) card, smart media (SM) card, multimedia card (MMC) or memory stick. Also, the storage unit 140 may be provided outside the device 100 to transmit or receive data to or from the device 100 in a wired or wireless fashion.

The communication unit 150 may receive information on a health care service from the server 170. For example, the communication unit 150 may receive advice necessary for patient care or diagnosis based on a bio-information measurement value from a doctor. Communication between the communication unit 150 and the server 170 may be performed using Wi-Fi, CDMA, a wired or wireless communication over a network, WiBro, 3G, 4G or a public switched telephone network (PSTN), to which, however, the communication mode is not limited.

The communication unit 150 may communicate with the bio-information measurement sensor 160 to receive a bio-information measurement value measured by the bio-information measurement sensor 160 or transmit the received measurement value to the server 170. Communication between the communication unit 150 and the bio-information measurement sensor 160 may be performed using Bluetooth, infrared communication, Wi-Fi, CDMA, a wired/wireless LAN, ZigBee, a serial port and USB, to which, however, the communication mode is not limited.

The processor 130 may control overall operation of elements constituting the device 100 and provide information on a health care service through the screen 110. For example, the processor 130 may control the supply of power to the elements to perform an initialization process. Upon completion of the initialization process, the processor 130 may control flow of signals to provide a health care service according to an exemplary embodiment. Views provided by the device 100, which will be described below, may be provided or controlled by the processor 130.

The device 100 may communicate with a patient via a user interface. The user interface is a medium that provides temporary or continuous access to achieve communication between the user and the device 100. The user interface may include an imaginary user interface or a physical user interface. For example, the imaginary user interface may include text or graphs provided on a screen. Also, the physical user interface may include a physical button and a mouse. In another example, the user interface may include a sensor to sense images or sound. For example, the device 100 may analyze facial expressions or sound waves of a user using the sensor and provide information based on the analysis result.

Hereinafter, exemplary embodiments of user interfaces and views provided through the screen 110 of the device 100 will be described. The views may be changed on the screen 110. The views may be provided as popup or overlaid views. Consequently, several views may be provided on a single screen 110.

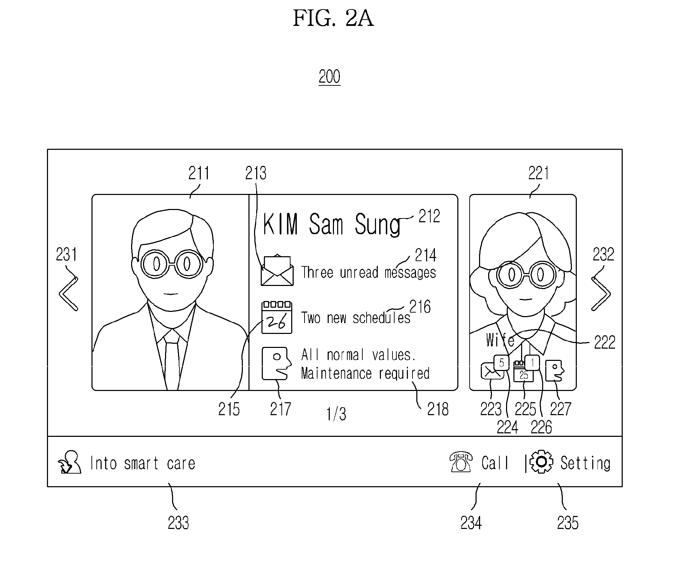

FIG. 2A shows a user selection view 200 of the device 100 according to an exemplary embodiment. In this exemplary embodiment, the user selection view 200 may include the following elements or a subset or superset thereof.
- An image 211 representing a first patient and name 212 of the first patient
- A message icon 213 related to a health state of the first patient and information 214 on the message icon
- A schedule icon 215 to measure bio-information of the first patient and information 216 on the schedule icon
- An icon 217 regarding a health state of the first patient and information 218 on the health state
- An image 221 representing a second patient and name 222 of the second patient
- A message icon 223 related to a health state of the second patient and abbreviated information 224 on the message icon
- A schedule icon 225 to measure bio-information of the second patient and abbreviated information 226 on the schedule icon
- An icon 227 regarding a health state of the second patient
- User interfaces 231 and 232 to display information on the health state of the second patient
- A user interface 233 to access the health care system
- A user interface 234 for telephone calls
- A user interface 235 to display an environment setting view

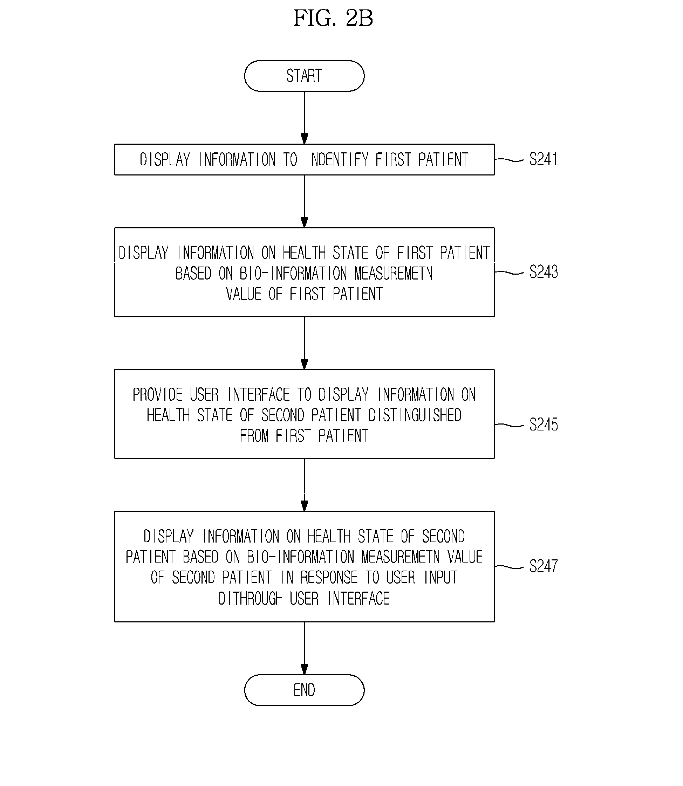

FIG. 2B is a flow chart showing the operation of a user in the user selection view 200 of the device 100 according to an exemplary embodiment.

In this exemplary embodiment, the device 100 may display information (for example, 211 and 212) to identify the first patient (S241). The information (for example, 211 and 212) to identify the first patient may include an image (for example, 211) representing the first patient expressed based on a bio-information measurement value of the first patient.

The device 100 may display information (for example, 218) on a health state of the first patient based on the bio-information measurement value of the first patient (S243). The information (for example, 218) on the health state may be provided as, without being limited to, text (for example, 218), graphs, figures, video or sound.

The device 100 may provide user interfaces (for example, 231 and 232) to display information on a health state of the second patient distinguished from the first patient (S245). Also, the device 100 may sense user input through the user interfaces (for example, 231 and 232). For example, the device 100 may sense user input to select at least a portion of the region where the information (for example 221) to identify the second patient. In this case, the user input may be a gesture to contact some of the user interfaces (for example, 231 and 232) or to release such contact. Also, as a user interface, the device 100 may include a medium to accept user input to select a portion of the view (for example, 200) without visual information related to a user interface. For example, the user input may be a gesture to contact a portion of the view 200, perform movement in contact, and release such contact. For example, flick, touch and move, swipe, drag and drop, etc. may be used as the gesture.

The device 100 may display information on a health state of the second patient based on a bio-information measurement value of the second patient in response to the user input through the user interfaces (for example, 200, 231, 232) (S247).

In this exemplary embodiment, the device 100 may identify a specific section having the bio-information measurement value of the first patient and display information (for example, 218) on the health state corresponding to the specific section based on the identification result. The device 100 may display at least a portion of the image (for example, 211) representing the first patient. For example, if the bio-information measurement value is within a normal range, the device 100 may display information (for example, 218) indicating that the health state of the patient is normal. If the bio-information measurement value deviates from the normal range, the device 100 may display information indicating that the health state of the patient is abnormal. In another example, the device 100 may transmit the bio-information measurement value of the first patient to the server 170, and receive and display information (for example, 218) on a health state related to the bio-information from the server 170. The specific section may be a section set based on the result of patient diagnosis performed by a doctor. The specific section may be one of the sections divided considering a range of bio-information measurement values which may be acquired from a patient.

In this exemplary embodiment, the visual effect of the region where information (for example, 211) to identify the first patient is displayed may be different from that of the region where information (for example, 221) to identify the second patient is displayed. For example, the region where information (for example, 211) to identify the first patient is displayed may have brightness or resolution different from that of the region where information (for example, 221) to identify the second patient is displayed. Also, the size of the region where information (for example, 211) to identify the first patient is displayed may be different from that of the region where information (for example, 221) to identify the second patient is displayed. Also, the region where information (for example, 211) to identify the first patient is displayed may be activated, and the region where information (for example, 221) to identify the second patient is displayed may be inactivated.

In this exemplary embodiment, the device 100 may display at least one selected from among abbreviated information (for example, 224) on a message of the second patient, abbreviated information (for example, 226) on a schedule of the second patient and abbreviated information on a health state of the second patient. For example, if information on a message of the second patient indicates that "there are five unread messages," the abbreviated information on the message of the second patient may be "5". If information on a schedule of the second patient indicates that "there are two new schedules," the abbreviated information on the schedule of the second patient may be "2".

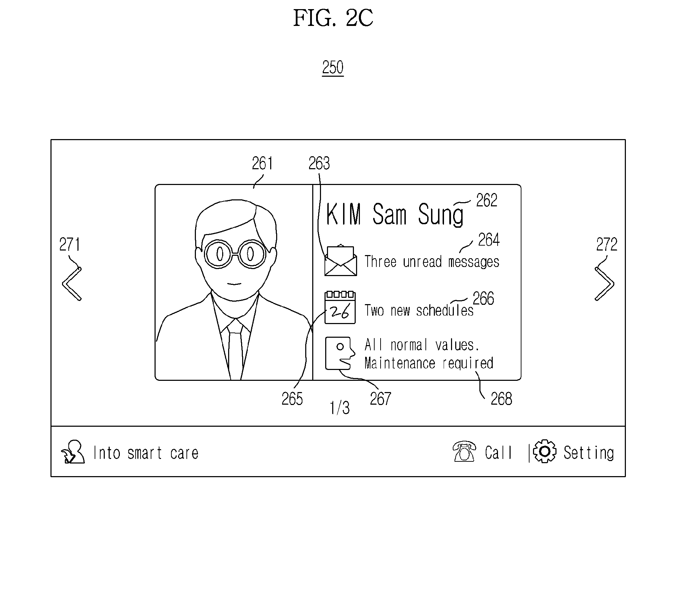

FIG. 2C shows a user selection view 250 of the device 100 according to another exemplary embodiment. In this exemplary embodiment, the user selection view 250 may include the following elements or a subset or superset thereof.
- An image 261 representing a first patient and name 262 of the first patient
- A message icon 263 related to a health state of the first patient and information 264 on the message icon
- A schedule icon 265 to measure bio-information of the first patient and information 266 on the schedule icon
- An icon 267 regarding a health state of the first patient and information 268 on the health state
- User interfaces 271 and 272 to display information on a health state of a second patient

In this embodiment, the device 100 may display information (for example, 261 and 262) to identify the first patient. Also, the device 100 may display information (for example, 268) on a health state of the first patient related to bio-information of the first patient. The device 100 may provide user interfaces (for example, 271 and 272) to display information on a health state of the second patient. The device 100 may display information on a health state of the second patient based on a bio-information measurement value of the second patient in response to user input through the user interfaces (for example, 271 and 272).

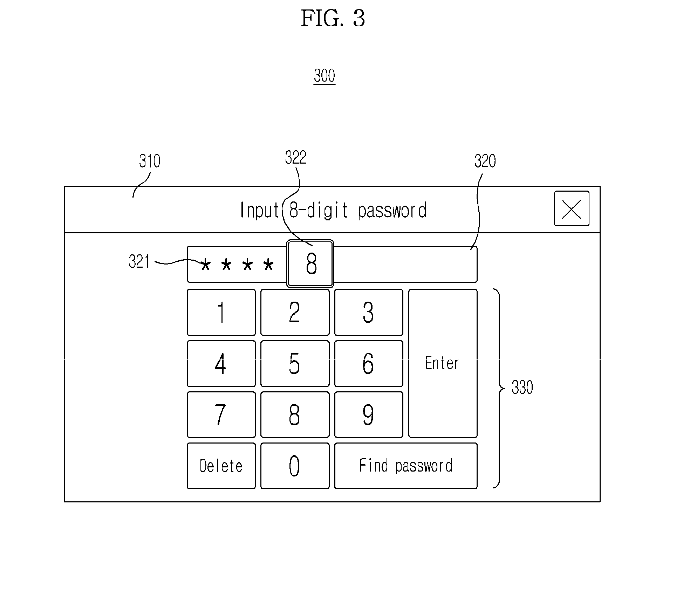

FIG. 3 shows a password input view 300 of the device 100 according to an exemplary embodiment.

In this embodiment, the device 100 may display the password input view 300 in response to user input to select a user interface (for example, 233, which is a region where information (for example, 211, 212, 261 and 262) to identify the first patient is displayed) to access the health care system in the user selection views (for example, 200 and 250).

The device 100 may display a password input request message (for example, 310), a password display part (for example, 320) and a user interface (for example, 330) to input a password. The device 100 may accept a password input by the user through the user interface (for example, 330). At this time, visual feedback (for example, 321 and 322) related to at least one figure of the password input by the user may be provided to the password display part (for example, 320).

The device 100 may confirm whether the password input by the user coincides with a password stored in the storage unit 140 or the server 170 corresponding to the user. In this exemplary embodiment, if the password input by the user coincides with the stored password corresponding to the user, the device 100 may provide a view based on user access to the health care system.

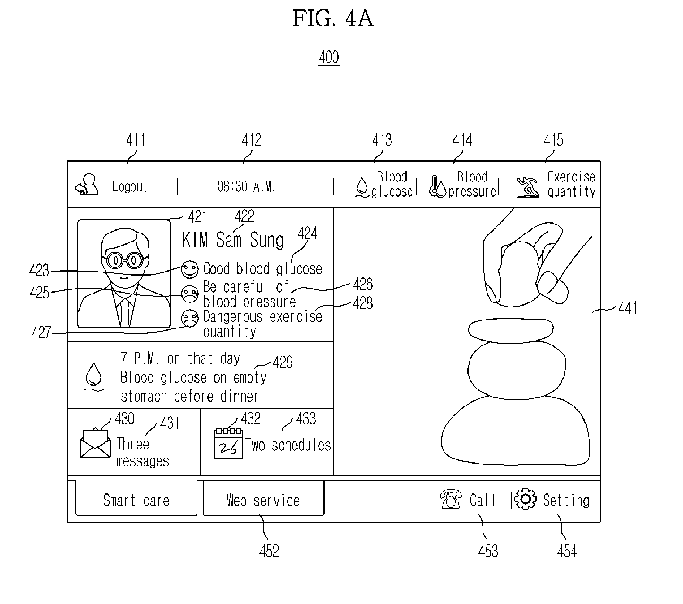

FIG. 4A shows a main view 400 of the device 100 according to an exemplary embodiment. In this exemplary embodiment, the main view 400 may include the following elements or a subset or superset thereof.
- An image 421 representing a patient and name 422 of the patient
- Images 423, 425 and 427 corresponding to a bio-information measurement value of the patient
- Text information 424, 426 and 428 on the bio-information measurement value of the patient
- Information 429 to request remeasurement of bio-information of the patient
- A message icon 430 related to a health state of the patient and information 431 on the message icon
- A schedule icon 432 to measure bio-information of the patient and information 433 on the schedule icon
- A logout icon 411 to log out of the system and a time 412 to display a current time
- A user interface 413 to acquire a blood glucose measurement value of the patient
- A user interface 414 to acquire a blood pressure measurement value of the patient
- A user interface 415 to acquire an exercise quantity measurement value of the patient
- A motivation message 441
- A device outside the device 100 (for example, a user interface 452 to access the server 170)
- A user interface 453 for telephone calls
- A user interface 454 to display an environment setting view

In this embodiment, the device 100 may display the main view 400 in response to user input to select a user interface (for example, 233, which is a region where information (for example, 211, 212, 261 and 262) to identify the first patient is displayed) to access the health care system in the user selection views (for example, 200 and 250). Alternatively, the device 100 may display the main view 400 if a user correctly inputs a password through the password input view 300.

In this embodiment, if the user logins in to the main view 400, the device 100 may provide the recent login records (for example, date, time, used items, health state, schedule execution state, etc.) through a new view (for example, popup) or sound. Also, the device 100 may output various sounds according to the health state or schedule execution state of the user. For example, for a user who has a good health state or schedule execution state, the device 100 may provide a voice to praise the user, such as ' Your current health state is good,' or 'Your steady health care habit is a good example to other patients,' or quiet or soft music. On the other hand, for a user who has a bad health state or schedule execution state, the device 100 may provide a voice to encourage the user, such as 'More effort will improve your health. Pull yourself together!,' or cheerful or loud music.

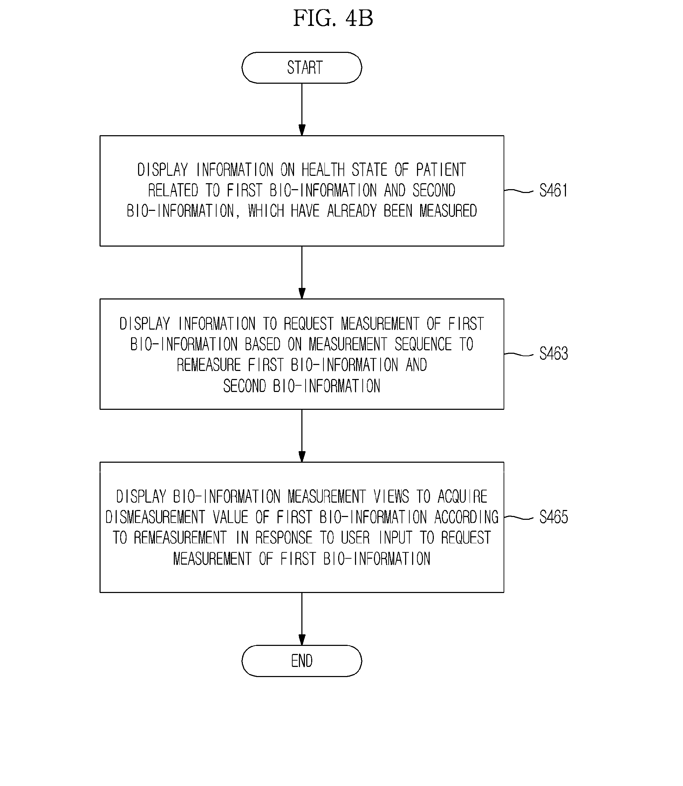

FIG. 4B is a flow chart showing the operation of a user in the main view 400 of the device 100 according to the embodiment.

In this embodiment, the device 100 may display information (for example, 421 and 423 to 428) on a health state of the patient related to first bio-information and second bio-information, which have already been measured (S461).

The information (for example, 421 and 423 to 428) on the health state of the patient may include an image (for example, 421) representing the patient. The image (for example, 421) representing the patient may include a facial expression, avatar or picture of the patient reflecting the health state of the patient. The device 100 may decide an image (for example, 421) representing the patient based on at least one bio-information measurement value, which has already been measured, and display the decided image (for example, 421).

The device 100 may display information (for example, 429) to request measurement of the first bio-information based on a measurement sequence to remeasure the first bio-information and the second bio-information (S463). For example, if time to remeasure the first bio-information from the current time 412 is earlier than time to remeasure the second bio-information from the current time 412, the device 100 may display information (for example, 429) to request measurement of the first bio-information.

The device 100 may display bio-information measurement views (for example, 2100, 2130, 2140, 2150, 2160 and 2170) to acquire a measurement value of the first bio-information according to remeasurement in response to user input to select user interfaces (for example, 413 to 415) to measure the first bio-information (S465).

In this exemplary embodiment, the device 100 may identify a specific section including the measured bio-information measurement value as information (for example, 421 and 423 to 428) on a health state of the patient and display information on the health state corresponding to the specific section based on the identification result. For example, the device 100 may display at least one image (for example, 423, 425 and 427) of the stored images as an image corresponding to the specific section. At this time, at least one image (for example, 423, 425 and 427) may be different from each other depending upon the bio-information or the bio-information measurement value. For example, at least one image (for example, 423, 425 and 427) may have different colors depending upon kinds of bio-information. Also, at least one image (for example, 423, 425 and 427) may have different shapes depending upon kinds of bio-information.

In this exemplary embodiment, the device 100 may display a motivation message (for example, 441) to remeasure at least one piece of bio-information. The motivation message (for example, 441) is a message related to a health state of the patient. The motivation message (for example, 441) may include various shapes, such as images, audio, video and text. The motivation message (for example, 441) may be acquired from the device 100 or the storage unit of the server 170. The motivation message (for example, 441) may be acquired through a communication network (for example, the Internet or a wired or wireless telephone network) connected to an external device.

The motivation message (for example, 441) may be identified from among a plurality of messages. The motivation message (for example, 441) may be identified from among a plurality of messages using at least one piece of information selected from among header information of the message, a message transmission mode, a sender of the message and the content of the message. For example, the device 100 may identify a message having a specific ID or tag related to the motivation message among a plurality of messages as the motivation message (for example, 441). The device may parse the content of the message and, if the message has content related to patient motivation, identify the message as the motivation message (for example, 441). Of the messages received through the Internet and the telephone network, the message received through the telephone network may be identified as a motivation message (for example, 441) depending upon a network form to transmit the motivation message. A message transmitted from an acquaintance of the patient (for example, a family member or doctor of the patient) may be identified as the motivation message (for example, 441).

If a plurality of motivation messages (for example, 441) is present, the device may identify at least one motivation message among the motivation messages (for example, 441) based on a health state of the patient related to at least one piece of measured bio-information. For example, if a health state of the patient related to blood glucose is not good, the device may identify a motivation message related to blood glucose based on the content or sender of the motivation message (for example, 441).

In this exemplary embodiment, the device 100 may provide a motivation message (for example, 441) to remeasure at least one piece of bio-information as a voice.

The device 100 may receive a motivation message to remeasure at least one piece of bio-information and decide a voice to output the received motivation message. For example, the device 100 may select one from among a plurality of voices based on information on a sender who has sent the motivation message. For example, the information on the sender may include age and/or sex of the sender. As an example, ages may be classified into under teenage, teenage, twenties, thirties, forties, fifties, sixties, and seventies or more. Also, sexes may be classified into male and female. The information on the sender may be input upon registering a telephone number of the sender. For example, if a user registers 'twelve years old and male' as information on a sender upon a user registering a telephone number of his/her grandson, the device 100 may output a motivation message as a voice of a boy in his teens using text to speech (TTS) technology. Upon receiving the motivation message output as his grandson's voice, the user may have a strong desire to continuously measure bio-information.

In this exemplary embodiment, the device 100 may display a user interface (for example, 452) connected to a device external to the health care device. The device 100 may display information on a health state of the patient stored in the device external to the device 100 in response to user input through the user interface. For example, the device 100 may provide a list of bio-information measurement values measured by the user through a web page provided by the server 170.

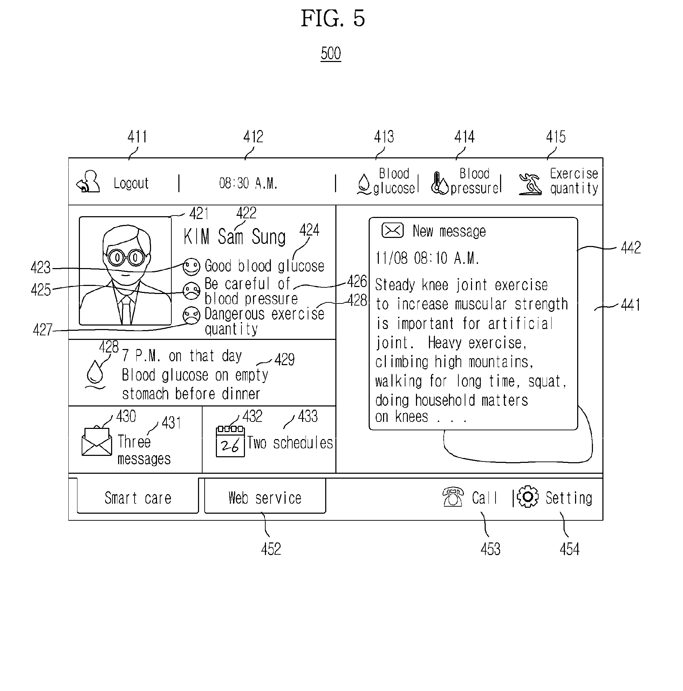

FIG. 5 shows a main view 500 of the device 100 according to another exemplary embodiment.

In this exemplary embodiment, the device 100 may display a new message (for example, 442) in the main view 500. For example, if a new message (for example, 442) is received in a state in which the motivation message (for example, 441) is displayed, the device 100 may display the new message (for example, 442) so that at least a portion of the region at which the new message (for example, 442) is displayed includes at least a portion of the region at which the motivation message (for example, 441) is displayed.

In this exemplary embodiment, the device 100 may display only a portion of the new message (for example, 442). For example, a portion of the new message (for example, 442) may be an abridged version of the new message (for example, 442) or a front part of the new message (for example, 442). The new message (for example, 442) may be at least one message on a message list view 600.

The new message (for example, 442) may be displayed in a pop-up fashion. In this case, the new message (for example, 442) may disappear after a predetermined time (for example, several seconds or several tens of seconds). If the device 100 senses other user input, the displayed new message (for example, 442) may disappear. If the device 100 receives another new message, the previous new message (for example, 442) may disappear, and the received new message may be displayed.

In this exemplary embodiment, the device 100 may acquire a prescription or menu for the patient based on a health state of the patient and display the location of a place where the prescription or menu is provided. At this time, the location of the place may be provided in the new message (for example, 442) in the form of text or an image. The location of the place may be provided in an audible form. For example, if a blood glucose measurement value of the patient is equal to or greater than a normal range, the device 100 may display the location of a restaurant providing a menu necessary for a blood glucose diet (for example, a restaurant providing a vegetable-centered menu). Upon receiving a prescription based on the health state of the patient from a doctor, the device 100 may display the location of a pharmacy providing medicines according to the prescription.

The location of the place providing the prescription or menu may be displayed as follows. The location of the place may be displayed on a map, an absolute location value of the place may be provided using an address or coordinates, or a relative location value of the place may be provided on the basis of the location of the device 100. If the location of the device 100 is changed, the relative location value may also be changed.

When the new message (for example, 442) is displayed, the device 100 may also provide a sound. For example, the device 100 may output various sounds according to degree of importance of the new message (for example, 442). The new message (for example, 442) may include a doctor message, a feedback message based on patient measurement results, an educational material message for health care and a patient counsel record message. For example, upon receiving a message having a high degree of importance (for example, a doctor message which a patient must read or a feedback message regarding poor measurement results), the device 100 may provide a cheerful, speedy or loud sound. On the other hand, upon receiving a message having a low degree of importance (for example, an educational material message or a feedback message regarding good measurement results), the device 100 may provide a quiet, slow or soft sound. The sound may include, without being limited to, background music, a beep sound, or voice announcement. For a message having a high degree of importance, the user may be encouraged to continue his/her health care program through the above-mentioned sound. Also, the device 100 may decide degree of importance of the new message (for example, 442) based on the content of the message. Alternatively, when the new message (for example, 442) is transmitted, a doctor may add information on degree of importance of the new message (for example, 442). The device 100 may acquire the degree of importance from the new message (for example, 442) and provide a sound based on the acquired degree of importance together with the new message.

In this exemplary embodiment, the new message (for example, 442) may be output as a voice. In this case, the device 100 may decide a voice to output the new message according to the degree of importance of the new message (for example, 442). For example, upon receiving a message having a high degree of importance (for example, a doctor message which a patient must read or a feedback message regarding poor measurement results), the device 100 may provide the new message as a cheerful or loud voice using a TTS method. On the other hand, upon receiving a message having a low degree of importance (for example, an educational material message or a feedback message regarding good measurement results), the device 100 may provide the new message as a quiet or soft voice using a TTS method.

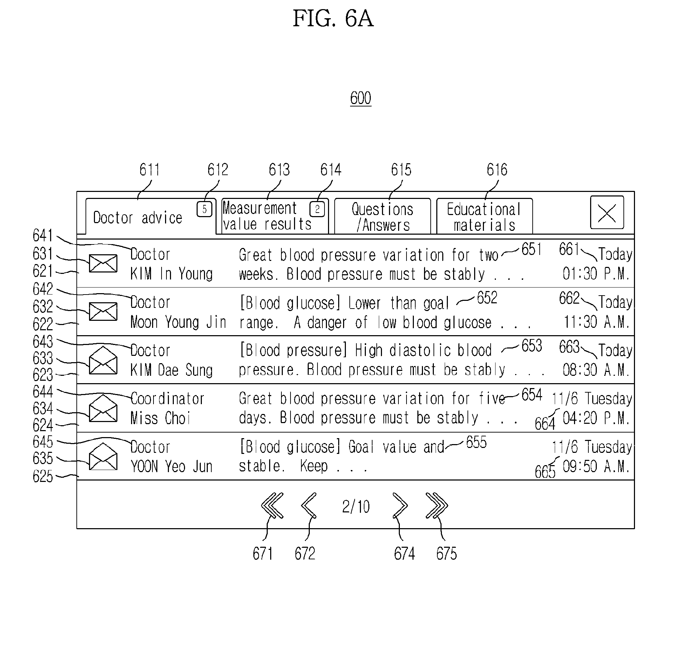

FIG. 6A shows a message list view 600 of the device 100 according to an exemplary embodiment.

In this exemplary embodiment, if a user selects the message icon (for example, 430) or the information (for example, 431) on the message as a user interface to display a message in the main views (for example, 400 and 500) of FIG. 4A, a message list view 600 may be displayed.

The message list view 600 may include a message related to a health state of the patient. For example, the message list view 600 may include advice (for example, 611) of a doctor to improve health of the patient, bio-information measurement value results (for example, 613), questions/answers (for example, 615), and educational materials (for example, 616).

The advice (for example, 611) of the doctor may be provided based on the bio-information measurement value of the user. For example, the device 100 may acquire the bio-information measurement value through the bio-information measurement sensor 160 used by the user and transmit the acquired value to the server 170. The server 170 may provide the received bio-information measurement value to the doctor. The doctor may diagnose the user based on the bio-information measurement value in a remote fashion and transmit a prescription result based on the diagnosis to the device 100. The server 170 may diagnose the user using a CDSS system and transmit a prescription result based on the diagnosis to the device 100.

The message list view 600 may include items (not shown) related to a prescription or menu for the patient. The items may include messages related to the prescription or menu for the patient. The messages may differ from each other according to prescription information or menu information. In this case, each message may include the location of a place providing the acquired prescription or menu.

The user may select tab menus representing the respective items to switch the message list between the items. At this time, the color of a tab (for example, 611) representing a displayed item of the message list may be different from that of tabs (for example, 613, 615 and 616) representing non-displayed items of the message list.

Messages (for example, 621 to 625) of the message list view 600 may include information (for example, 631 to 635) indicating whether the user has read the messages, senders (for example, 641 to 645) of the messages, at least some (for example, 651 to 655) of the message content, and message transmission time (for example, 661 to 665). For example, the messages (for example, 631 and 632), which have not been read by the user, may be indicated as chromatic icons or closed envelope icons. On the other hand, the messages (for example, 633 and 635), which have been read by the user, may be indicated as achromatic icons or open envelope icons.

In this exemplary embodiment, the number (for example, 612 and 614) of the messages corresponding to the items which have not been read may be displayed at one side of the tabs representing the respective items. Also, the messages included in the message list of the items may be arranged and displayed according to a received time sequence.

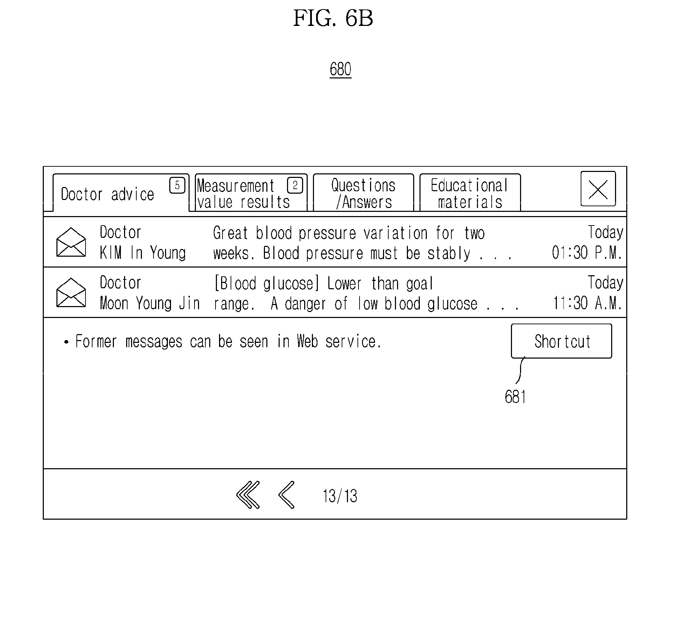

If the number of the messages which are displayed in the message list view 600 at once is limited, the undisplayed messages may appear on other views through user interfaces (for example, 671, 672, 674 and 675). For example, if the user selects the user interfaces (for example, 672 and 674), the message list of the view before or after the displayed view may be displayed. If the user selects the user interfaces (for example, 671 and 675), the message list of the first or last view may be displayed. FIG. 6B shows a message list view 680 of the device 100 according to another exemplary embodiment.

In this exemplary embodiment, if a user selects the user interfaces (for example, 674 and 675) in FIG. 6A, the device 100 may display the messages which have not been displayed in the message list view (for example, 600) in another message list view (for example, 680).

In this exemplary embodiment, the device 100 may provide messages within a predetermined period through the messages list views (for example, 600 and 680). Meanwhile, the messages before the predetermined period are stored in the server 170. If the user selects the user interface (for example, 681), the device 100 may acquire and display the messages before the predetermined period from the server 170.

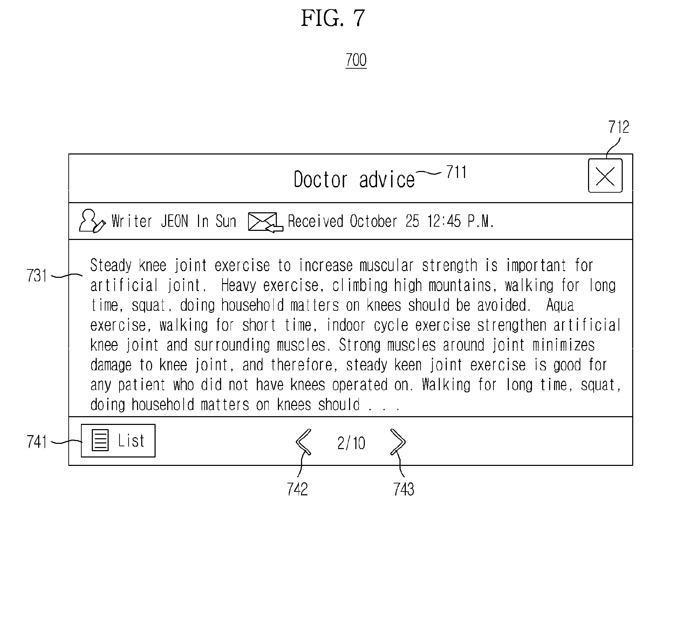

FIG. 7 shows a message detail view 700 according to an exemplary embodiment.

In this exemplary embodiment, if a user selects at least one message from a message list related to advice of a doctor in the message list views (for example, 600 and 680), the device 100 may display a message detail view 700. A message item (for example, 711) related to a health state may be displayed in the view 700. The message item may include at least one selected from among advice of the doctor, measurement value results, questions/answers and educational materials. Also, the view 700 may be provided with a user interface (for example, 712) to return the previous view.

The message detail view 700 may include a portion 731 of the message content. The portion 731 of the message content may include content related to the message item (for example, 711). The text size of the displayed message may be set by the device 100 or the user. Also, the device 100 may display other content of the message, which is not displayed in the view 700, in response to user input. For example, the user input may be a gesture to contact a portion of the region where the message content 731 are displayed, perform movement in contact, and release such contact.

The message detail view 700 may include a user interface 741 to display the message list views (for example, 600 and 680). Also, the message detail view 700 may include user interfaces (for example, 742 and 743) to display messages received before or after the displayed message.

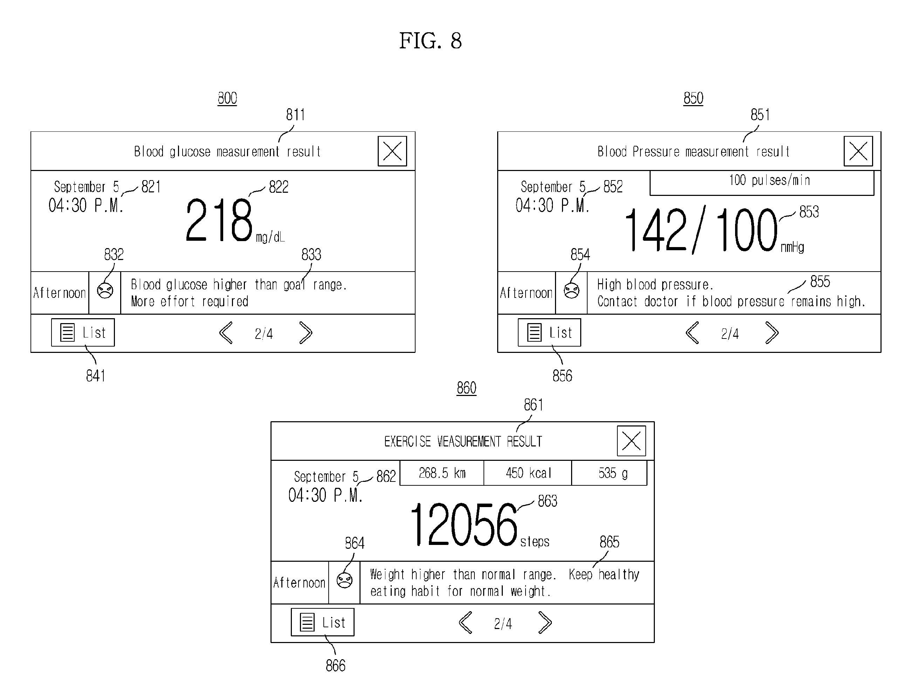

FIG. 8 shows message measurement value views 800, 850 and 860 according to an exemplary embodiment.

In this exemplary embodiment, if a user selects at least one message from a message list of measurement values in the message list views (for example, 600 and 680), the device 100 may display message measurement value views (for example, 800, 850 and 860).

In this exemplary embodiment, the message measurement value views (for example, 800, 850 and 860) may include a bio-information measurement value. 800 of FIG. 8 shows a measurement value of blood pressure if the bio-information is blood pressure. 850 of FIG. 8 shows a measurement value of blood glucose if the bio-information is blood glucose. 860 of FIG. 8 shows a measurement value of exercise quantity if the bio-information is exercise quantity.

The message measurement value views (for example, 800, 850 and 860) may include at least one selected from among kinds (for example, 811, 851 and 861) of bio-information, time (for example, 821, 852 and 862) for the patient to measure bio-information, bio-information measurement values (for example, 822, 853 and 863), images (for example, 832, 854 and 864) indicating health states based on the measurement values, and information (for example, 833, 855 and 865) indicating health states based on the measurement values. Each of the images (for example, 832, 854 and 864) may be one of the images stored in the storage unit 140 based on a health state of the patient according to the bio-information measurement value. Also, the message measurement value views (for example, 800, 850 and 860) may include icons (for example, 841, 856 and 866) to display a message list of measurement values in response to user input.

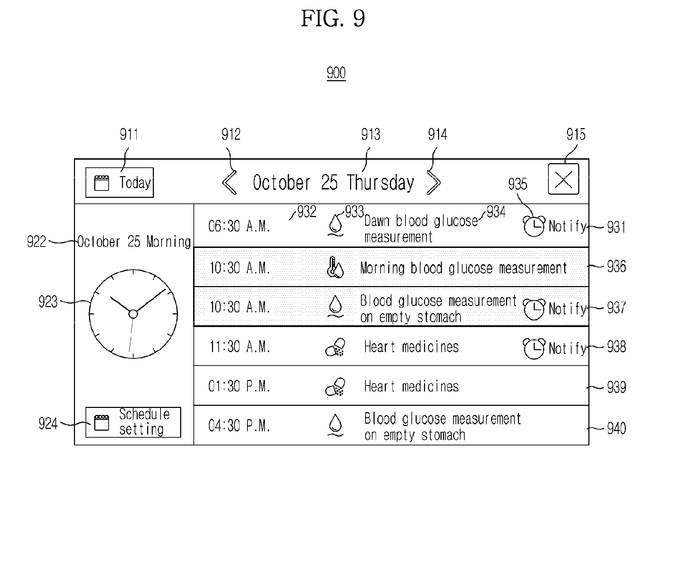

FIG. 9 shows a schedule list view 900 according to an exemplary embodiment.

In this exemplary embodiment, if a user selects a schedule icon (for example, 432) or schedule information (for example, 433) in the main view (for example, 400) of FIG. 4A, the device 100 may display a schedule list view 900 as a schedule to measure patient bio-information.

In this exemplary embodiment, the device 100 may provide a schedule list of a date (for example, 913) displayed in the schedule list view 900 and a date before or after the displayed date in response to user input through user interfaces (for example, 912 and 914). Also, the device 100 may provide a schedule list of a today's date in response to user input through a user interface (for example, 911). Also, the device 100 may provide a view before entering the schedule list view 900 in response to user input through a user interface (for example, 915).

In this exemplary embodiment, a today's date 922 and/or graphs 923 indicating current time may be displayed at one side of the schedule list view 900.

In this exemplary embodiment, a schedule list (for example, 931, 936 to 940) related to a health state of the patient may be displayed at the other side of the schedule list view 900. The device 100 may provide visual effects to schedules (for example, 936 and 937) having priorities so that the schedules of the schedule list are divided according to the priorities. As an example of the visual effects, the color or resolution of a region where a schedule having a high priority is displayed may be different from that of a region where a schedule having a low priority is displayed.

In this exemplary embodiment, the schedule list (for example, 931 and 936 to 940) may include a schedule to measure bio-information of the patient or a dosage schedule of the patient. For example, the schedule (for example, 931) may include, without being limited to, at least one selected from among bio-information measurement time (for example, 932), an icon (for example, 933) representing bio-information, content (for example, 934) related to measurement of bio-information, and a notification icon (for example, 935) to notify the user of the bio-information measurement time (for example, 932).

The bio-information measurement time may be notified through a new view (for example, a popup view) or sound. Various sounds may be provided based on a health state of the patient. For example, if the patient has a good health state, a quiet or soft sound may be provided. On the other hand, if the patient has a bad health state, a cheerful or loud sound may be provided. The sound may include, without being limited to, specific music, a beep sound, or voice announcement. The patient may be encouraged to measure bio-information and actively maintain his/her health care through the notification.

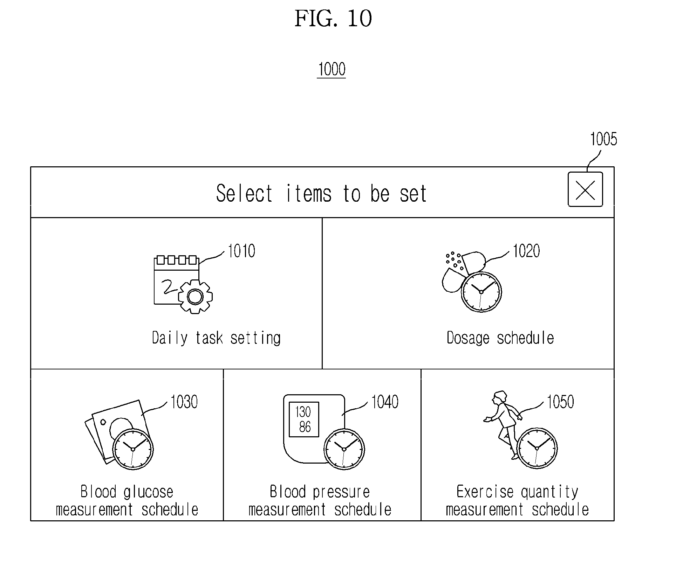

FIG. 10 shows a schedule setting view 1000 according to an exemplary embodiment.

In this exemplary embodiment, if a user selects the user interface 924 in FIG. 9 according to the schedule setting of the schedule list view 900, the device 100 may display a schedule setting view 1000.

In this exemplary embodiment, the schedule setting view 1000 may include a daily task setting user interface (for example, 1010) to set a daily task, a dosage schedule user interface (for example, 1020) to set a dosage schedule, and user interfaces (for example, 1030, 1040 and 1050) to set a bio-information measurement schedule. For example, the user interfaces (for example, 1030, 1040 and 1050) may include a user interface (for example, 1030) to set a blood glucose measurement schedule, a user interface (for example, 1040) to set a blood pressure measurement schedule, and a user interface (for example, 1050) to set an exercise quantity measurement schedule. Also, the device 100 may provide a view before entering the schedule setting view 1000 in response to user input through a user interface (for example, 1005).

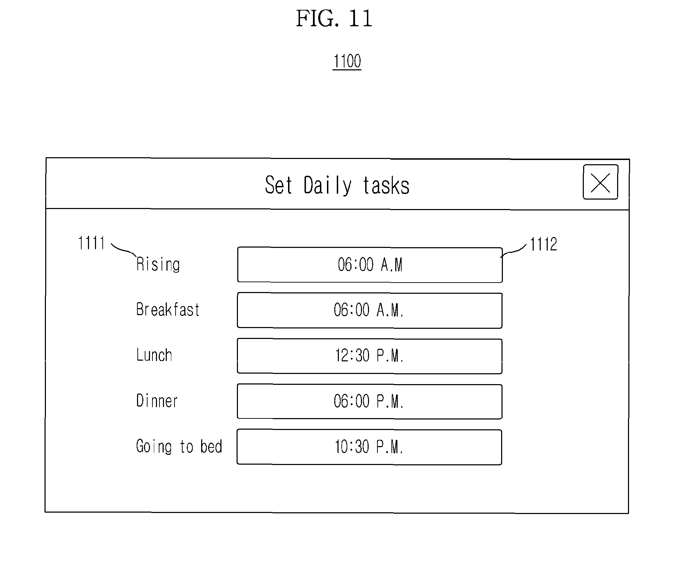

FIG. 11 shows a day schedule setting view 1100 according to an exemplary embodiment.

In this exemplary embodiment, if a user selects the user interface 1010 related to the daily task setting of the schedule setting view 1000, the device 100 may display a day schedule setting view 1100.

In this exemplary embodiment, the user may set detailed time (for example, 1112) of main schedules (for example, 1111). For example, the main schedules may include, without being limited to, rising, breakfast, lunch and dinner. The day schedule setting view 1100 may further include a user interface (not shown) to add a new main schedule.

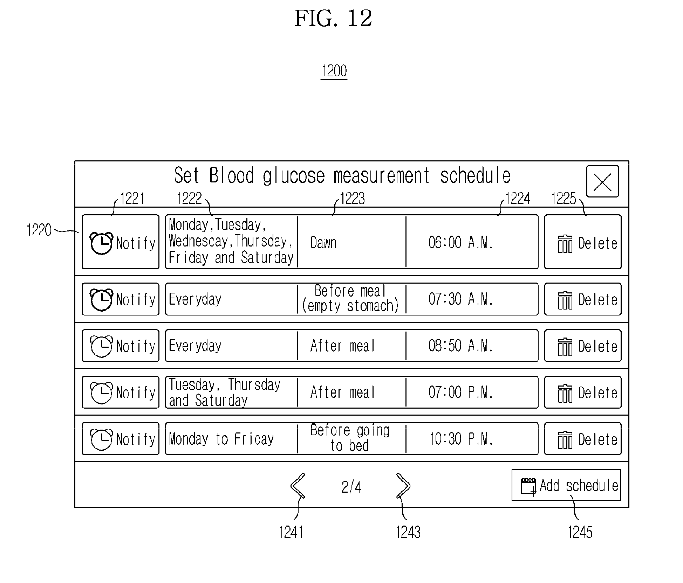

FIG. 12 shows a measurement schedule setting view 1200 according to an exemplary embodiment.

In this exemplary embodiment, if a user selects the user interfaces (for example, 1030, 1040 and 1050) related to the bio-information measurement schedule of the schedule setting view (for example, 1000), the device 100 may display a measurement schedule setting view 1200. For example, the bio-information measurement schedule of the schedule setting view (for example, 1000) may include at least one selected from among a blood glucose measurement schedule, a blood pressure measurement schedule and an exercise quantity measurement schedule. In this exemplary embodiment, the measurement schedule setting view 1200 may include a user interface (for example, 1221) to set a notification requesting bio-information measurement, user interfaces (for example, 1222 to 1224) to set a bio-information measurement cycle, and a user interface (for example, 1225) to delete a schedule. The user interfaces (for example, 1222 to 1224) to set the bio-information measurement cycle may include an item (for example, 1222) indicating a measurement day of the week, an item (for example, 1223) indicating measurement time, and an item (for example, 1224) indicating measurement hours.

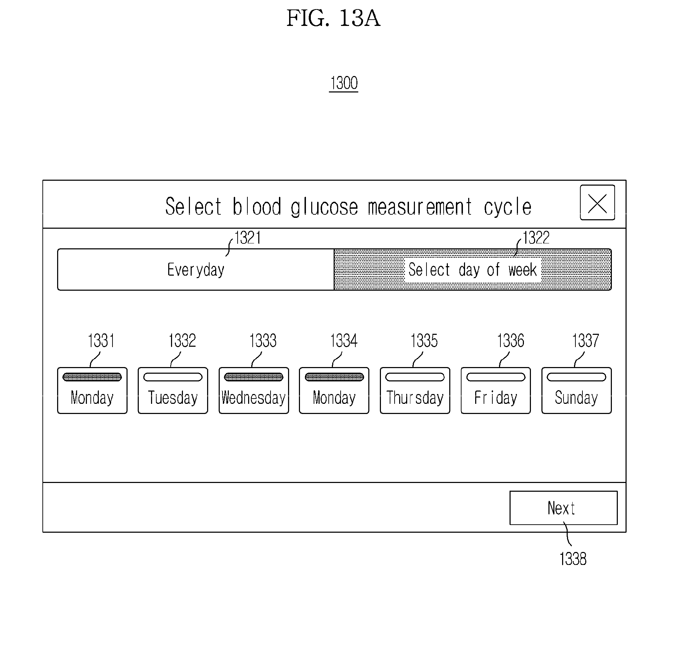

In this exemplary embodiment, if the user selects the user interface (for example, 1221) to set the notification, notification of a selected schedule may be set, and the user interface (for example, 1221) may be activated. Also, if user interfaces (for example, 1241 and 1243) are selected, the device 100 may further display another schedule which is not displayed in the measurement schedule setting view 1200. FIG. 13A shows a schedule addition view 1300 according to an exemplary embodiment.

In this exemplary embodiment, if a user selects a user interface (for example, 1245 in FIG. 12) related to schedule addition in the measurement schedule setting view 1200, the device 100 may display a schedule addition view 1300.

The schedule addition view 1300 may include a user interface (for example, 1321) to set a bio-information measurement cycle on a per day basis and a user interface (for example, 1322) to set the measurement cycle on a per day-of-the-week basis. Also, the user may set days of the week for measurement through user interfaces (for example, 1331 to 1337).

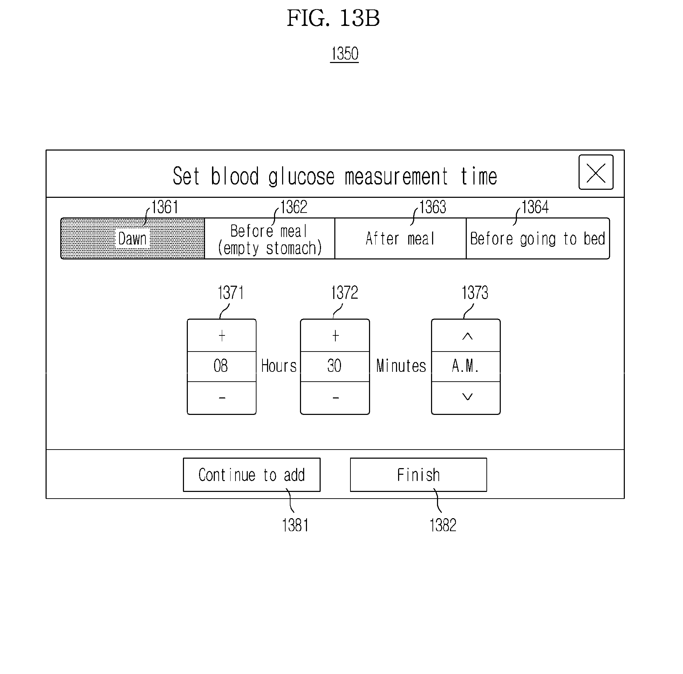

FIG. 13B shows a schedule addition view 1350 according to an exemplary embodiment.

In this exemplary embodiment, if a user selects a user interface (for example, 1338 in FIG. 13A) to set time in the schedule addition view 1300, the device 100 may display a schedule addition view 1350 to set time.

The schedule addition view 1350 may include user interfaces (for example, 1361 to 1364) to set main time to measure bio-information and user interfaces (for example, 1371 to 1373) to set detailed time. For example, the user interfaces (for example, 1361 to 1364) may include at least one setting items selected from among dawn, before a meal (empty stomach), after a meal, and before going to bed. Also, the user interfaces (for example, 1371 to 1373) may include hour (for example, 1371), minute (for example, 1372) and morning/afternoon (for example, 1373). The value of each item may be adjusted through a user interface such as +/- or a caret/inverted caret.

The user may add a schedule through a user interface (for example, 1381 in FIG. 13B) for continuous addition of the schedule addition view 1350 or finish the addition of a schedule through a user interface (for example, 1382 in FIG. 13B) for completion, and return to the measurement schedule setting view (for example, 1200).

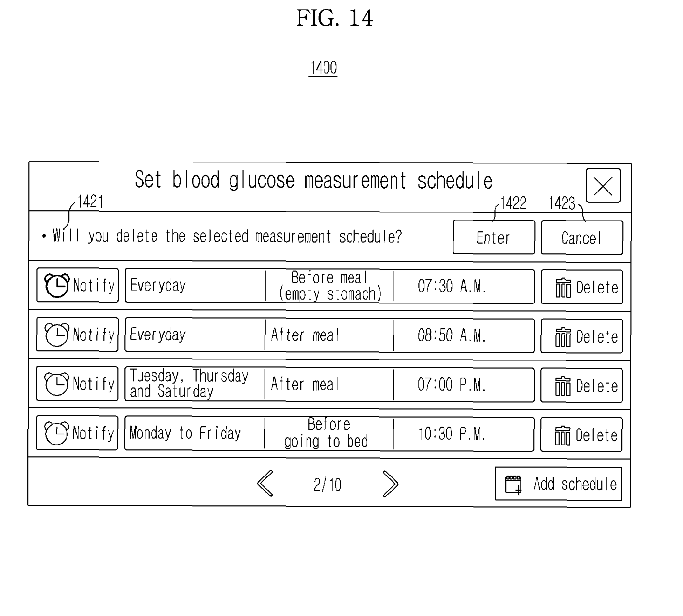

FIG. 14 shows a schedule deletion view 1400 according to an exemplary embodiment.

In this exemplary embodiment, if a user selects the user interface (for example, 1225) to delete a schedule in the measurement schedule setting view (for example, 1200), the device 100 may display a schedule deletion view 1400.

The schedule deletion view 1400 may display information 1421 to confirm whether a measurement schedule (for example, 1220 in FIG. 12) corresponding to the user interface (for example, 1225) is to be deleted. If the user selects a confirmation user interface 1422, the device 100 may delete the measurement schedule (for example, 1220). Also, if the user selects a cancelation user interface 1423, the device 100 may cancel the deletion of the measurement schedule (for example, 1220).

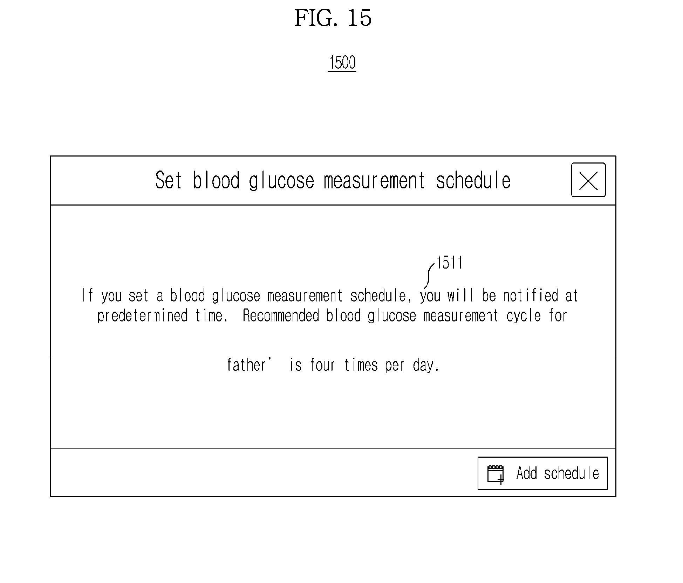

FIG. 15 shows a measurement schedule request view 1500 according to an exemplary embodiment.

In this exemplary embodiment, if a user selects the user interfaces (for example, 1030, 1040 and 1050) related to the bio-information measurement schedule of the schedule setting view 1000 and no measurement schedules are stored, the device 100 may display a measurement schedule request view 1500.

The measurement schedule request view 1500 may display information 1511 to request that the user input a measurement schedule. For example, the measurement schedule request information 1511 may include bio-information necessary to be measured, a bio-information measurement cycle, bio-information measurement time and addition of a schedule.

Meanwhile, the device 100 may provide a basic schedule to measure bio-information although the user does not add a schedule. For example, the basic schedule may be transmitted from the server 170 via a doctor or may be selected from a schedule list stored in the storage unit 140 or the server 170 in consideration of user bio-information.

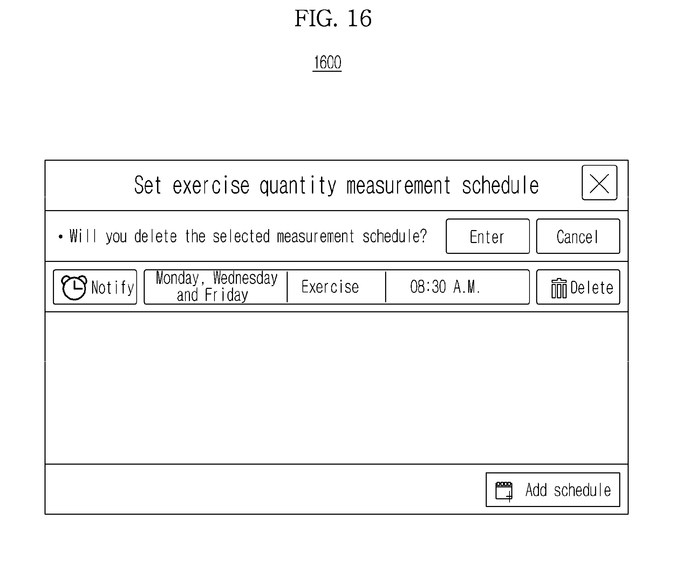

FIG. 16 shows a schedule deletion view 1600 according to an exemplary embodiment.

In this exemplary embodiment, if a user selects the user interface to delete a schedule in the exercise quantity measurement schedule view, the device 100 may display a schedule deletion view 1600.

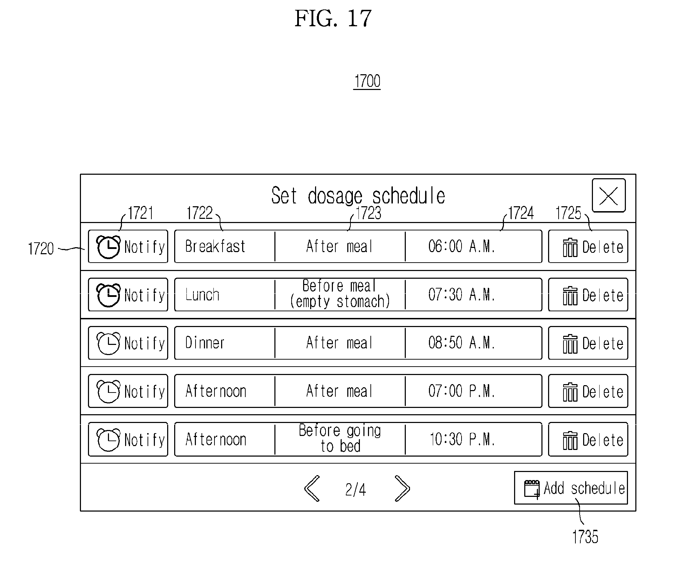

FIG. 17 shows a dosage schedule view 1700 according to an exemplary embodiment.

In this exemplary embodiment, if a user selects the dosage schedule user interface (for example, 1020) of the schedule setting view 1000, the device 100 may display a dosage schedule view 1700.

In this exemplary embodiment, the dosage schedule view 1700 may include at least one selected from among a user interface (for example 1721) to set a notification requesting dosage at a predetermined time, user interfaces (for example, 1722 to 1724) to set dosage time, and a user interface 1725 to delete a schedule. The user interfaces (for example, 1722 to 1724) to set dosage time may include at least one selected from among an item (for example, 1722) indicating a first dosage time (for example, breakfast, lunch, dinner, morning and afternoon), an item (for example, 1723) indicating a second dosage time (for example, after a meal, before a meal, between meals, and before going to bed), which has a unit time smaller than that of the first dosage time, and an item (for example, 1724) indicating a unit time smaller than that of the second dosage time. A process of setting a dosage schedule in the dosage schedule view 1700 may be equal to the process of setting the measurement schedule in the measurement schedule view 1200 of FIG. 12.

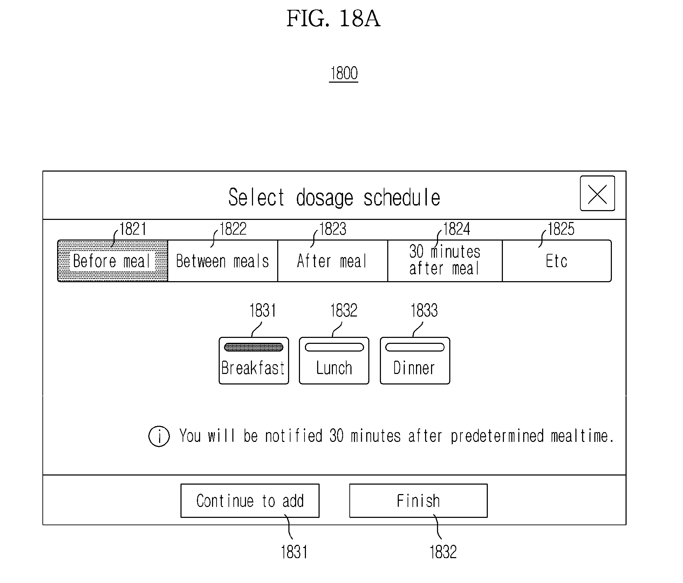

FIG. 18A shows a schedule addition view 1800 according to an exemplary embodiment.

In this exemplary embodiment, if a user selects the user interface (for example, 1735 in FIG. 17) related to the schedule addition in the dosage schedule view (for example, 1700), the device 100 may display a schedule addition view 1800.

The schedule addition view 1800 may include user interfaces (for example, 1831 to 1833) to select at least one from among the first dosage time (for example, breakfast, lunch and dinner). Also, the schedule addition view 1800 may include user interfaces (for example, 1821 to 1825) to select at least one from among the second dosage time (for example, before a meal, after a meal, 30 minutes after a meal, etc.)

The user may select at least one from among the user interfaces (for example, 1831 to 1833) and the user interfaces (for example, 1821 to 1825) to add a dosage schedule. Meanwhile, the user may select a user interface (for example, 1831) for continuous addition of a dosage schedule to add the schedule, or select a user interface (for example, 1832) for completion to finish the addition of a schedule, and return to the dosage schedule view (for example, 1700).

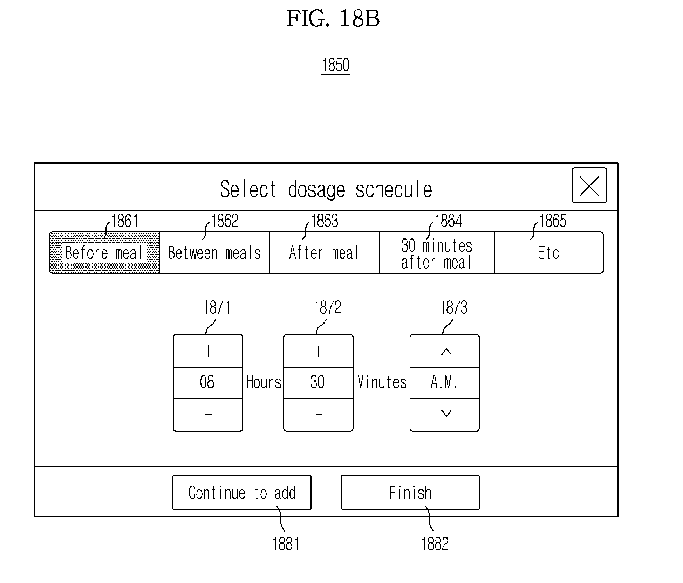

FIG. 18B shows a schedule addition view 1850 according to an exemplary embodiment.

In this exemplary embodiment, if a user selects the user interface (for example, 1735) related to the schedule addition in the dosage schedule view (for example, 1700), the device 100 may display a schedule addition view 1850. If the user selects the user interfaces (for example, 1821 to 1825 and 1831 to 1833) in the schedule addition view (for example, 1800), the device 100 may display the schedule addition view 1850.

The schedule addition view 1850 may include user interfaces (for example, 1861 to 1865) to select a second dosage time (for example, before a meal, after a meal, 30 minutes after a meal, etc.) and user interfaces (for example, 1871 to 1873) to set a third dosage time. For example, user interfaces (for example, 1871 to 1873) may include hour (for example, 1871), minute (for example, 1872) and morning/afternoon (for example, 1873). The value of each item may be adjusted through a user interface such as +/- or a caret/inverted caret. The user may add a schedule through a user interface (for example, 1881) for continuous addition of a dosage schedule or finish the addition of a schedule through a user interface (for example, 1882) for completion, and return to the dosage schedule view 1700.

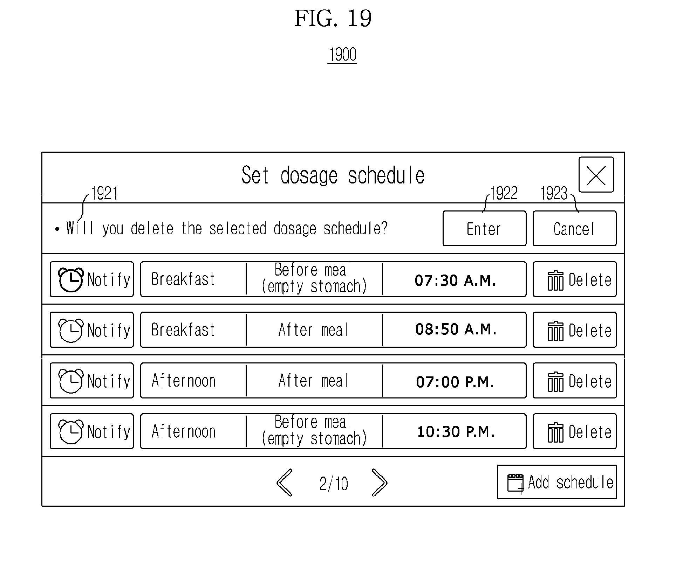

FIG. 19 shows a schedule deletion view 1900 according to an exemplary embodiment.

In this exemplary embodiment, if a user selects the user interface (for example, 1725) to delete a dosage schedule in the dosage schedule view (for example, 1700), the device 100 may display a schedule deletion view 1900.

The schedule deletion view 1900 may display information 1921 to confirm whether a dosage schedule (for example, 1720 in FIG. 17) corresponding to the user interface (for example, 1725) is to be deleted. If the user selects a confirmation user interface 1922, the device 100 may delete the dosage schedule (for example, 1720). Also, if the user selects a cancelation user interface 1923, the device 100 may cancel the deletion of the dosage schedule (for example, 1720).

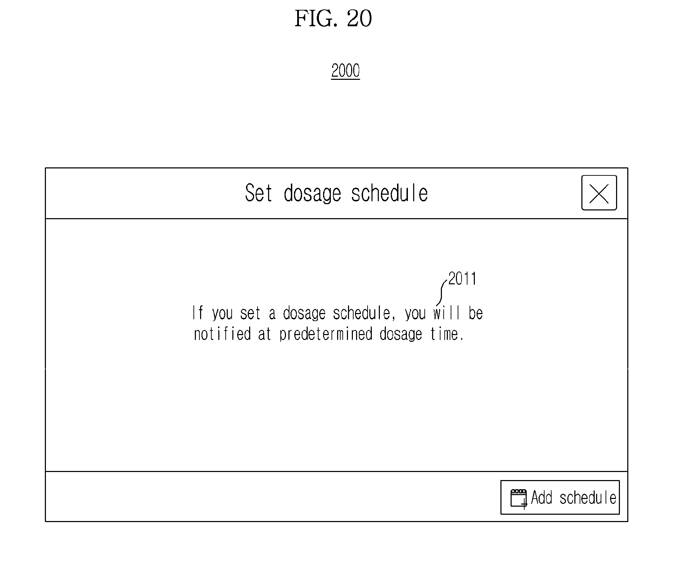

FIG. 20 shows a dosage schedule request view 2000 according to an exemplary embodiment.

In this exemplary embodiment, if a user selects the user interface 1020 related to the dosage schedule in the schedule setting view 1000 and no dosage schedules are stored, the device 100 may display a dosage schedule request view 2000.

The dosage schedule request view 2000 may display information 2011 to request that the user input a dosage schedule.

Meanwhile, the device 100 may provide a basic dosage schedule although the user does not add a schedule. For example, the basic dosage schedule may be transmitted from the server 170 via a doctor or user dosage or bio-information may be selected from a dosage schedule list stored in the storage unit 140 or the server 170.

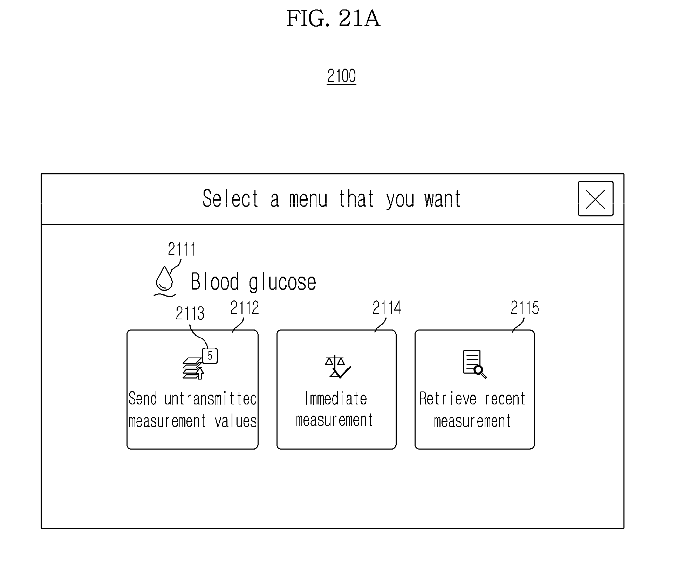

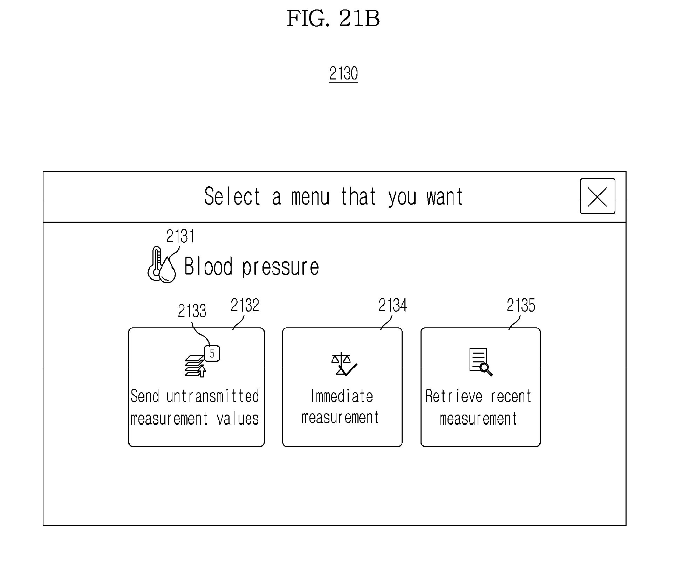

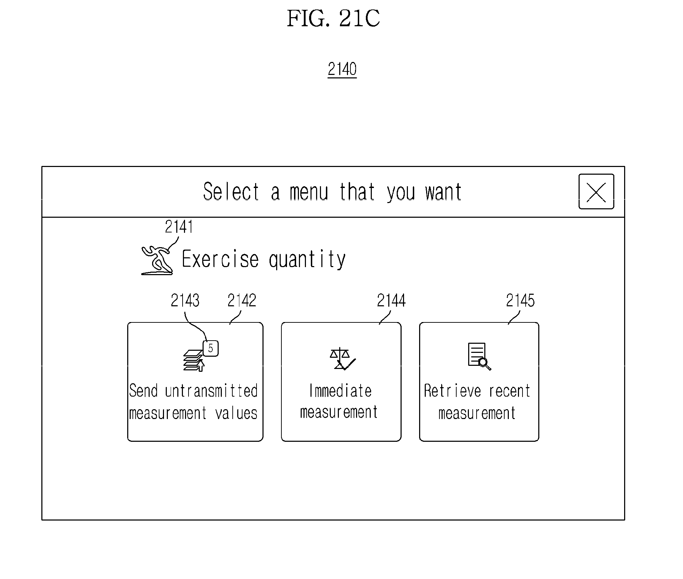

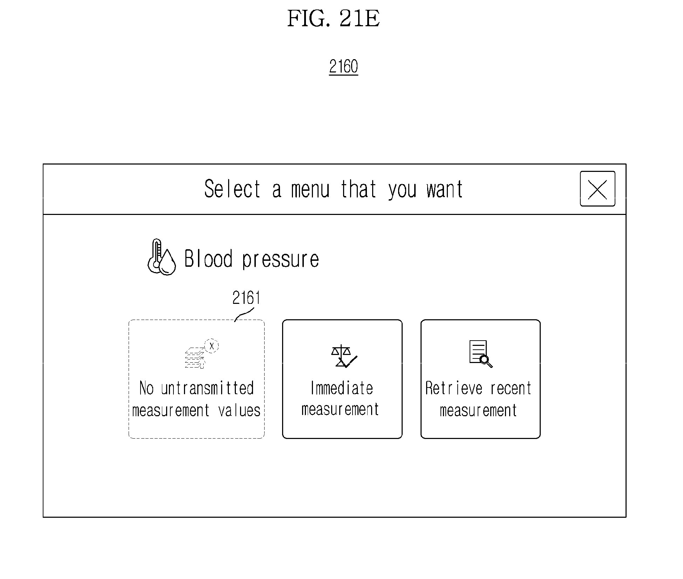

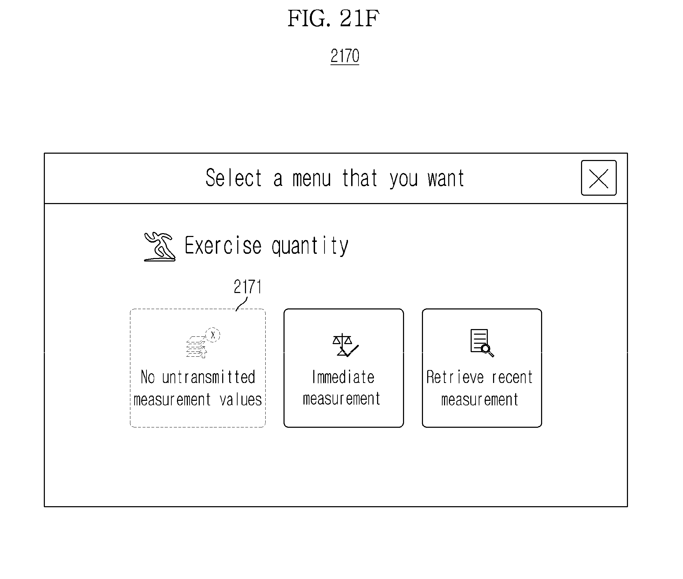

FIGS. 21A to 21F show bio-information measurement views 2100, 2130 to 2170 according to an exemplary embodiment.

In this exemplary embodiment, if a user selects the user interfaces (for example, 413 to 415) related to bio-information measurement in the main view (for example, 400), the device 100 may display bio-information measurement views 2100, 2130, 2140, 2150, 2160 and 2170.

For example, if the user selects the user interface (for example, 413) in the main view 400, the device 100 may display blood glucose measurement views 2100 and 2150. If the user selects the user interface (for example, 414) in the main view 400, the device 100 may display blood pressure measurement views 2130 and 2160. If the user selects the user interface (for example, 415) in the main view 400, the device 100 may display exercise quantity measurement views 2140 and 2170.

The bio-information measurement views (for example, 2100, 2130 and 2140) may include at least one selected from among icons (for example, 2111, 2131 and 2141) indicating bio-information to be measured, user interfaces (for example, 2112, 2132 and 2142) to transmit untransmitted measurement values of bio-information, user interfaces (for example, 2114, 2134 and 2144) to measure bio-information, and user interfaces (for example, 2115, 2135 and 2145) to provide a list of bio-information measurement values which have been recently measured.

In this exemplary embodiment, referring to FIGS. 21A to 21C, the device 100 may display the number (for example, 2113, 2133 and 2143) of untransmitted bio-information measurement values on regions of the user interfaces (for example, 2112, 2132 and 2142). In this case, the user may select the user interfaces (for example, 2112, 2132 and 2142) to transmit the untransmitted measurement values of bio-information, which are stored in the device 100, to the server 170.

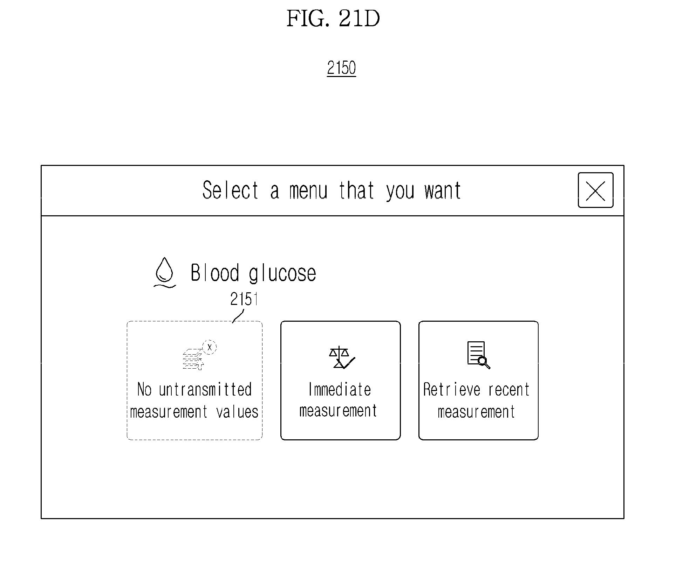

In this exemplary embodiment, referring to FIGS. 21D to 21F, if there are no untransmitted measurement values of bio-information, the device 100 may display the bio-information measurement views (for example, 2150 to 2170). At this time, the bio-information measurement views (for example, 2150 to 2170) may include inactivated user interfaces (for example, 2151, 2161 and 2171) indicating that there are no untransmitted measurement values of bio-information.

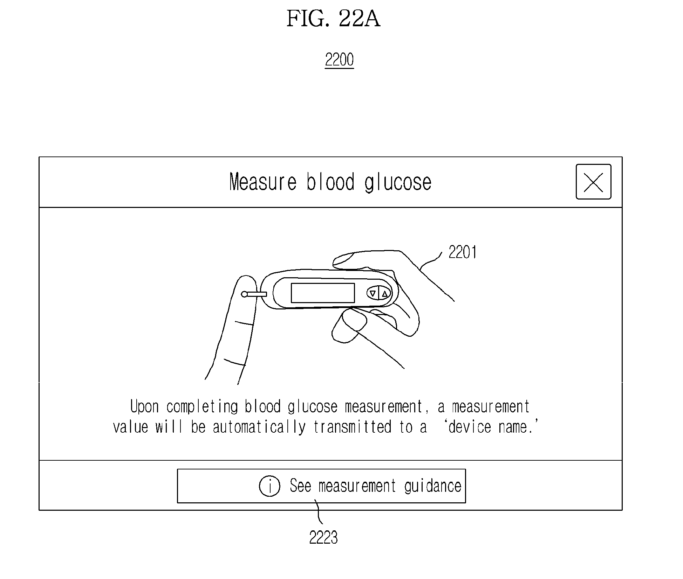

FIGS. 22A to 22C show immediate measurement views 2200, 2250 and 2260 according to an exemplary embodiment.

In this exemplary embodiment, if a user selects the user interfaces (for example, 2114, 2134 and 2144) to immediately measure bio-information in the bio-information measurement views (for example, 2100, 2130 and 2140), the device 100 may display immediate measurement views 2200, 2250 and 2260.

For example, if the user selects the user interface (for example, 2114) in the bio-information measurement view 2100, the device 100 may display the immediate measurement view 2200 to measure blood glucose. If the user selects the user interface (for example, 2134) in the bio-information measurement view 2130, the device 100 may display the immediate measurement view 2250 to measure blood pressure. If the user selects the user interface (for example, 2144) in the bio-information measurement view 2140, the device 100 may display the immediate measurement view 2260 to measure exercise quantity.

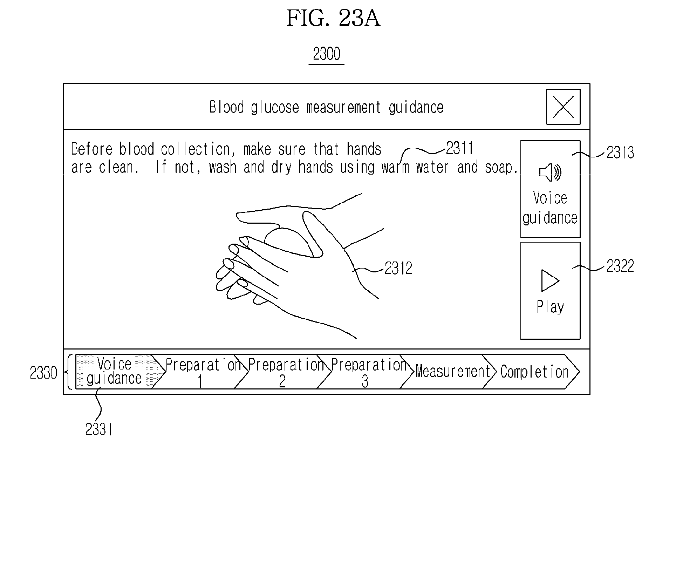

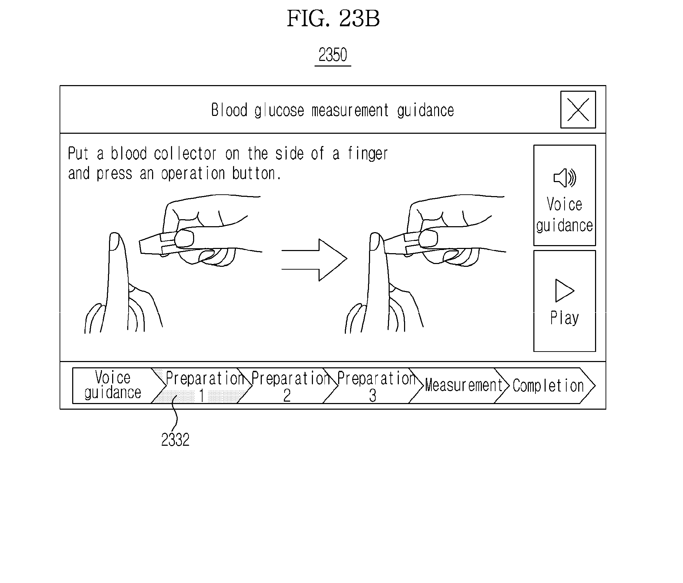

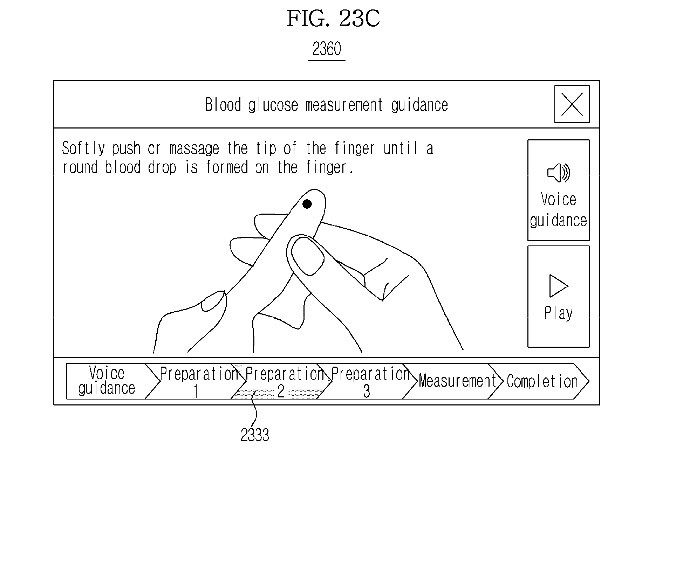

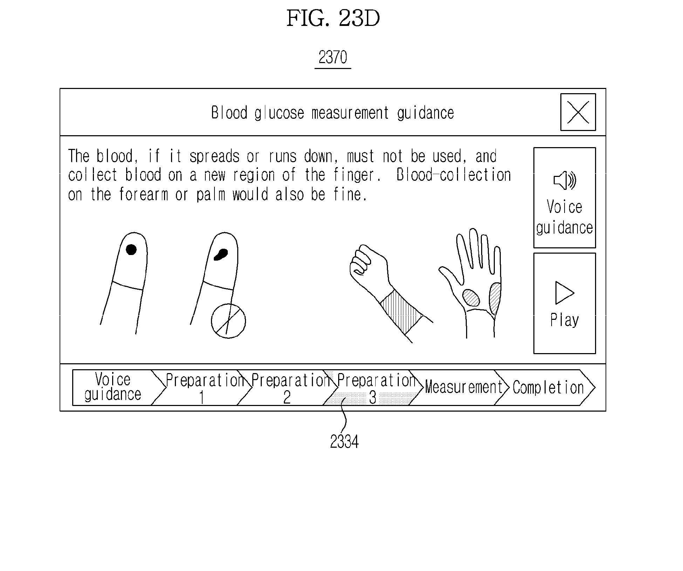

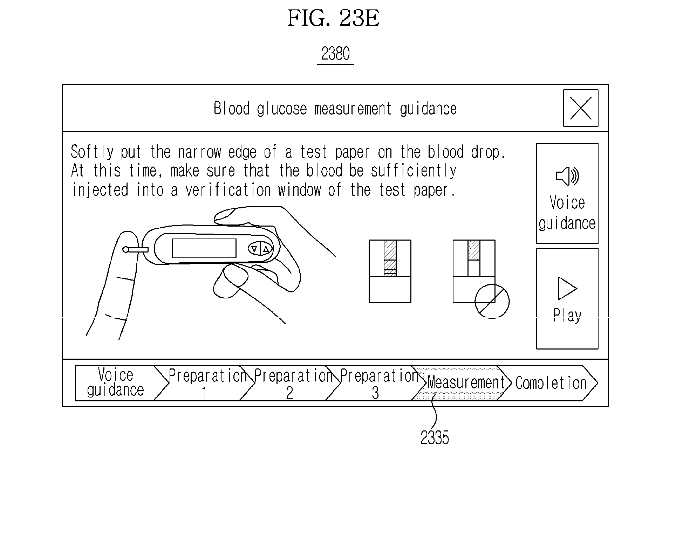

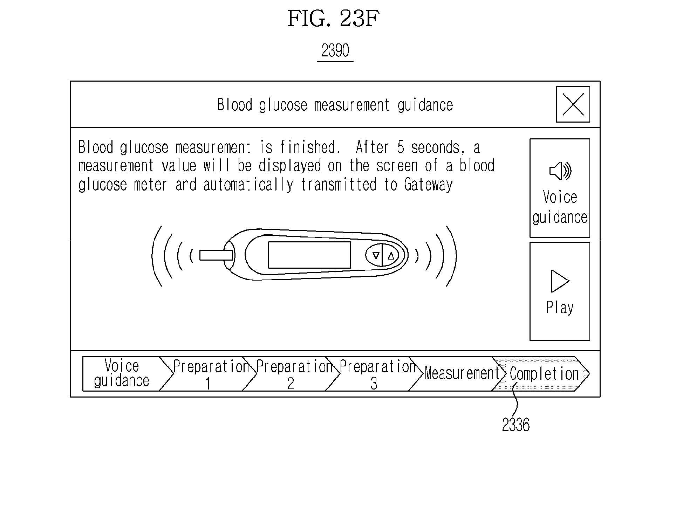

The immediate measurement views 2200, 2250 and 2260 may include information (for example, 2201, 2251 and 2261) to request that the user perform bio-information measurement. For example, the information to request bio-information measurement may be provided as, without being limited to, graphs, text or sound. FIGS. 23A to 23F show measurement guidance views 2300, 2350, 2360, 2370, 2380 and 2390 according to an exemplary embodiment.

In this exemplary embodiment, if a user selects user interfaces (for example, 2223, 2253 and 2263) to guide bio-information measurement in the immediate measurement views 2200, 2250 and 2260, the device 100 may display bio-information measurement guidance views 2300, 2350, 2360, 2370, 2380 and 2390. For example, if the user selects the user interface (for example, 2223 in FIG. 22A) to guide blood glucose measurement in the immediate measurement view 2200, the bio-information measurement guidance views may include blood glucose measurement guidance views (for example, 2300, 2350, 2360, 2370, 2380 and 2390).

The bio-information measurement operations may be provided in the form of a progress bar 2230 at one side of the measurement guidance view (for example, 2300). The measurement guidance views (for example, 2300, 2350, 2360, 2370, 2380 and 2390) may include at least one selected from among a pre- measurement operation (for example, 2331), preparation operations (for example, 2332, 2333 and 2334), a measurement operation (for example, 2335) and a completion operation (for example, 2336).

The measurement guidance view (for example, 2300) may include information (for example, 2311, 2312 and 2313) related to measurement guidance. For example, the information related to measurement guidance may be provided in the form of graphs (for example, 2311), text (for example, 2312) or sound (for example, 2313). If the information related to measurement guidance is information to measure blood glucose, at least one of the measurement guidance views (for example, 2300, 2350, 2360, 2370, 2380 and 2390) may include information on how to use a blood glucose meter.

In this exemplary embodiment, the user may select one of the operations included in the progress bar 2330 of the measurement guidance view (for example, 2300) to shift a view corresponding to the selected operation. If the user contacts a portion of the measurement guidance view (for example, 2350), performs movement in contact, and releases such contact, the device 100 may display the view (for example, 2300) before the measurement guidance view (for example, 2350) or the view (for example, 2360) after the measurement guidance view (for example, 2350). Also, the user may use a user interface (for example, 2322) to sequentially display the measurement guidance views (for example, 2300, 2350, 2360, 2370, 2380 and 2390).

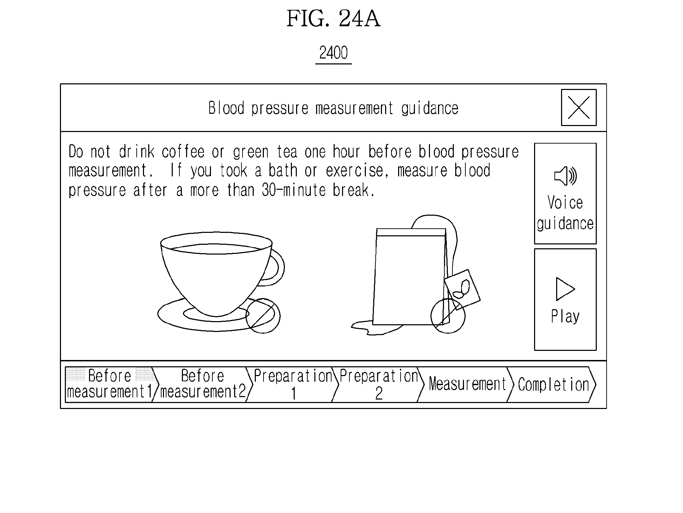

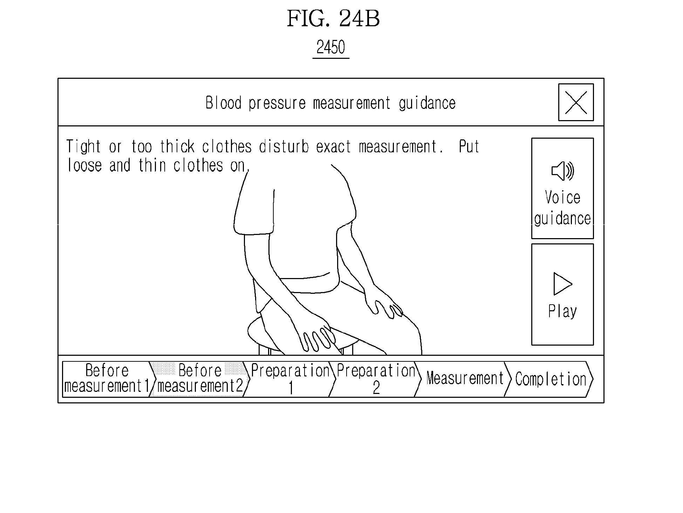

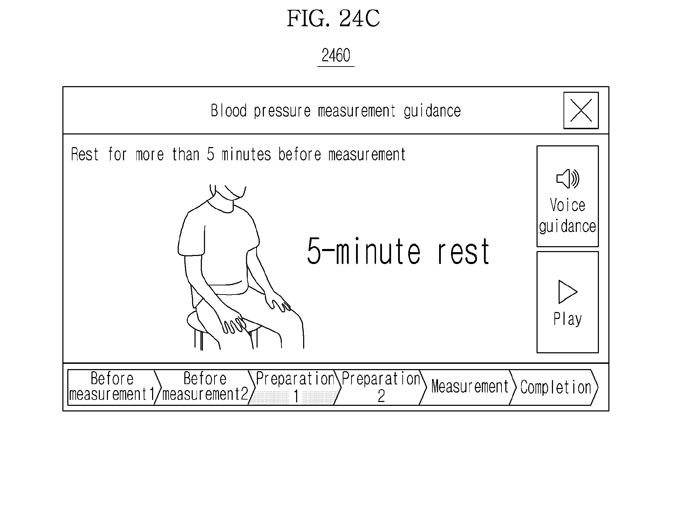

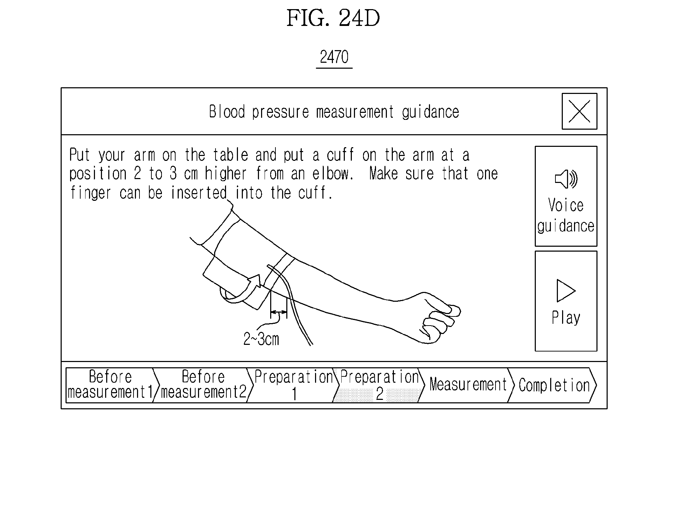

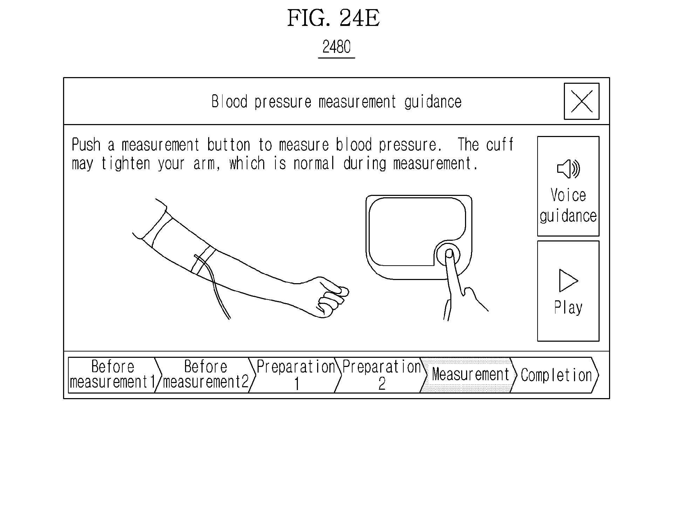

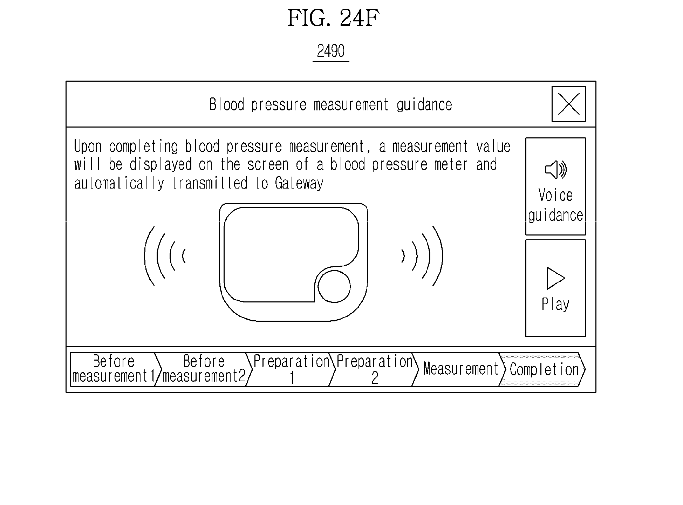

FIGS. 24A to 24F show measurement guidance views according to an exemplary embodiment.

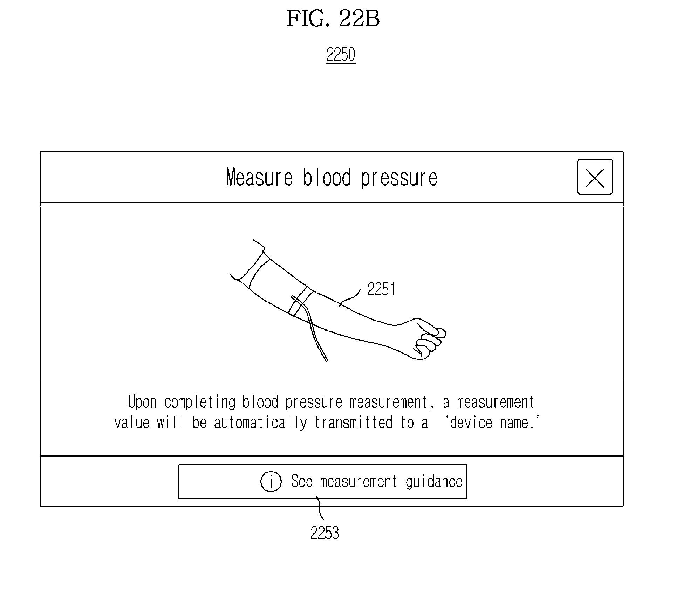

In this embodiment, if a user selects the user interface (for example, 2253 in FIG. 22B) to guide blood pressure measurement in the immediate measurement view 2250, the bio-information measurement guidance views may include blood pressure measurement guidance views (for example, 2400, 2450, 2460, 2470, 2480 and 2490).

In this exemplary embodiment, the measurement guidance views (for example, 2400, 2450, 2460, 2470, 2480 and 2490) may include information related to measurement guidance necessary before the user measures blood pressure and information on caution during measurement. Also, at least one of the measurement guidance views (for example, 2400, 2450, 2460, 2470, 2480 and 2490) may include information on how to use a blood pressure meter.

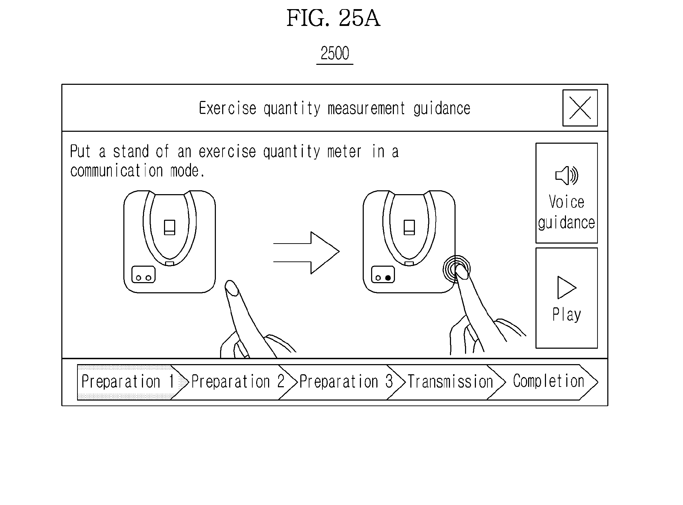

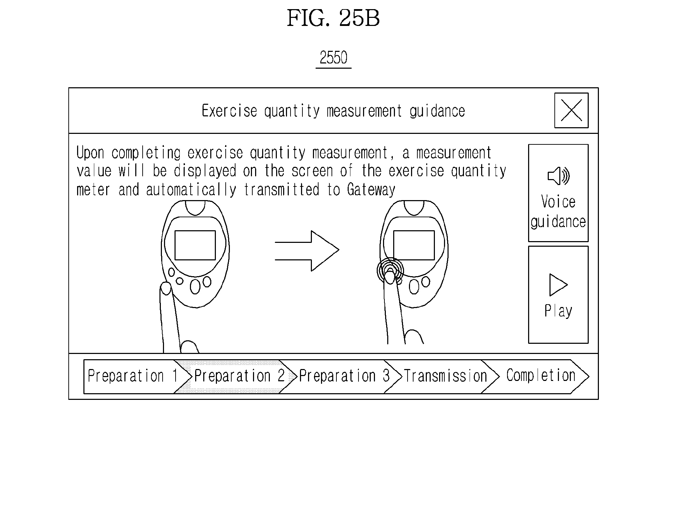

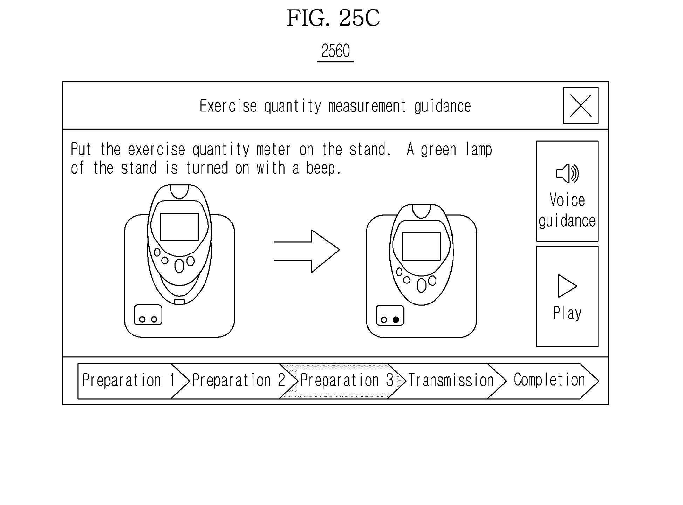

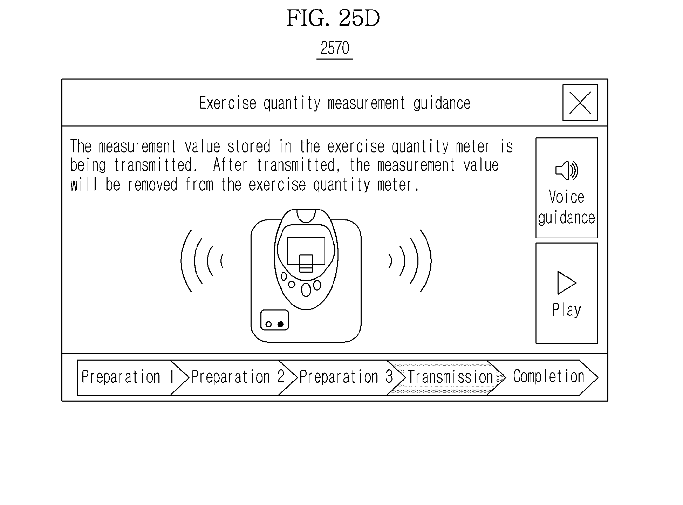

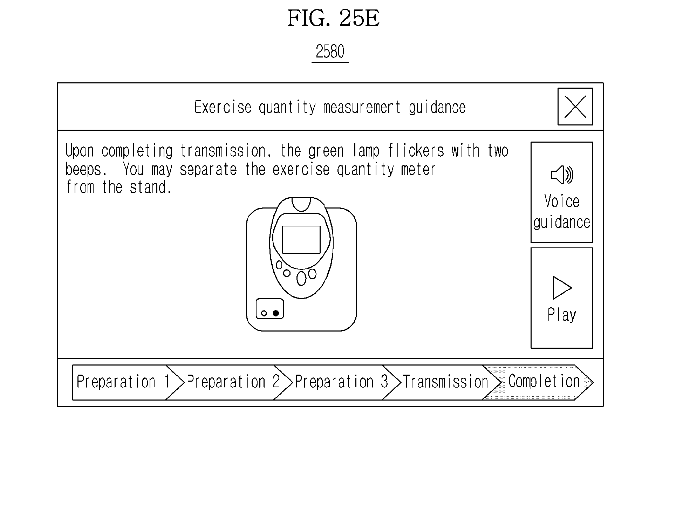

FIGS. 25A to 25E show measurement guidance views according to an exemplary embodiment.

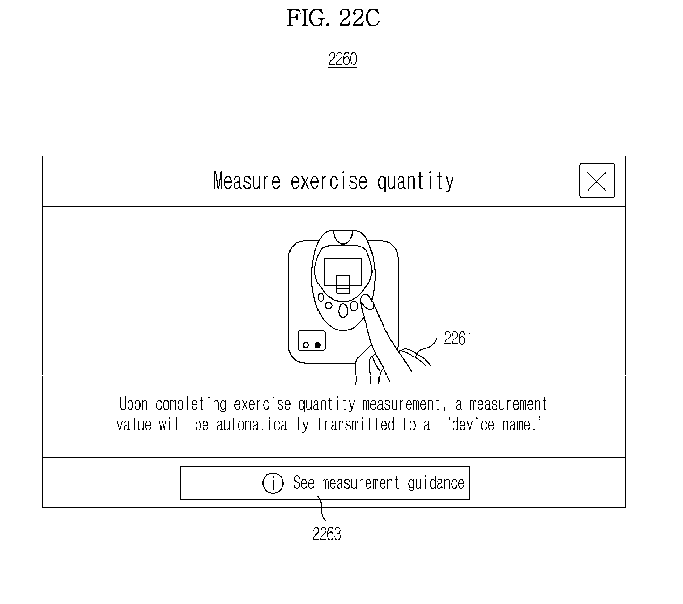

In this exemplary embodiment, if a user selects the user interface (for example, 2263 in FIG. 22C) to guide exercise quantity measurement in the immediate measurement view 2260, the bio-information measurement guidance views may include exercise quantity measurement guidance views (for example, 2500, 2550, 2560, 2570 and 2580).

In this exemplary embodiment, the measurement guidance views (for example, 2500, 2550, 2560, 2570 and 2580) may include information related to measurement guidance necessary before the user uses an exercise quantity meter and information on caution during measurement. Also, at least one of the measurement guidance views (for example, 2500, 2550, 2560, 2570 and 2580) may include information on how to use an exercise quantity meter or information to transmit an exercise quantity measurement value stored in the exercise quantity meter to the device 100.

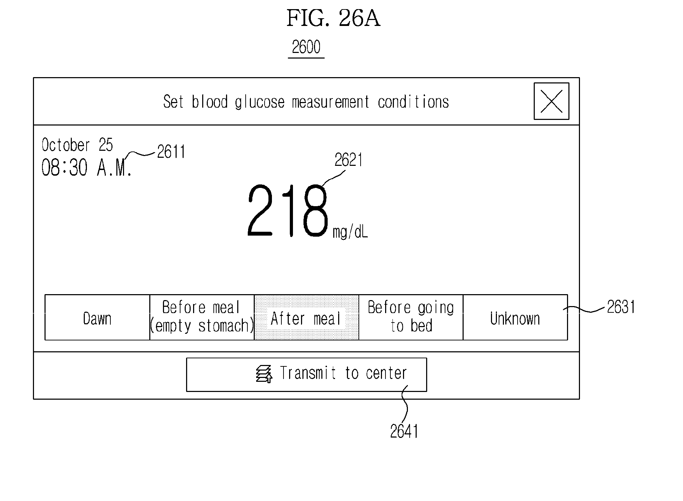

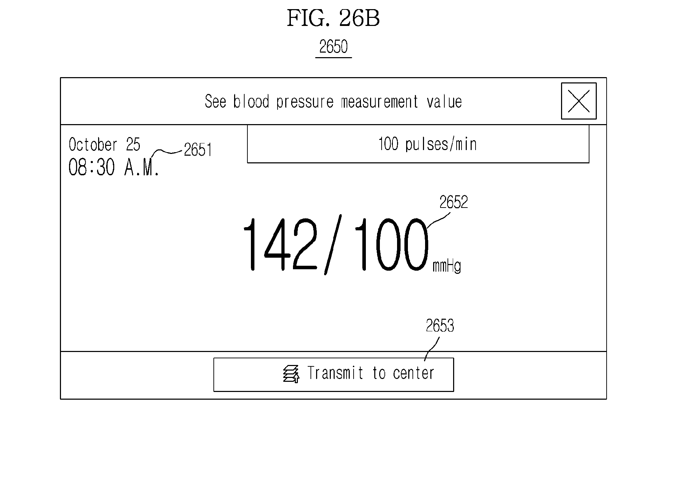

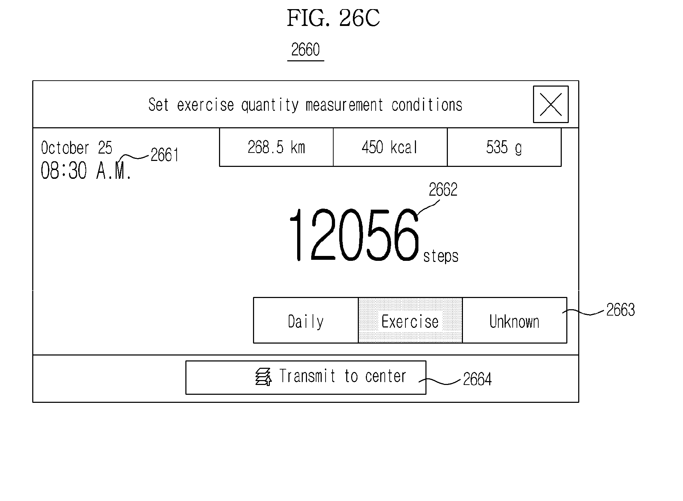

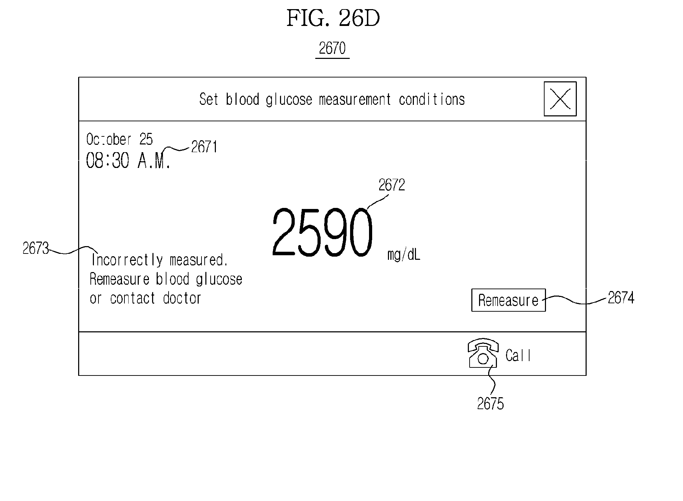

FIGS. 26A to 26D show measurement result views according to an exemplary embodiment.

In this exemplary embodiment, if a user measures bio-information in the immediate measurement views (for example, 2200, 2250 and 226) using the bio-information measurement sensor 160, the device 100 may display measurement result views 2600, 2650, 2660 and 2670.

The measurement result views 2600, 2650, 2660 and 2670 may include at least one selected from among bio-information measurement time (for example, 2611, 2651, 2661 and 2671), bio-information measurement values (for example, 2621, 2652, 2662 and 2672), user interfaces (for example, 2631 and 2663) to input measurement conditions through which bio-information is measured, and user interfaces (for example, 2641, 2653 and 2664) to transmit bio-information measurement information to the server 170.

In this exemplary embodiment, if the bio-information measured by the user is blood glucose, the user interface (for example, 2631) to input blood glucose measurement conditions may include at least one selected from among dawn, before a meal (empty stomach), after a meal, before going to bed and unknown. If the bio-information measured by the user is blood pressure, a user interface (not shown) to input blood pressure measurement conditions may include at least one selected from among daily, after exercise, after rising and unknown. If the bio-information measured by the user is exercise quantity, the user interface (for example, 2663) to input exercise quantity measurement conditions may include at least one selected from among daily, exercise and unknown.

In this exemplary embodiment, if the user selects the user interfaces (for example, 2641, 2653 and 2664) to transmit bio-information measurement information to the server 170, the bio-information measurement information measured by the user may be transmitted to the server 170 or the storage unit 140. For example, the bio-information measurement information may include at least one selected from among bio-information measurement date and time (for example, 2611, 2651, 2661 and 2671), bio-information measurement values (for example, 2621, 2652, 2662 and 2672), and bio-information measurement conditions (for example, 2631 and 2663).

In this exemplary embodiment, the bio-information measurement value measured by the user deviates from a specific range, the device 100 may display the view 2670 to request remeasurement of bio-information. For example, if the bio-information measurement value deviates from the limit of measurement values which may be measured from the patient or the limit of measurement values previously measured on a per patient basis, the view 2670 to request remeasurement of bio-information may be displayed. The limit of measurement values may be stored in the device 100 or the server 170 and changed by a doctor.

The view 2670 to request remeasurement of bio-information may include at least one selected from among remeasurement information (for example, 2673), a user interface 2674 for remeasurement and a user interface 2675 to contact a doctor. For example, the remeasurement information 2673 may include text, graphs and sound to request that the user perform remeasurement or contact the doctor. Also, the user interface 2675 to contact the doctor may include, without being limited to, a telephone call, e-mail and short message service (SMS).

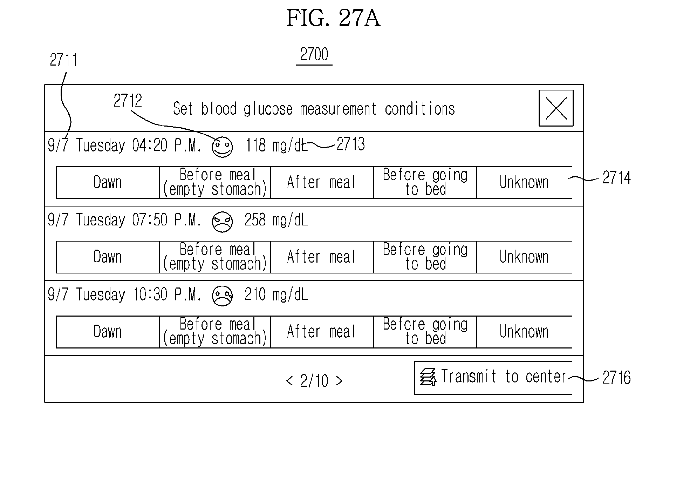

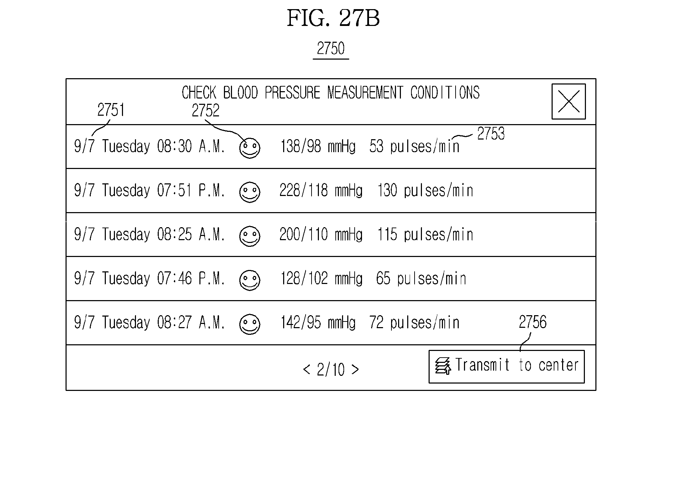

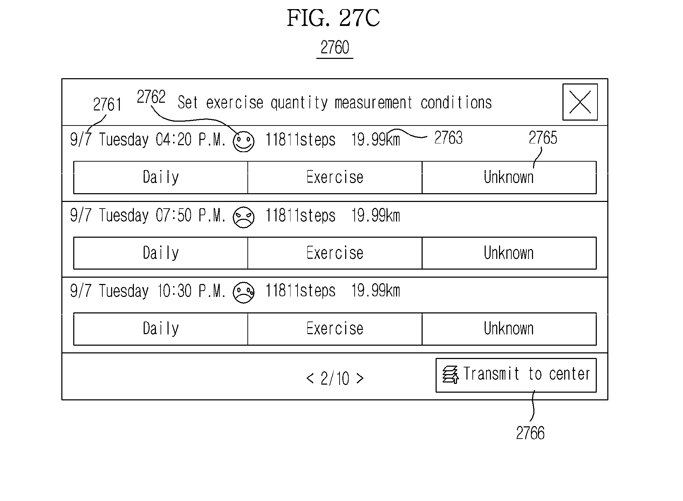

FIGS. 27A to 27C show untransmitted measurement value list views according to an exemplary embodiment.

In this exemplary embodiment, if a user selects the user interfaces (for example, 2112, 2132 and 2142) to transmit untransmitted measurement values of bio-information in the bio-information measurement views (for example, 2100, 2130 and 2140), the device 100 may display untransmitted measurement value list views 2700, 2750 and 2760.

The untransmitted measurement value list views 2700, 2750 and 2760 may include a plurality of untransmitted measurement values of bio-information. Also, the untransmitted measurement value list views 2700, 2750 and 2760 may include at least one selected from among measurement date and time (for example, 2711, 2751 and 2761) corresponding to measurement values, images (for example, 2712, 2752 and 2762) indicating a health state of the patient based on the measurement values, untransmitted measurement values (for example, 2713, 2753 and 2763), and measurement conditions (for example, 2714 and 2765) under which bio-information is measured.

In this embodiment, if the bio-information is blood glucose, the measurement condition (for example, 2714) may include at least one selected from among dawn, before a meal (empty stomach), after a meal, before going to bed and unknown. If the bio-information is exercise quantity, the measurement condition (for example, 2765) may include at least one selected from among daily, exercise and unknown.

In this embodiment, the untransmitted measurement value list views 2700, 2750 and 2760 may include user interfaces (for example, 2716, 2756 and 2766) to transmit bio-information measurement information to the server 170. Also, the untransmitted measurement value list views 2700, 2750 and 2760 may include a user interface (not shown) to select at least one untransmitted measurement value to be transmitted to the server 170 from the untransmitted measurement value list.

In this embodiment, the untransmitted measurement value list views 2700, 2750 and 2760 may include a user interface (not shown) to delete at least one untransmitted measurement value from the untransmitted measurement value list. The user may select the user interface (not shown) to delete the untransmitted measurement value to delete at least on untransmitted measurement value from the untransmitted measurement value list. Also, the user may select the user interfaces (for example, 2716, 2756 and 2766) to transmit other untransmitted measurement values, which are not deleted, to the server.

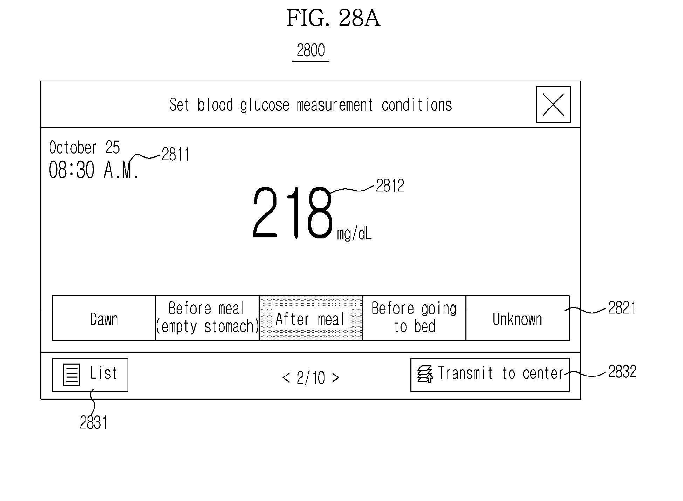

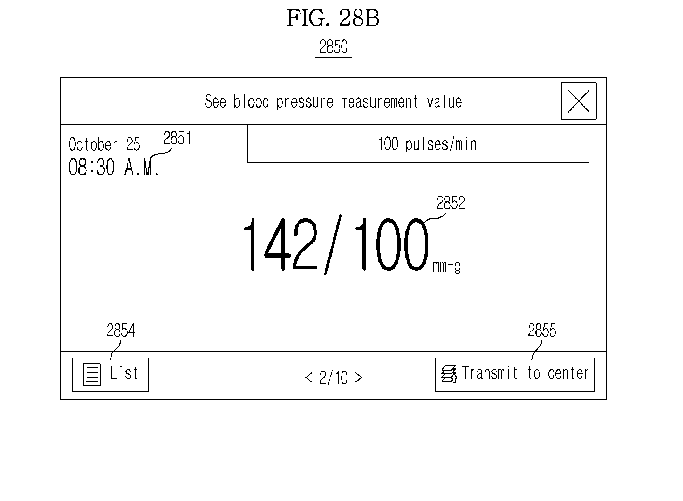

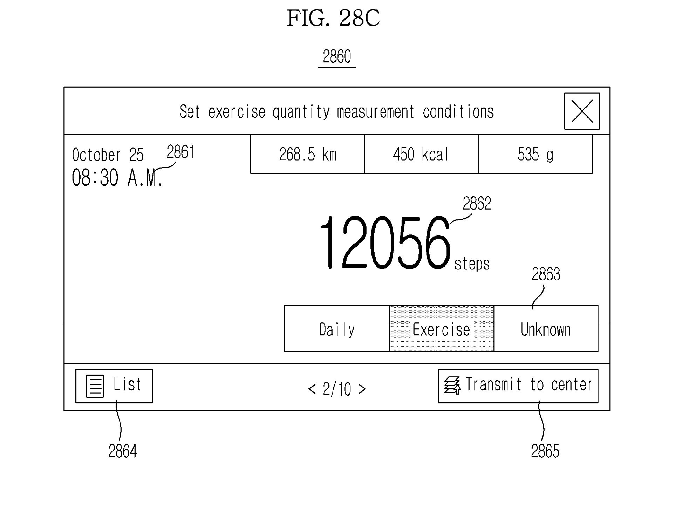

FIGS. 28A to 28C show untransmitted measurement value views according to an exemplary embodiment.

In this exemplary embodiment, if a user selects a measurement value in the untransmitted measurement value list views (for example, 2700, 2750 and 2760), the device 100 may display untransmitted measurement value views 2800, 2850 and 2860. The untransmitted measurement value views 2800, 2850 and 2860 may include at least one selected from among measurement date and time (for example, 2811, 2851 and 2861) when the untransmitted measurement values were measured, untransmitted measurement values (for example, 2812, 2852 and 2862), user interfaces (for example, 2821 and 2863) to input measurement conditions when untransmitted measurement values were measured, and user interfaces (for example, 2832, 2855 and 2865) to transmit untransmitted measurement values to the server 170 or the storage unit 140. Also, the untransmitted measurement value views 2800, 2850 and 2860 may further include user interfaces (for example, 2831, 2854 and 2864) to return to the untransmitted measurement value list views (for example, 2700, 2750 and 2760).

In this exemplary embodiment, the untransmitted measurement value views 2800, 2850 and 2860 may further include a user interface (not shown) to delete a displayed one of the untransmitted measurement values. If the user selects the user interface, the untransmitted measurement value views 2800, 2850 and 2860 may be switched to the untransmitted measurement value list views (for example, 2700, 2750 and 2760).

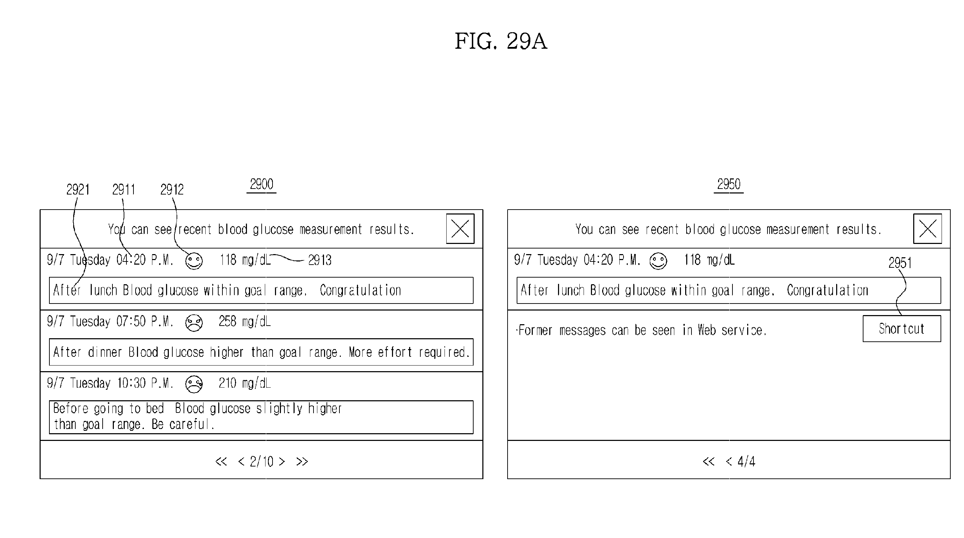

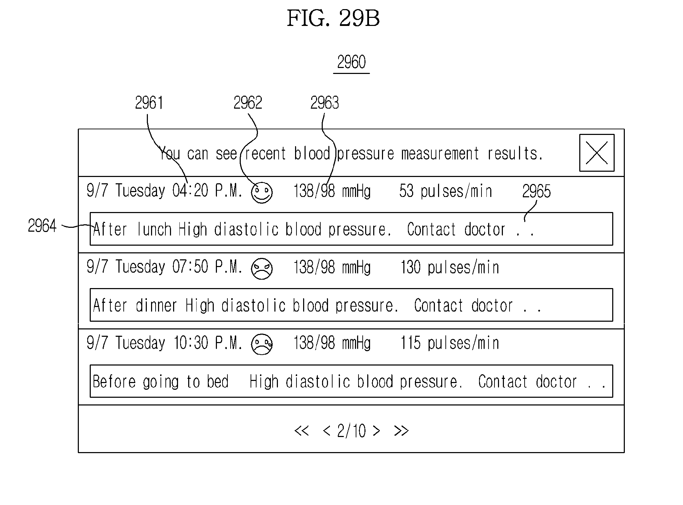

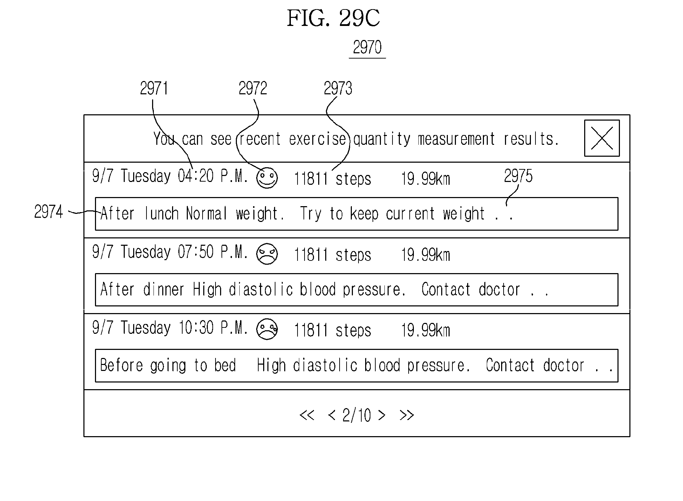

FIGS. 29A to 29C show recent measurement value list views according to an exemplary embodiment.

In this exemplary embodiment, if a user selects the user interfaces (for example, 2115, 2135 and 2145) to retrieve a list of bio-information measurement values which have been recently measured by the user in the bio-information measurement views (for example, 2100, 2130 and 2140), the device 100 may display recent measurement value list views 2900, 2950, 2960 and 2970.

The recent measurement value list views 2900, 2950, 2960 and 2970 may include a plurality of bio-information measurement values. In this case, the bio-information measurement values may include at least one selected from among measurement values stored in the server 170 or the storage unit 140 and untransmitted measurement values.

The recent measurement value list views 2900, 2950, 2960 and 2970 may include at least one selected from among measurement date and time (for example, 2911, 2961 and 2971) corresponding to measurement values, images (for example, 2912, 2962 and 2972) indicating a health state of the patient based on the measurement values, measurement conditions (for example, 2921, 2964 and 2974) under which bio-information is measured, bio-information measurement values (for example, 2913, 2963 and 2973), and feedback information (for example, 2965 and 2975) based on the measurement values. A piece of information may be selected from the feedback information (for example, 2965 and 2975) based on the measurement values. Alternatively, if the measurement values are transmitted to the server, the feedback information (for example, 2965 and 2975) based on the measurement values may be provided based on information input by a doctor. The feedback information (for example, 2965 and 2975) based on the measurement values may be provided as, without being limited to, text, graphs or sound.

In this exemplary embodiment, the recent measurement value list views 2900, 2950, 2960 and 2970 may provide bio-information measurement values within a predetermined period (for example, one month or one year). In this case, the recent measurement value list views 2900, 2950, 2960 and 2970 may include a user interface (for example, 2951) to display bio-information measurement values after the predetermined period. If the user interface (for example, 2951) is selected, the device 100 may acquire and provide the bio-information measurement values after the predetermined period from the server 170 or the storage unit 140.

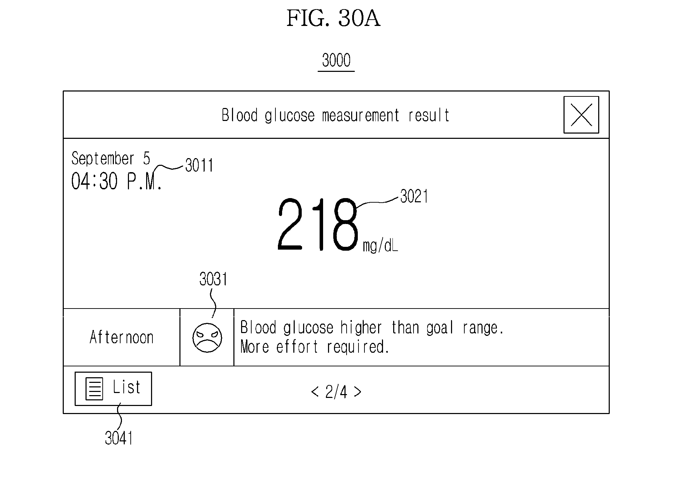

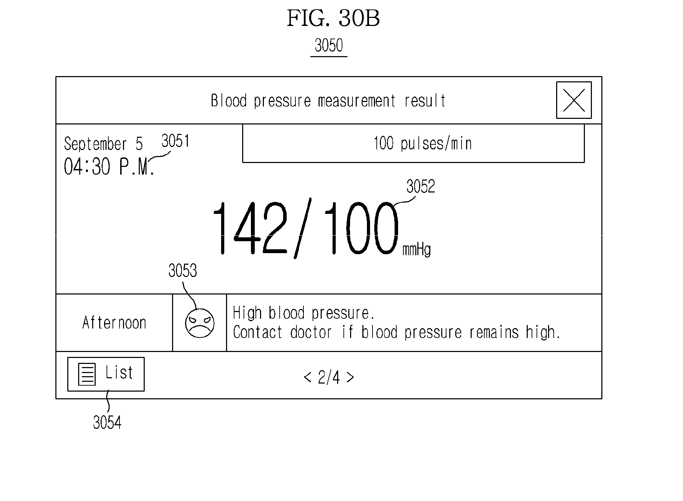

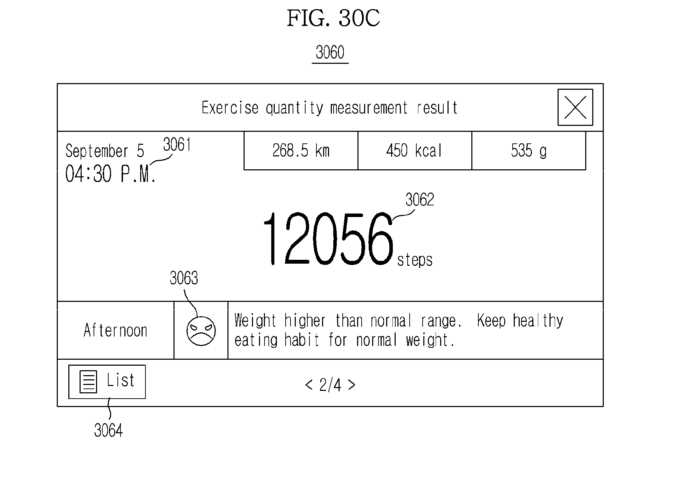

FIGS. 30A to 30C show recent measurement value views according to an exemplary embodiment.

In this exemplary embodiment, if a user selects an item from the recent measurement value list views (for example, 2900, 2950, 2960 and 2970), the device 100 may display recent measurement value views 3000, 3050 and 3060 including measurement value information of the selected item.

The recent measurement value views 3000, 3050 and 3060 may include date or time (for example, 3011, 3051 and 3061) when measurement values were measured, recent measurement values (for example, 3021, 3052 and 3062), and feedback information (for example, 3031, 3053 and 3063) based on the recent measurement values. If the user selects user interfaces (for example, 3041, 3054 and 3064), the device may display the recent measurement value list views (for example, 2900, 2950, 2960 and 2970).

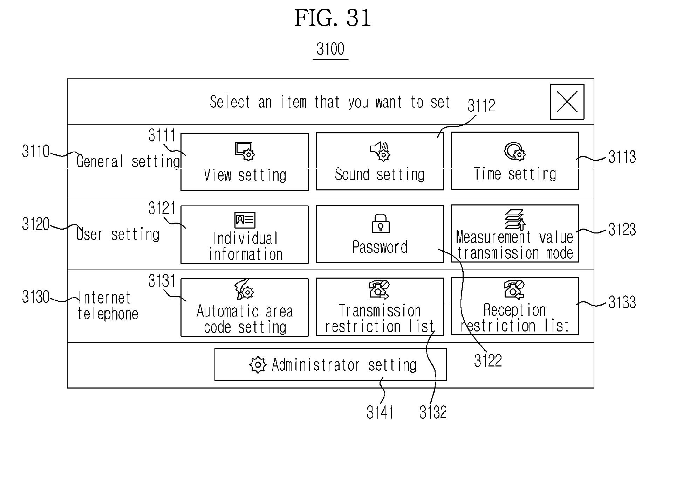

FIG. 31 shows an environment setting view according to an exemplary embodiment. In this exemplary embodiment, if a user selects the user interface (for example, 454) to set an environment in the main view (for example, 400), the device 10 may display an environment setting view 3100.

The environment setting view 3100 may include at least one selected from among a general setting item (for example, 3110) to set a general state provided through the view, a user setting item (for example, 3120) to set information related to the user, and an Internet telephone item (for example, 3130) related to an Internet telephone call.

In this exemplary embodiment, the general setting item (for example, 3110) may include at least one selected from a view setting user interface (for example, 3111 ) to set brightness of the view, a user interface (for example, 3112) to set sound output from the device 100, and a time setting user interface (for example, 3113) to set time of the device 100. The user setting item (for example, 3120) may include at least one selected from a user interface (for example, 3121) to set individual user information, a user interface (for example, 3122) to set a password necessary to access the health care system, and a user interface (for example, 3123) to set a mode in which bio-information measurement values are transmitted. The Internet telephone item (for example, 3130) may include at least one selected from a user interface (for example, 3131 ) to automatically set an area code upon making an Internet telephone call, a user interface (for example, 3132) to set a transmission restriction list, and a user interface (for example, 3133) to administer a reception restriction list.

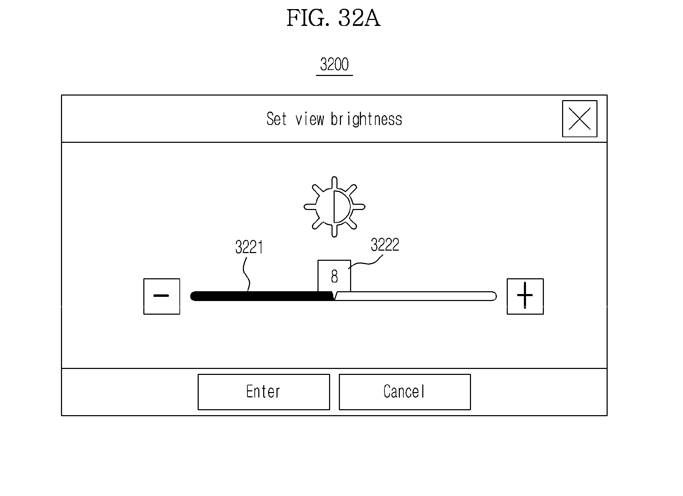

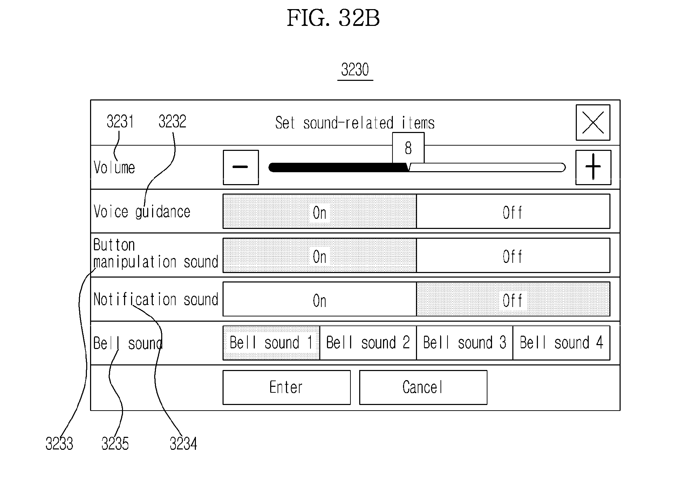

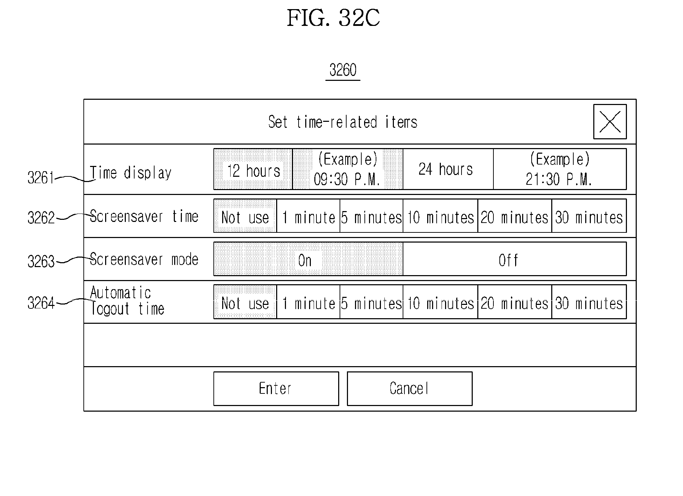

FIGS. 32A to 32C show general setting views according to an exemplary embodiment.

In this exemplary embodiment, if a user selects the view setting user interface (for example, 3111) to set brightness of the view from the general setting item (for example, 3110) of the environment setting view (for example, 3100), the device 100 may display a view brightness setting view 3200.

In the view brightness setting view 3200, the user may adjust the position of a bar (for example, 3221) indicating view brightness to adjust the brightness of the view. The view 3220 may provide information (for example, 3222) on the view brightness based on the position of the bar as at least one selected from among a figure, text, graphs and sound.

In this exemplary embodiment, if the user selects the view setting user interface (for example, 3112) to set sound output from the device 100 from the general setting item (for example, 3110) of the environment setting view (for example, 3100), the device 100 may display a sound setting view 3230.

The sound setting view 3230 may include at least one selected from among an item (for example, 3231) to adjust the volume of sound, an item (for example, 3232) to turn on or off voice guidance, an item (for example, 3233) to turn on or off a button manipulation sound, an item (for example, 3234) to turn on or off a notification sound, and an item (for example, 3235) to decide a bell sound.

In this exemplary embodiment, if the user selects the time setting user interface (for example, 3113) to set time of the device 100 from the general setting item of the environment setting view (for example, 3100), the device 100 may display a time setting view 3260.

The time setting view 3260 may include at least one selected from among a time display item (for example, 3261) to display time on a twelve-hour basis or on a twenty four-hour basis, a screensaver time item (for example, 3262) to set screensaver operation time of the device, a screensaver mode item (for example, 3263) to turn on or off a screensaver mode, and an automatic logout time item (for example, 3264) to automatically perform user logout when there is no user input to the device 100 for a predetermined time.

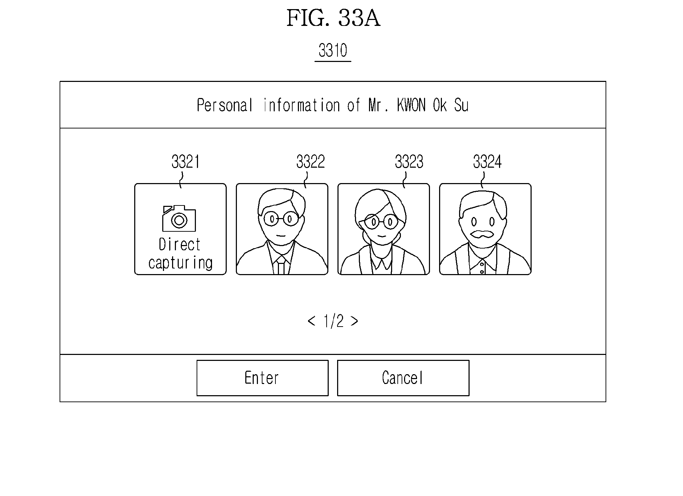

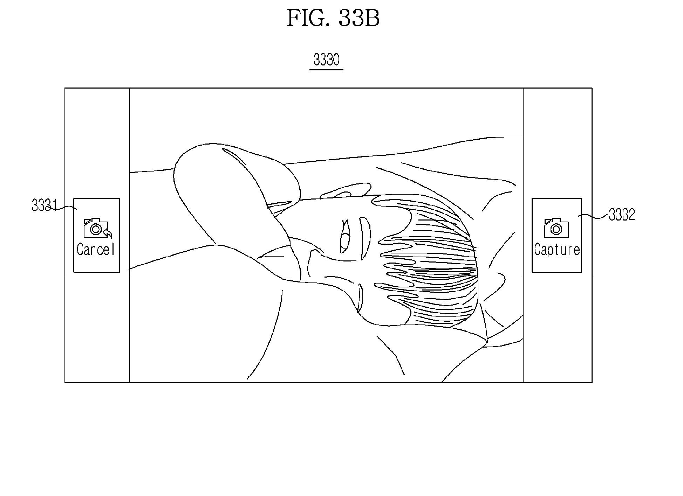

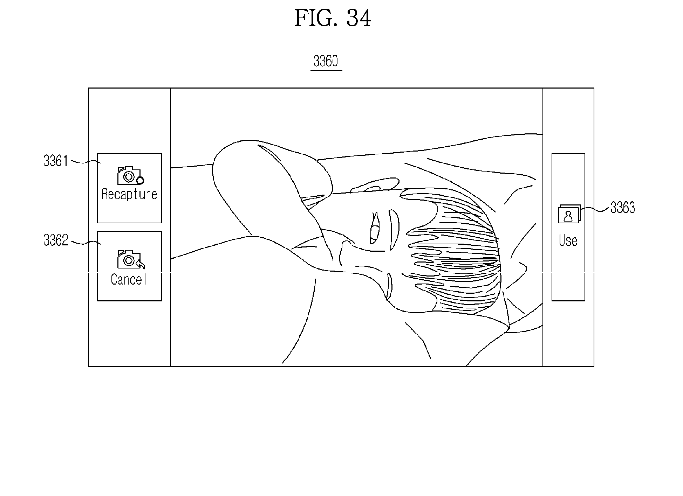

FIGS. 33A to 34 show individual user information setting views according to an exemplary embodiment.

In this exemplary embodiment, if a user selects the user interface (for example, 3121) to set individual user information from the user setting item (for example, 3120) of the environment setting view (for example, 3100), the device 100 may display an individual user information setting view 3310.

The individual user information setting view 3310 may include a plurality of images (for example, 3322 to 3324). The user may select one of the images (for example, 3322 to 3324) and set the selected one as an image representing the user. The images may be stored in the storage unit 140 or the storage unit of the server 170.

In this embodiment, the individual user information setting view 3310 may include a user interface (for example, 3321) for the user to directly capture an image representing the user. The device 100 may include a camera to capture images. Alternatively, the device 100 may capture an image using an external camera and receive the captured information through the communication unit 150.

If the user selects the user interface (for example, 3321), the camera may provide a user capture view 3330. Alternatively, the user capture view 3330 may be provided through the screen 110 of the device 100. If the user decides a subject through the user capture view (for example, 3330) and captures an image of the subject using a user interface (for example, 3332), the device may store the image of the subject. Meanwhile, if the user selects a user interface (for example, 3331) to cancel capturing, the device 100 may display the individual user information setting view (for example, 3310) before capturing.

If the user captures an image using the user interface (for example, 3332), the camera may provide a captured picture application view (for example, 3360). Alternatively, the captured picture application view (for example, 3360) may be provided through the screen 110 of the device 100. The captured picture application view (for example, 3360) may include a user interface (for example, 3361) to recapture the captured picture and a user interface (for example, 3362) to cancel capturing. The user may select an interface (for example, 3363) to use the captured picture as an image representing the user.

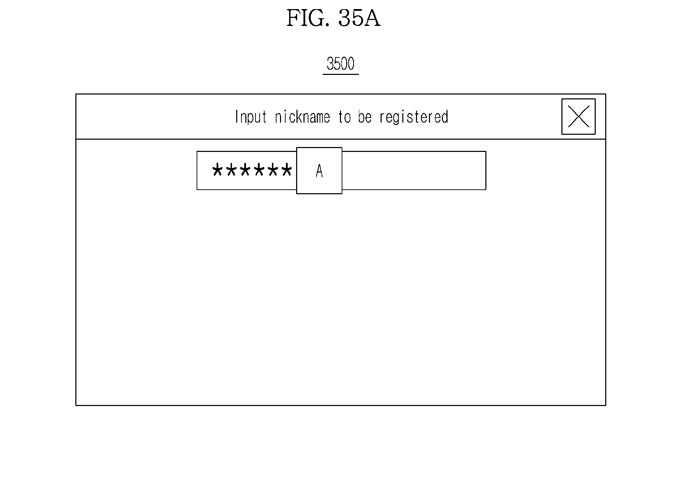

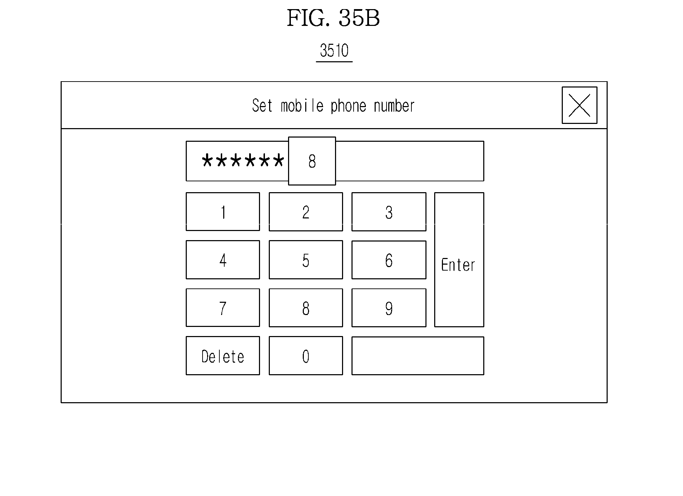

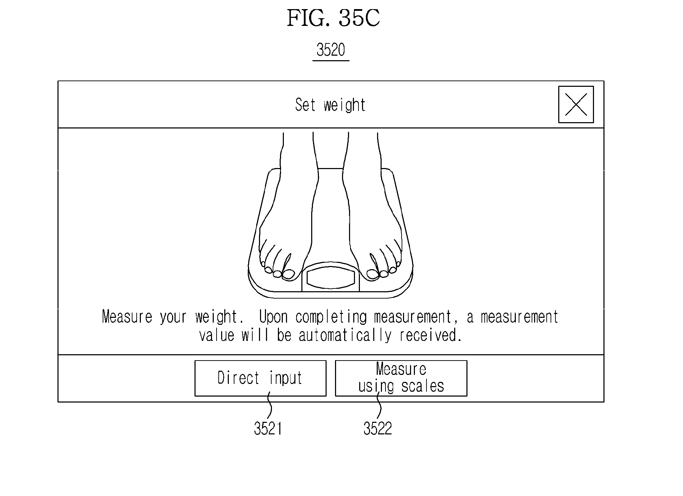

FIGS. 35A to 35C show individual user information setting views according to an exemplary embodiment.

In this exemplary embodiment, if a user selects the user interface (for example, 3121) to set individual user information from the user setting item (for example, 3120) of the environment setting view (for example, 3100), the device 100 may display individual user information setting views 3500, 3510 and 3520.

In this exemplary embodiment, the individual user information setting views 3500, 3510 and 3520 may include a user nickname input view (for example, 3500). For example, the user may perform login using the nickname or receive or transmit information related to health of a patient from or to a doctor or an acquaintance of the patient.

In this exemplary embodiment, the individual user information setting views 3500, 3510 and 3520 may include a user telephone number input view (for example, 3510). The user may receive or transmit information related to health from or to a doctor or an acquaintance of the patient through the changed mobile phone number. In this exemplary embodiment, the individual user information setting views 3500, 3510 and 3520 may include a user bio-information input view (for example, 3520). For example, user bio-information may be weight of the user. The device 100 receives weight of the user through a user interface (for example, 3521) for the user to directly input weight, or weight of the user using the bio-information measurement sensor 160 may be acquired through a user interface (for example, 3522) using the bio-information measurement sensor 160.

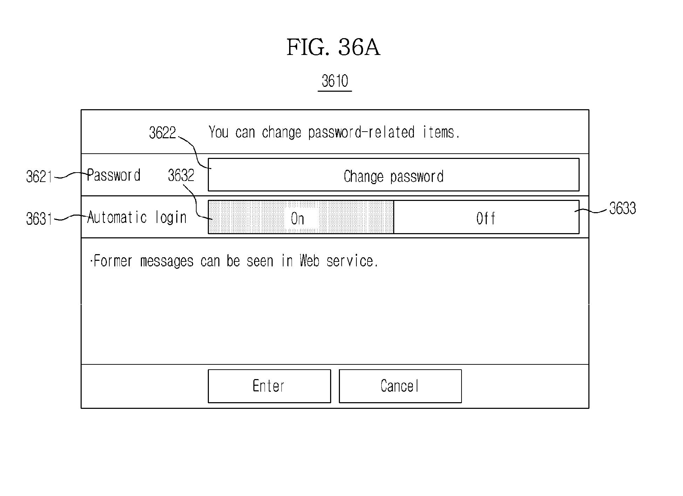

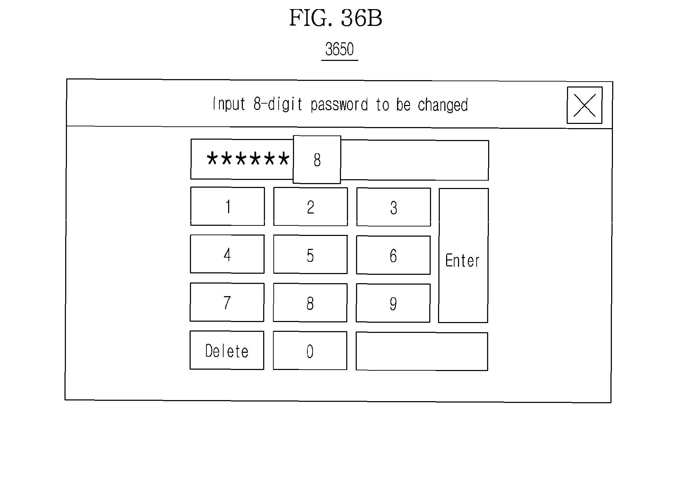

FIGS. 36A and 36B show password setting views according to an exemplary embodiment.

In this exemplary embodiment, if a user selects the user interface (for example, 3122) to set a password necessary to access the health care system from the user setting item (for example, 3120) of the environment setting view (for example, 3100), the device 100 may display a password setting view 3610.

In this exemplary embodiment, the password setting view 3610 may include at least one selected from among an item (for example, 3621) to change a password and an item (for example, 3622) to decide whether automatic login is to be performed 3631, 3632. The user may change the password through a password change user interface (for example, 3622) of the item (for example, 3621). Alternatively, the user may decide whether a password input view is to be provided upon performing automatic login through a turn-on user interface 3622 or a turn-off user interface 3633 of the item (for example, 3621).

In this exemplary embodiment, if the user selects the password change user interface (for example, 3622), the device 100 may display a password change view (for example, 3650). For example, the user may change the password using numerical keys in the view (for example, 3650) and store the changed password. The device 100 may confirm whether the password input by the user upon performing login coincides with the changed password, and access the health care system according to the confirmation result.

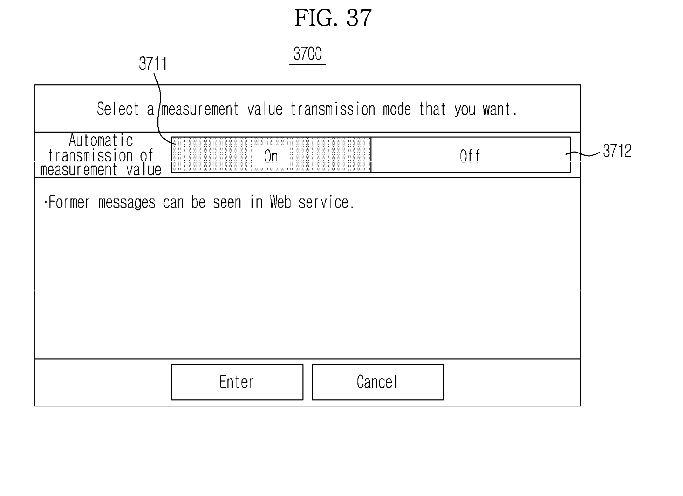

FIG. 37 shows a measurement value transmission mode setting view according to an exemplary embodiment.

In this exemplary embodiment, if a user selects the user interface (for example, 3123) to set a mode in which the measured bio-information measurement values are transmitted from the user setting item (for example, 3120) of the environment setting view (for example, 3100), the device 100 may display a measurement value transmission mode setting view 3700.

If the user selects a turn-on user interface (for example, 3711) in the measurement value transmission mode setting view 3700, the device 100 may automatically transmit the bio-information measurement values to the server 170 or the storage unit 140. If the user selects a turn-off user interface (for example, 3712), the device 100 may transmit the bio-information measurement values to the server 170 or the storage unit 140 according to additional transmission request input of the user after the measurement of bio-information.

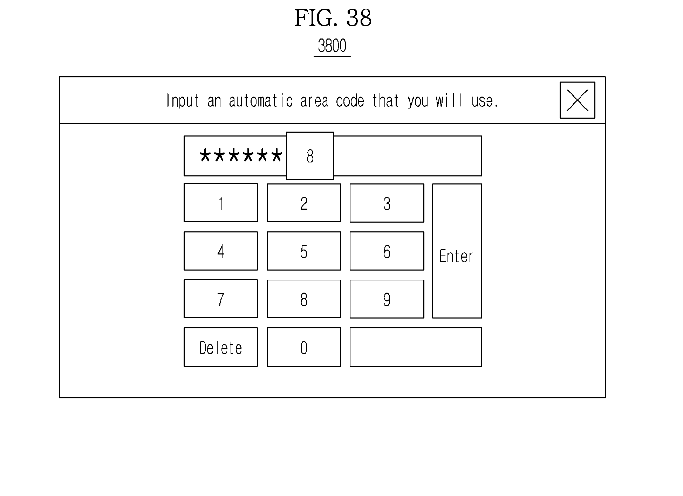

FIG. 38 shows an automatic area code setting view according to an exemplary embodiment.

In this exemplary embodiment, if a user selects the user interface (for example, 3131) to set an automatic area code from the Internet telephone item 3130 of the environment setting view (for example, 3100), the device 100 may display an automatic area code setting view 3800.

If the user inputs an area code to set the area code in the automatic area code setting view 3800, the device may automatically provide the set area code in the telephone number input view.

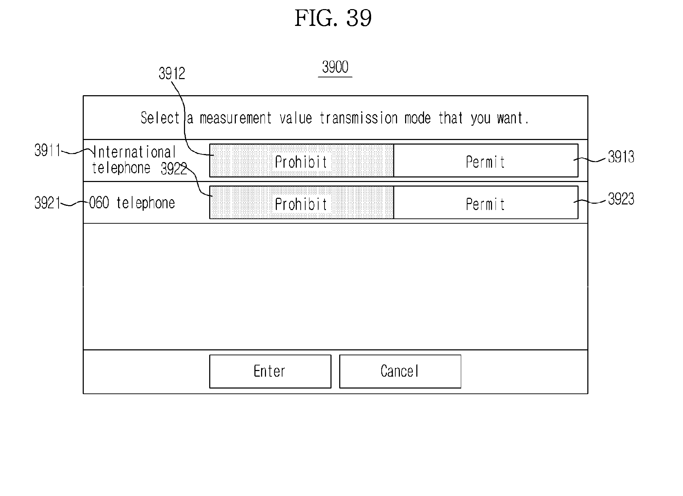

FIG. 39 shows a transmission restriction list setting view according to an exemplary embodiment.

In this exemplary embodiment, if a user selects the user interface (for example, 3132) to set a transmission restriction list from the Internet telephone item (for example, 3130) of the environment setting view (for example, 3100), the device 100 may display a transmission restriction list setting view 3900.

For example, the transmission restriction list setting view 3900 may include an international telephone transmission restriction item (for example, 3911) and a spam telephone transmission restriction item 3921 to restrict, for example, 060 telephone transmission. The transmission restriction list setting view 3900 may include a user interface (for example, 3912) to prohibit international telephone transmission or a user interface (for example, 3913) to permit international telephone transmission. Also, the transmission restriction list setting view 3900 may include a user interface (for example, 3922) to prohibit spam telephone transmission or a user interface (for example, 3923) to permit spam telephone transmission.

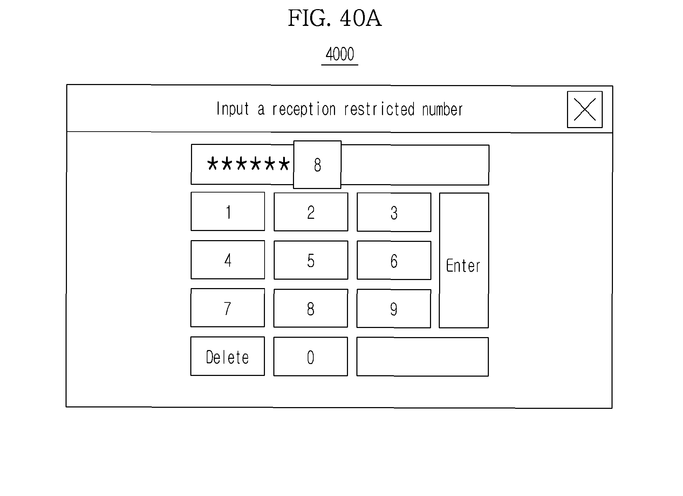

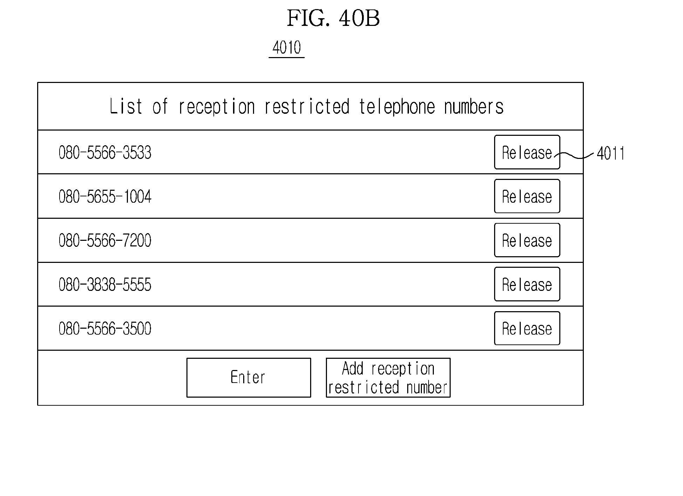

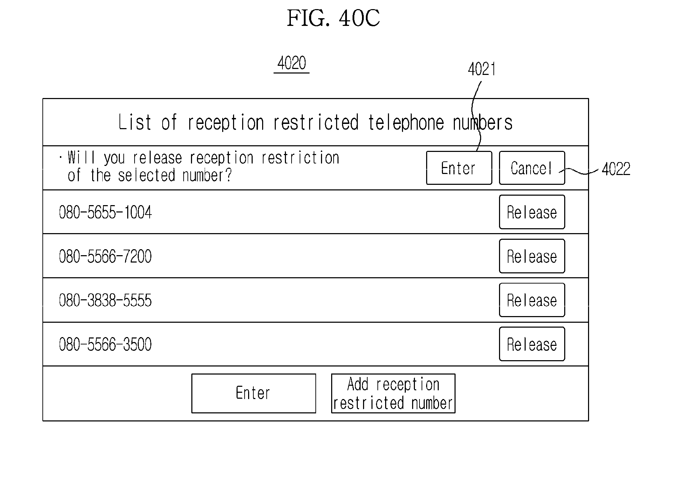

FIGS. 40A to 40C show reception restriction list setting views according to an exemplary embodiment.

In this exemplary embodiment, if a user selects the user interface (for example, 3133) to set a reception restriction list from the Internet telephone item (for example, 3130) of the environment setting view (for example, 3100), the device 100 may display reception restriction list setting views 4000, 4010 and 4020.

The reception restriction list setting views 4000, 4010 and 4020 may include a reception restricted number input view (for example, 4000). The user may input a reception restricted number through the view 4000. Upon receiving a telephone call having the reception restricted number, the device 100 may interrupt reception of the telephone call or transmit a stored voice or text message.

The reception restriction list setting views 4000, 4010 and 4020 may include reception restricted number release views (for example, 4010 and 4020). In the view 4010, the user may select a user interface (for example, 4011) to release the reception restricted number. Meanwhile, if the user selects a user interface (for example, 4011) to release the reception restricted number, the device 100 may further provide a view (for example, 4020) to confirm whether the reception restricted number is released. The user may select a user interface (for example, 4021) to confirm reception restriction or a user interface (for example, 4022) to cancel reception restriction so as to restrict the reception of the reception restricted number or to cancel the reception restriction.

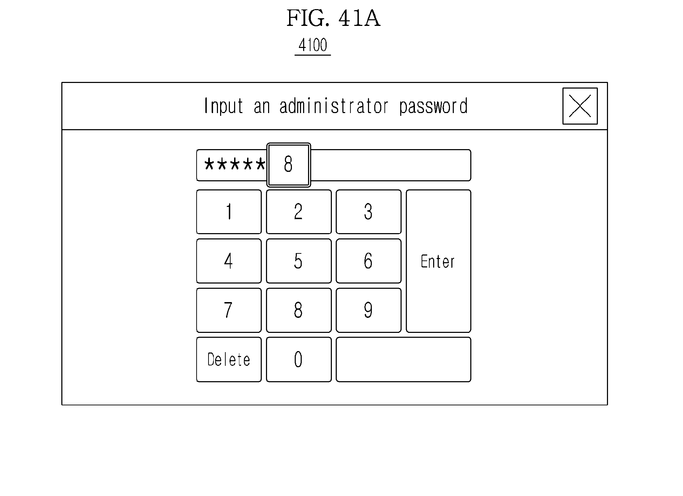

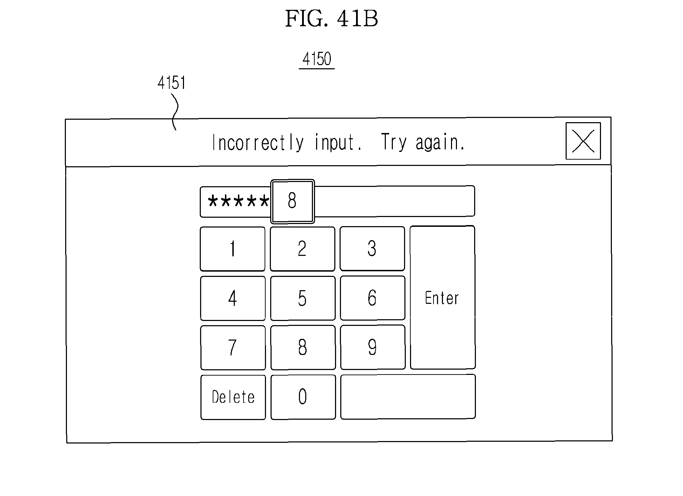

FIGS. 41A and 41B show administrator password input views according to an exemplary embodiment.

In this exemplary embodiment, if a user selects an administrator setting user interface (for example, 3141 in FIG. 31) in the environment setting view (for example, 3100), the device 100 may display administrator password input views 4100 and 4150.

In the administrator password input view 4100, the user may input an administrator password to enter an administrator mode. If the user incorrectly inputs an administrator password, the device 100 may provide information (for example, 4151) to notify incorrect input of the administrator password in at least one form selected from among text, sound and graphs.

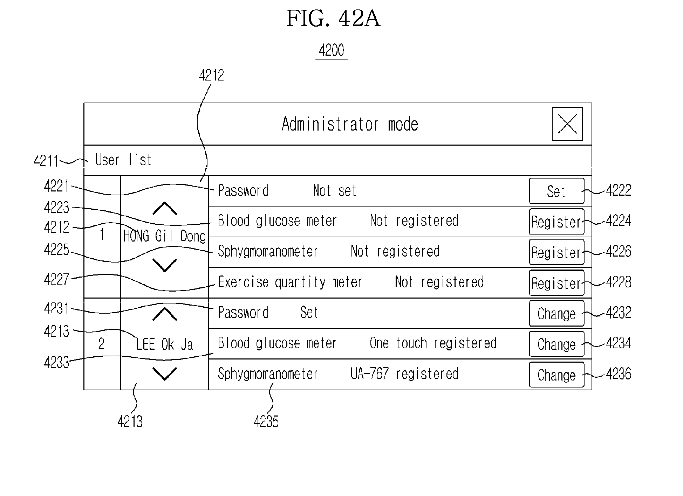

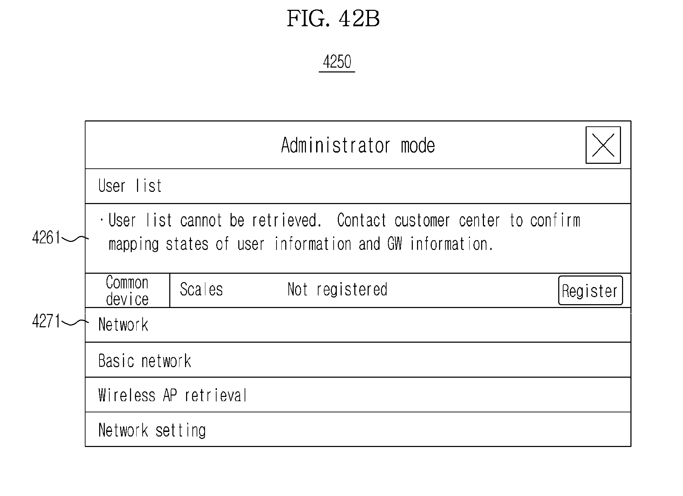

FIGS. 42A and 42B show administrator mode views according to an exemplary embodiment.

In this exemplary embodiment, if a user (for example, the administrator) selects the administrator setting user interface (for example, 3141) in the environment setting view (for example, 3100), the device 100 may display administrator mode views 4200 and 4250. Alternatively, if the administrator password input by the administrator in the administrator password input view 4100 coincides with an administrator password stored in the device 100, the device 100 may display the administrator mode views 4200 and 4250.

The administrator mode views 4200 and 4250 may include a list (for example, 4211) of users who use the device 100. For example, the user list (for example, 4211) may include information, such as user identification information (for example, 4212, 4213), a user password (for example, 4221) and bio-information measurement sensors (for example, 4223, 4225 and 4227). The bio-information measurement sensors (for example, 4223, 4225 and 4227) provided in the user list may include, without being limited to, a blood glucose meter, a sphygmomanometer and an exercise quantity meter.

If there is no registered user who uses the device 100 or information on a user who uses the device 100 is not acquired, the device 100 may provide information (for example, 4261) indicating that there is no user list to be displayed.

In this exemplary embodiment, the administrator mode views 4200 and 4250 may include a network item (for example, 4271) to set or change a network, via which the bio-information measurement sensors 160 are connected to the device 100 or the device 100 is connected to the server 170. The user may set a network for connection of the device 100 through the network item (for example, 4271) so that the device is connected to the bio-information measurement sensors 160 or the server 170 via the set network.

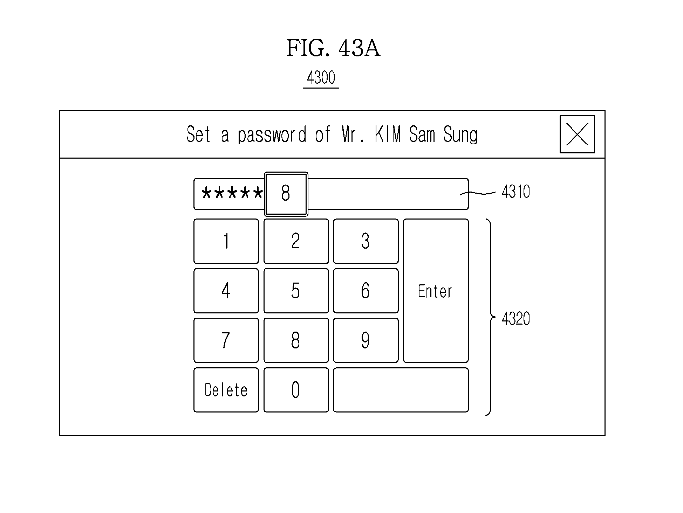

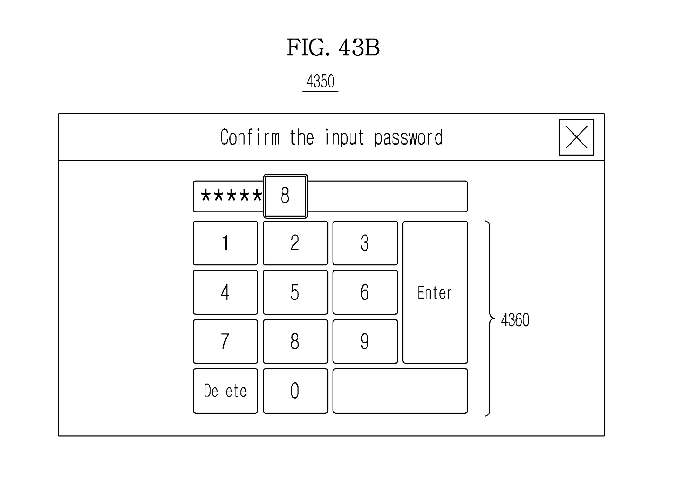

FIGS. 43A and 43B show user password setting views according to an exemplary embodiment.

In this embodiment, if an administrator selects user interfaces (for example, 4222 and 4232 in FIG. 42A) to set a user password in the administrator mode view 4200, the device 100 may display user password setting views 4300 and 4350.

In the user password setting view 4300, the user may input a password through a user interface (for example, 4320). At this time, visual feedback (for example, 321 and 322) related to at least one figure of the password input by the user may be provided to a password display part (for example, 4310). Also, in the user password confirmation view 4350, the user may confirm the input password through a user interface (for example, 4360).

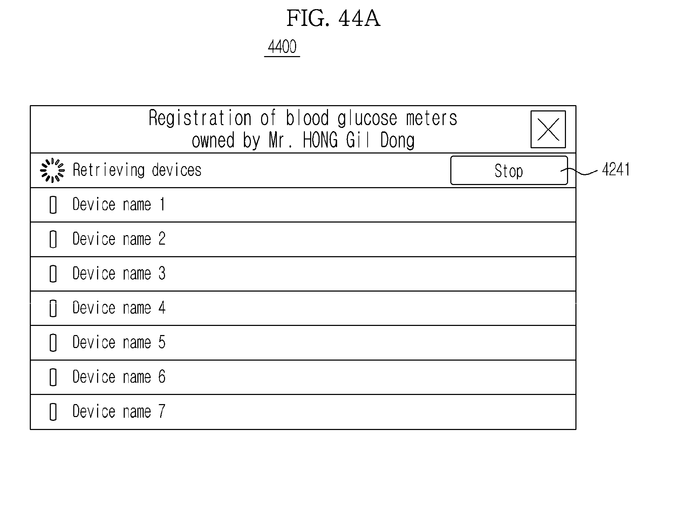

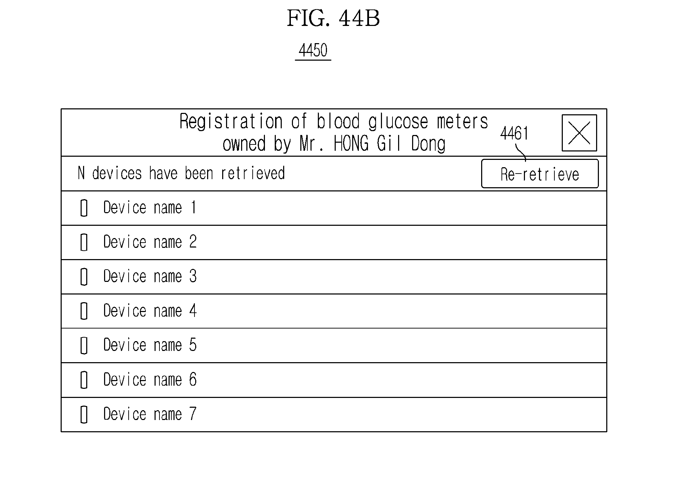

FIGS. 44A and 44B show bio-information measurement sensor registration views according to an exemplary embodiment.

In this exemplary embodiment, if an administrator selects bio-information measurement sensor registration user interfaces (for example, 4224, 4226 and 4228 in FIG. 42A) to set user bio-information measurement sensors (for example, 4223, 4225 and 4227) in the administrator mode view 4200, the device 100 may display bio-information measurement sensor registration views 4400 and 4450 to register bio-information measurement sensors.

In the bio-information measurement sensor registration views 4400 and 4450, the device 100 may retrieve bio-information measurement sensors 160 which may be connected to the device 100 and display the retrieved bio-information measurement sensors 160.

In this exemplary embodiment, if a user selects a user interface (for example, 4241) to interrupt the retrieval of the bio-information measurement sensors, the device 100 may interrupt the retrieval of the bio-information measurement sensors 160. If the user selects a user interface (for example, 4461) to request re-retrieval of the bio-information measurement sensors 160, the device 100 may re-retrieve bio-information measurement sensors 160 which may be connected to the device 100 and display the retrieved bio-information measurement sensors 160.

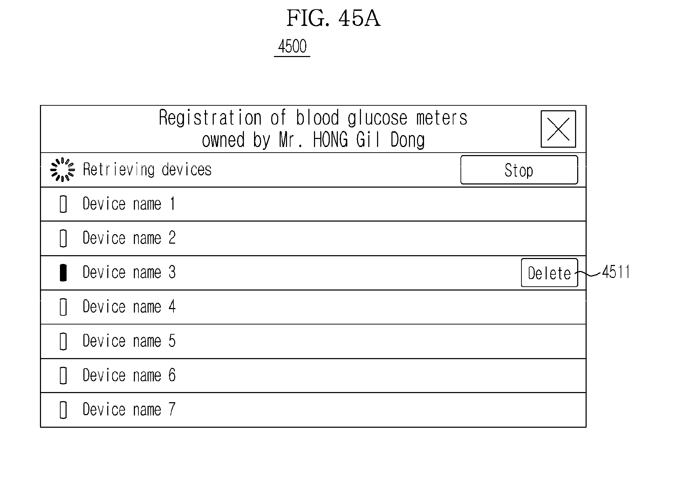

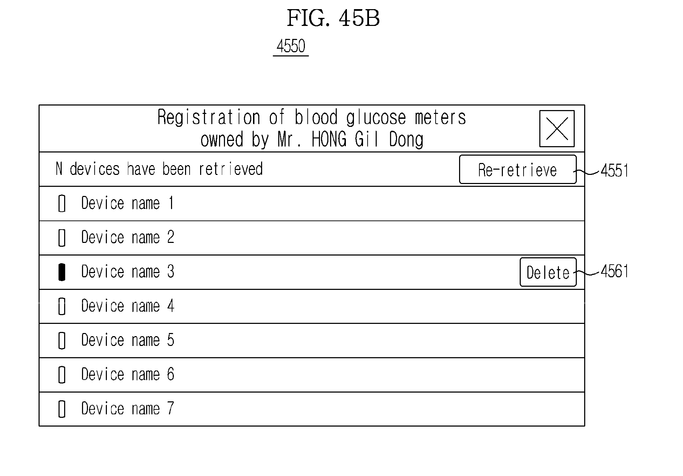

FIGS. 45A and 45B show bio-information measurement sensor change views according to an exemplary embodiment.

In this exemplary embodiment, if an administrator selects bio-information measurement sensor change user interfaces (for example, 4232, 4234 and 4236 in FIG. 42A) to change user bio-information measurement sensors (for example, 4231, 4233 and 4235 in FIG. 42A) in the administrator mode view 4200, the device 100 may display bio-information measurement sensor change views 4500 and 4550 to change the registered bio-information measurement sensors.

In the bio-information measurement sensor change views 4500 and 4550, the device 100 may re-retrieve bio-information measurement sensors 160 which may be connected to the device 100 and display the retrieved bio-information measurement sensors 160.

In this exemplary embodiment, if there is a bio-information measurement sensor 160 which is not desired to be connected to the device 100 among the retrieved bio-information measurement sensors 160, a user may select deletion user interfaces (for example, 4511 and 4561) to release connection of the corresponding bio-information measurement sensor. If the user selects a user interface (for example, 4551) to request re-retrieval of the bio-information measurement sensors 160, the device 100 may re-retrieve bio-information measurement sensors 160 which may be connected to the device 100 and display the retrieved bio-information measurement sensors 160.

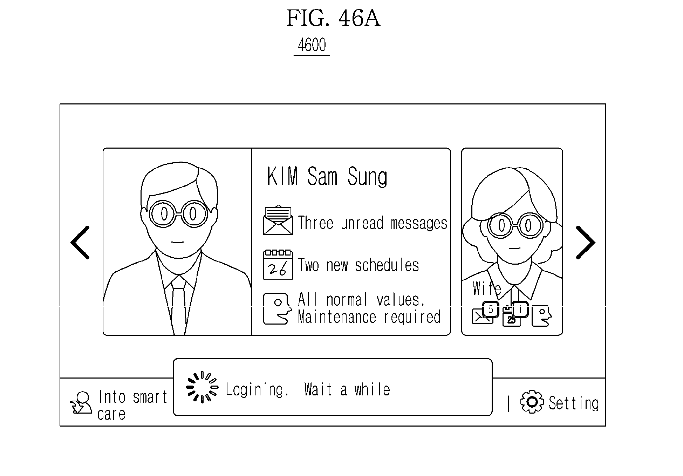

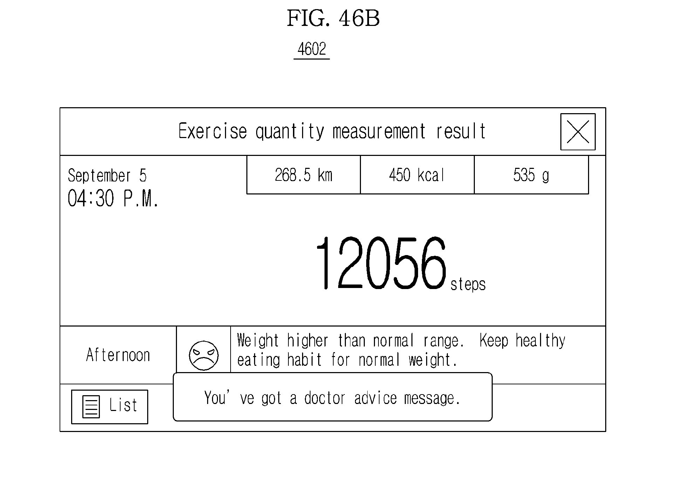

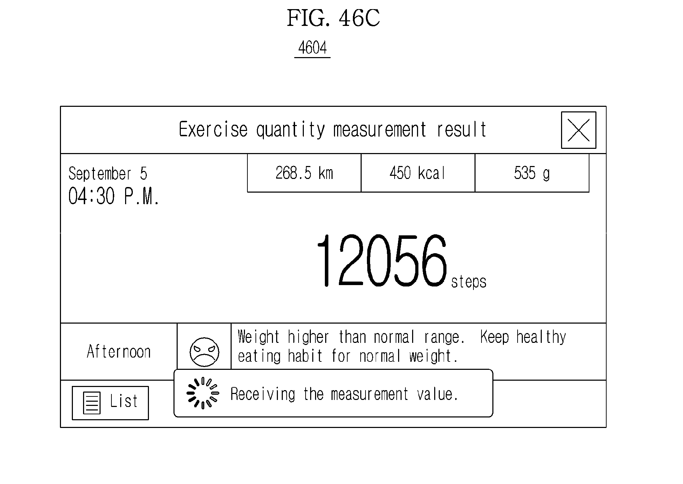

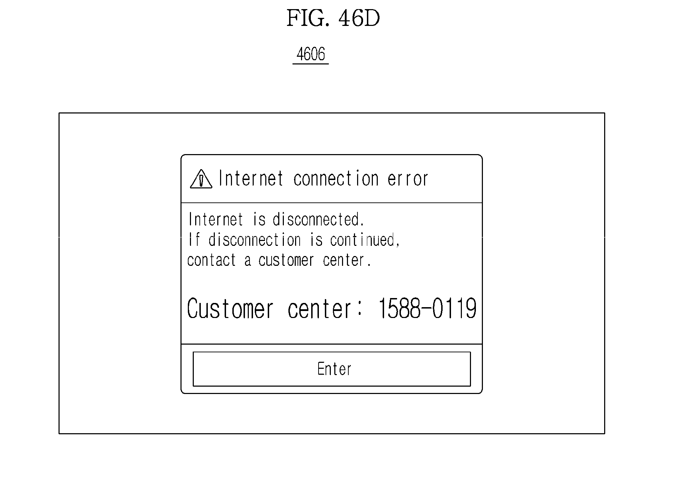

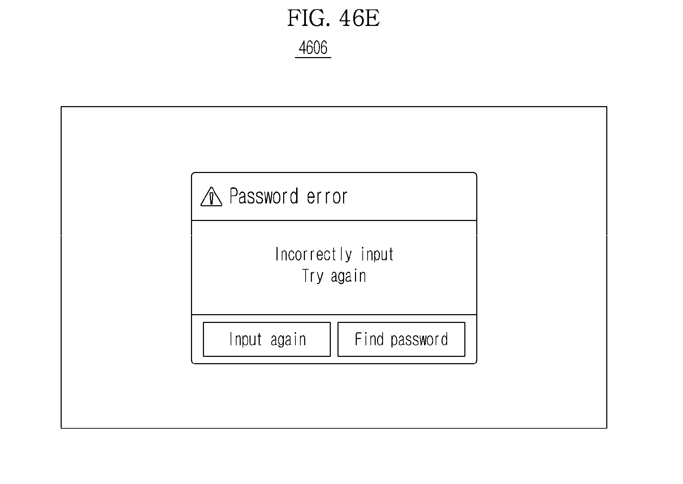

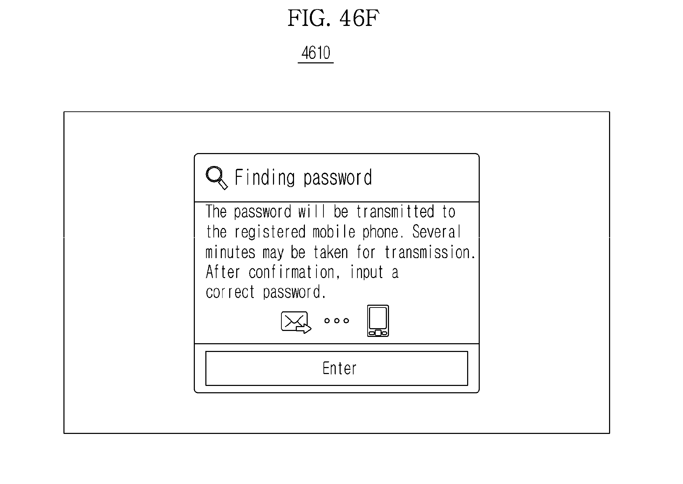

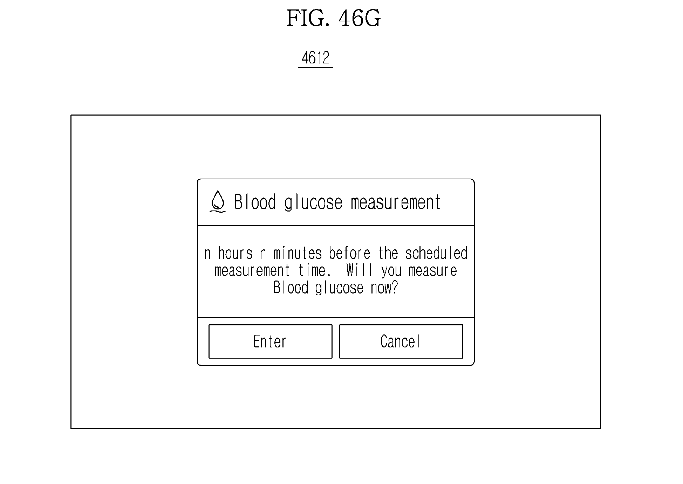

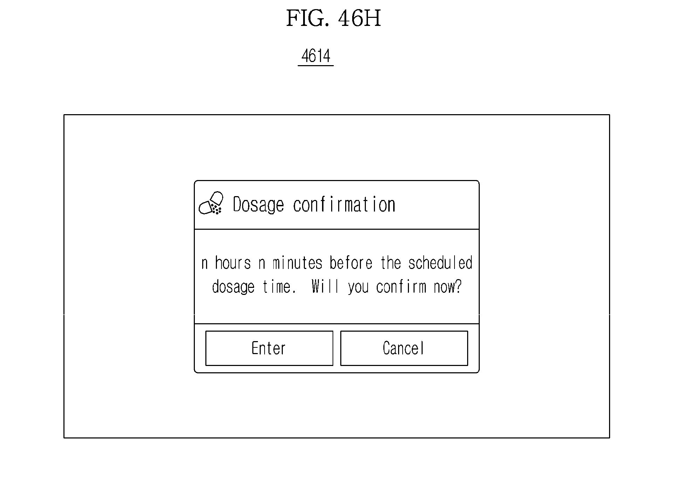

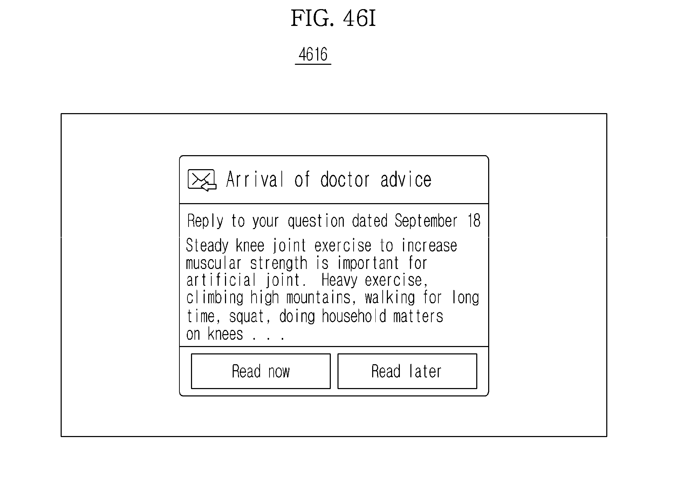

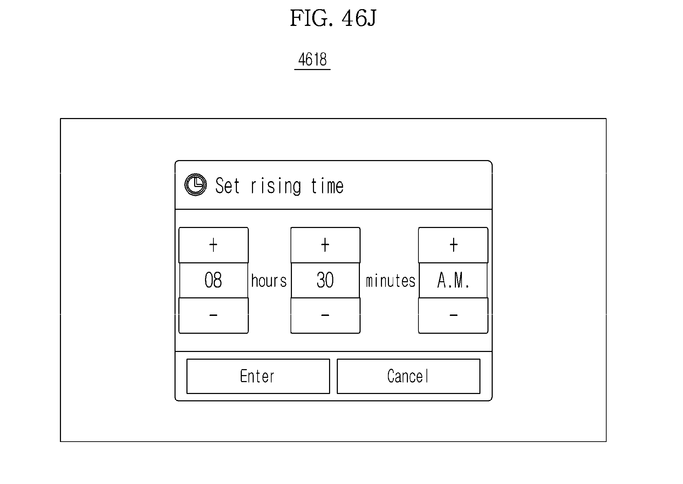

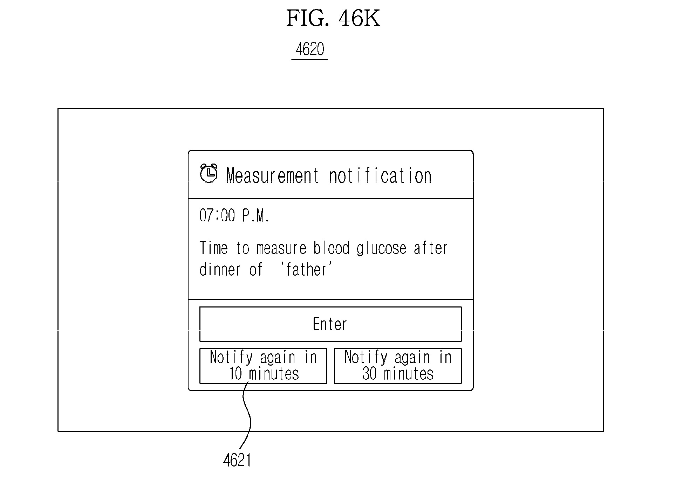

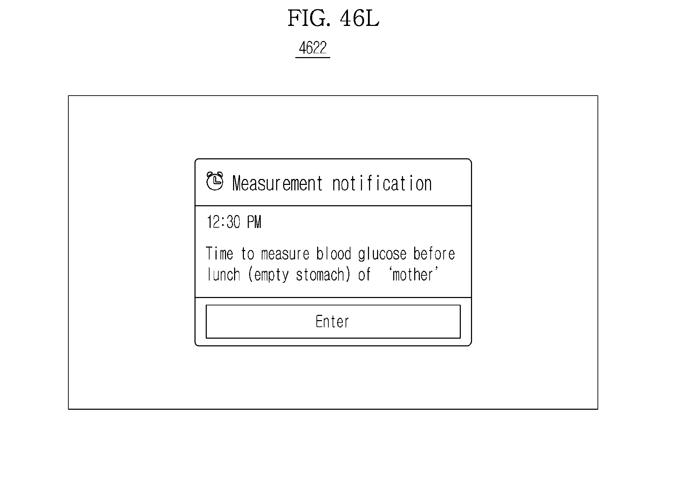

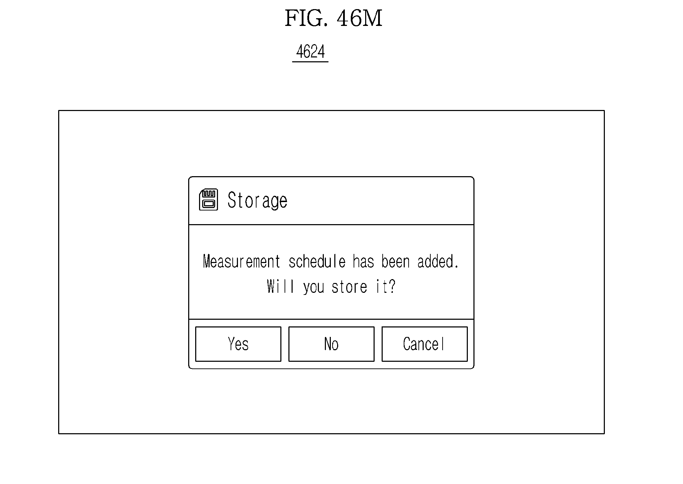

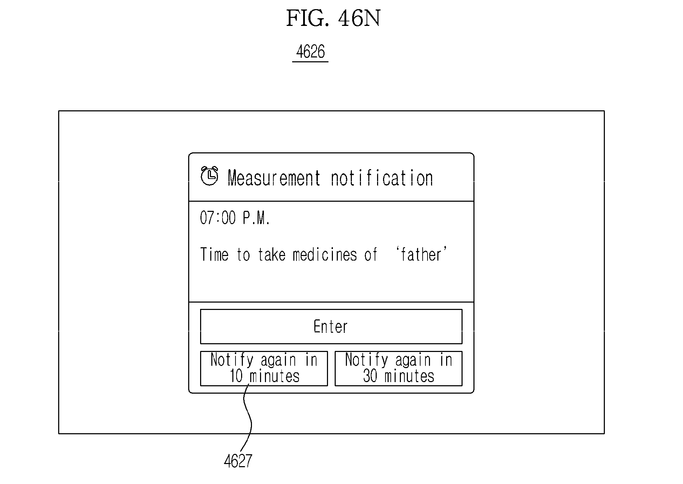

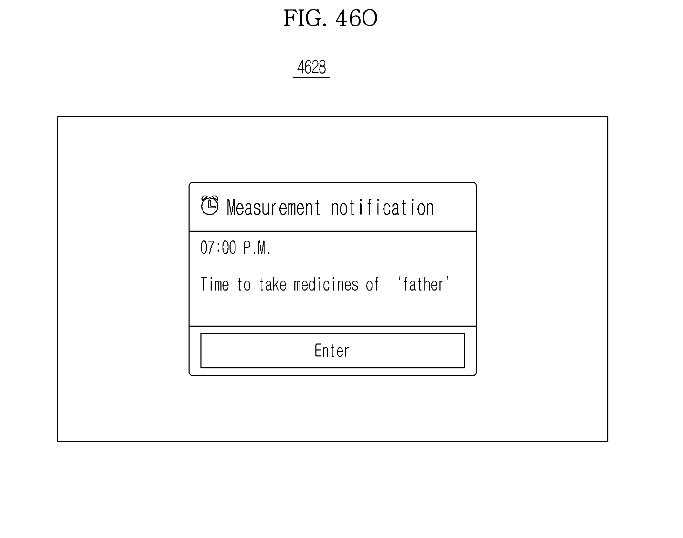

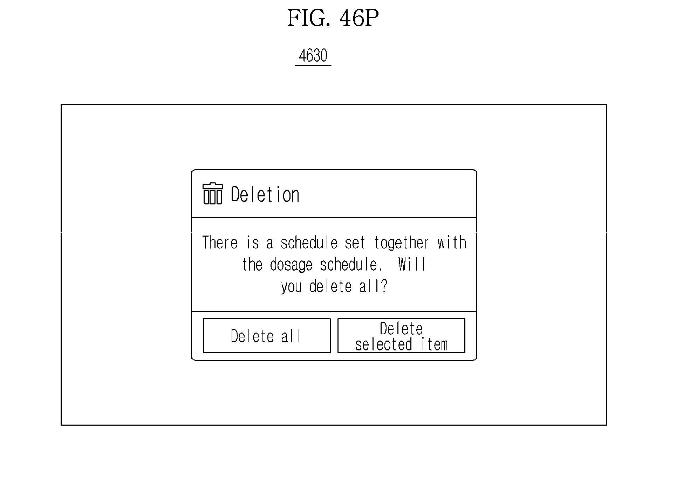

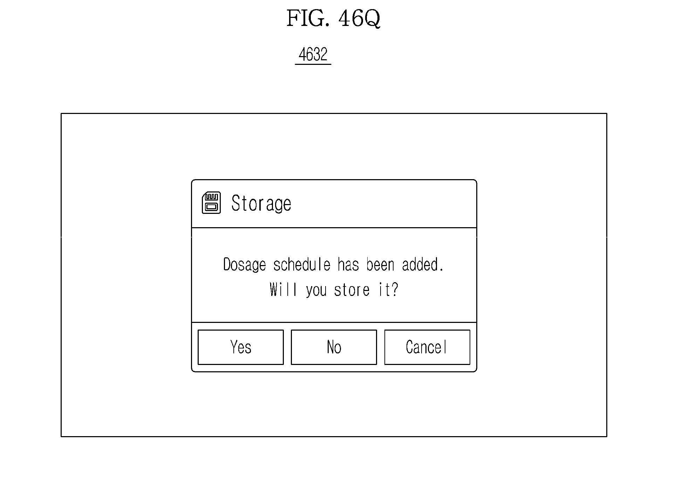

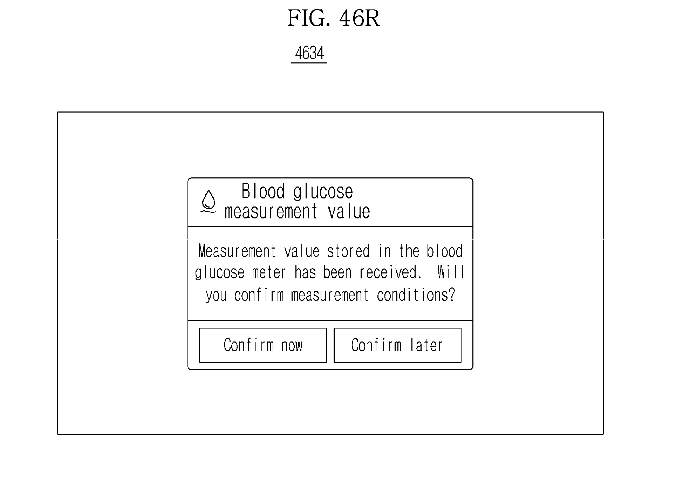

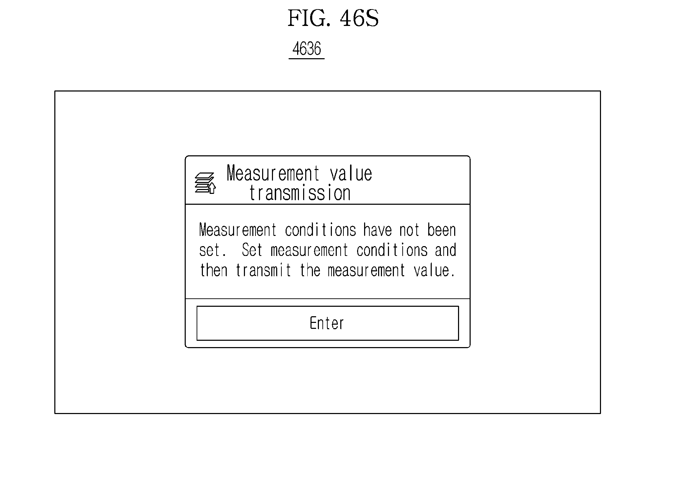

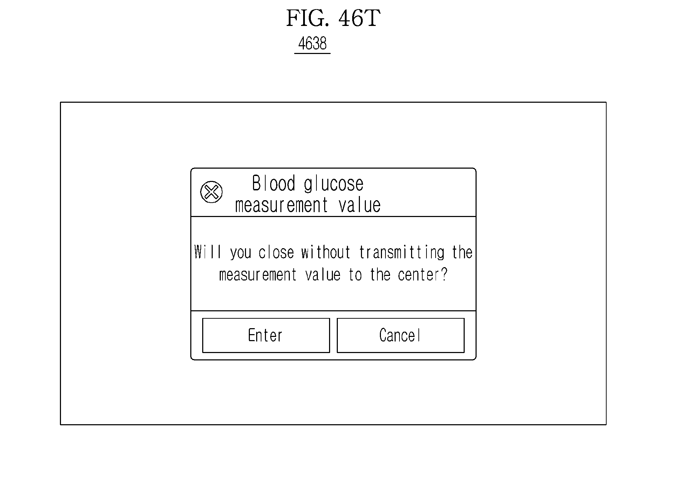

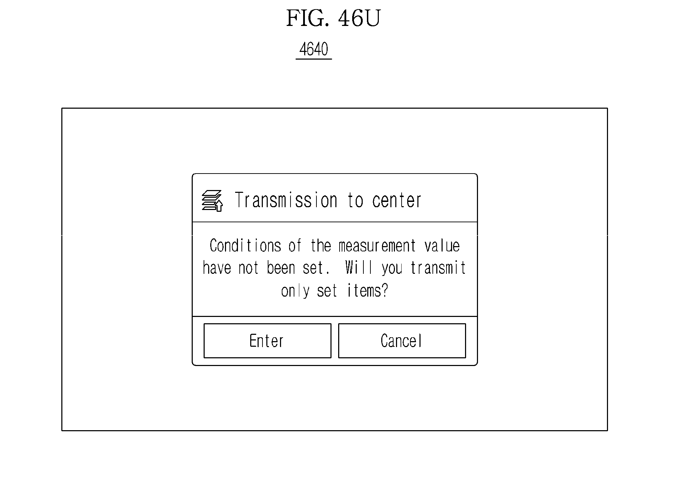

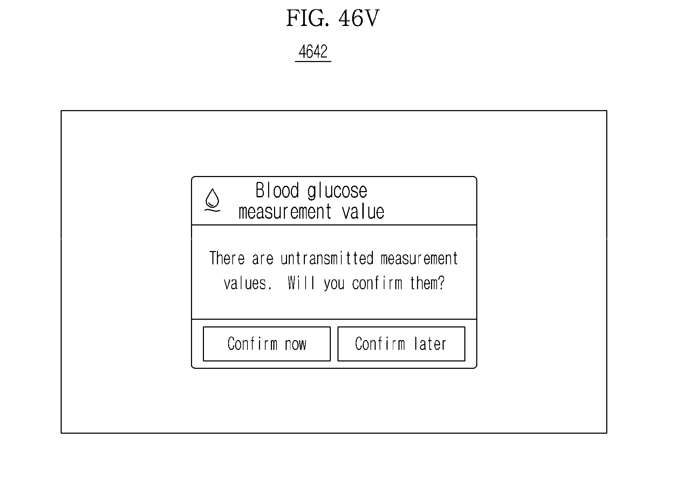

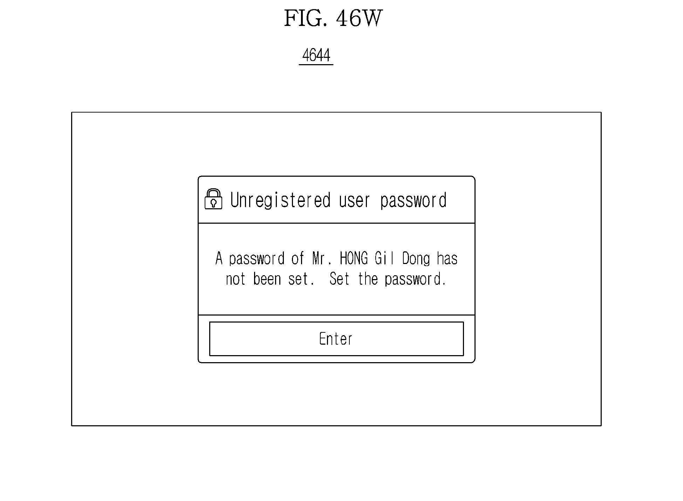

FIGS. 46A to 46W show health care notification views according to an exemplary embodiment.

In this exemplary embodiment, at least one selected from among health care notification views 4600 to 4644 may be provided as, without being limited to, a popup view, an overlaid view, a picture-in-picture (PIP) view or a picture-out-picture (POP) view on at least one of the views shown in the above-mentioned drawings (FIGS. 2A to 45B). Also, one or more health care notification views 4600 to 4644 may be provided in the views shown in the above-mentioned drawings (FIGS. 2A to 45B). Also, information included in the health care notification views is not limited to test or graphs. Such information may be provided together with the above-mentioned drawings (FIGS. 2A to 45B) through sound and various sensors.

In this exemplary embodiment, if a predetermined time elapses in the above-mentioned views (FIGS. 2A to 45B), a screensaver may be operated. In this case, if information related to the health care notification views 4600 to 4644 is provided, the operation of the screensaver may be stopped, and at least one of the health care notification views 4600 to 4644 may be displayed. Meanwhile, during the operation of the screensaver, logout of the user may be automatically performed.

In this exemplary embodiment, the view 4600 is a view provided during login of the user in the main view (for example, 400). In this exemplary embodiment, the view 4602 is a view provided when advice of a doctor is received while the user uses the device 100. For example, the advice of the doctor in the view 4602 may be provided based on bio-information measurement values measured by the user. In this exemplary embodiment, the view 4604 is a view provided when a predetermined time elapses while measurement values are received from the bio-information measurement sensor 160 or the server 170. In this exemplary embodiment, the view 4606 is a view provided when the Internet is disconnected while the user uses the device 100. In this exemplary embodiment, the view 4608 is a view provided when the user incorrectly inputs a password. In this exemplary embodiment, the view 4610 is a view provided when a user password is transmitted to a mobile phone registered by the user. In this exemplary embodiment, the view 4612 is a view to confirm whether the user has measured bio-information according to a bio-information measurement schedule. In this exemplary embodiment, the view 4614 is a view to confirm whether dosage has been performed according to a dosage schedule. In this exemplary embodiment, the view 4616 is a view to provide advice received from a doctor. In this exemplary embodiment, the view 4618 is a view to request that the user set daily tasks. In this exemplary embodiment, the views 4620 and 4622 are views to notify the user of time to measure bio-information according to a bio-information measurement schedule. In the view 4620, the user may adjust time to measure bio-information through a user interface 4621. In this exemplary embodiment, the view 4624 is a view to confirm whether a bio-information measurement schedule is to be stored if the measurement schedule is added. In this exemplary embodiment, the views 4626 and 4628 are views to notify the user of dosage time according to a dosage schedule. In the view 4628, the user may adjust dosage time through a user interface 4627. In this exemplary embodiment, the view 4630 is a view to confirm whether a dosage schedule is to be deleted if there is a schedule set together with the dosage schedule. In this exemplary embodiment, the view 4632 is a view to confirm whether a dosage schedule is to be stored if the dosage schedule is added. In this exemplary embodiment, the view 4634 is a view to confirm measurement conditions of measurement values measured by the bio-information measurement sensor 160. In this exemplary embodiment, the view 4636 is a view to request that the user set measurement conditions of the bio-information measurement sensor 160 if the measurement conditions are not set. In this exemplary embodiment, the view 4638 is a view to confirm whether bio-information measurement values are to be transmitted to the server 170. In this exemplary embodiment, the view 4640 is a view to confirm whether items, measurement conditions of which have been set, of the bio-information measurement values are to be transmitted to the server 170. In this exemplary embodiment, the view 4642 is a view to confirm whether untransmitted bio-information measurement values are to be transmitted to the server 170 if untransmitted bio-information measurement values are present. In this exemplary embodiment, the view 4644 is a view to request that the patient or the administrator set a password if the individual patient password is not set.

The health care method of the health care device according to the exemplary embodiments may be embodied in a program command form which may be executed through various computer units and recorded in computer-readable media. The computer-readable media may contain program commands, data files, data structures, and combinations thereof. The program commands recorded in the medium may be specially designed for the exemplary embodiments. Alternatively, the program commands may be well-known by those skilled in computer software. The computer-readable media may include hardware devices specially configured to store and execute program commands. For example, magnetic media, such as a hard disk, a floppy disk and a magnetic tape, optical media, such as a CD-ROM and a DVD, a magneto-optical media, such as a floptical disk, a ROM, a RAM and a flash memory may be used as the computer-readable media. The program commands may include a machine language prepared by a compiler and a high-level language code prepared by an interpreter so as to be executed by a computer. The above-mentioned hardware devices may be configured to operate as one or more software modules to operate the exemplary embodiments and vice versa.

As is apparent from the above description, the health care device may provide information to motivate a patient so that the patient continuously measures bio-information and to efficiently perform health care. Also, the health care device may provide user interfaces through which a user may intuitively use the device. Also, the health care device may provide various views and sounds to encourage a user to use the health care device and to further motivate a patient who wishes to measure bio-information.

Although a few exemplary embodiments have been shown and described, it would be appreciated by those skilled in the art that changes may be made in these exemplary embodiments without departing from the principles and spirit of the invention, the scope of which is defined in the claims and their equivalents.

## Claims

1. A health care management method, comprising:
displaying, by a health care device, information on a health state of a patient related to a first measurement of first bio-information and a first measurement of second bio-information of the patient;
displaying information that requests the patient to obtain a second measurement of one of the first bio-information and the second bio-information based on a schedule of the patient in which the second measurement of the first bio-information and the second measurement of the second bio-information are to be obtained; and
displaying a bio-information measurement view to acquire the second measurement through measuring the one of the first bio-information and the second bio-information in response to an input to obtain the second measurement.

2. The health care management method according to claim 1, wherein the displaying information on the health state comprises:
determining an image that represents the patient based on at least one of the first measurement value of the first bio-information and the first measurement value of the second bio-information; and
displaying the determined image.

3. The health care management method according to claim 1, wherein the displaying information on the health state comprises:
identifying a section of a display of the health care device in which the first measurement value of the first bio-information is displayed; and
displaying information on the health state corresponding to the first measurement value of the first bio-information in the identified section.

4. The health care management method according to claim 1, further comprising:
Determining a motivation message to obtain the second measurement;
displaying the determined motivation message;
determining a plurality of motivation messages;
selecting the motivation message from among the plurality of motivation messages based on at least one of header information of the motivation message, a transmission mode of the motivation message, a sender of the motivation message, and a content of the motivation message;
receiving a new message; and
displaying the new message so that at least a portion of a region where the new message is displayed includes at least a portion of a region where the motivation message is displayed, wherein the motivation message comprises a plurality of motivation messages, and
the determining the motivation message comprises selecting at least one motivation message from among the plurality of motivation messages based on the health state of the patient.

5. The health care management method according to claim 1, further comprising:
displaying a user interface connected to an external device external to the health care device; and
providing information on the health state of the patient stored in the external in response to user input through the user interface.

6. The health care management method according to claim 1, further comprising:
displaying information on the schedule to measure at least one of the first bio-information and the second bio-information of the patient or a dosage schedule of the patient; and
displaying the schedule to measure the at least one of the first bio-information and the second bio-information of the patient or the dosage schedule of the patient in response to user input through a user interface,
wherein the displaying the schedule to measure the at least one of the first bio-information and the second bio-information of the patient or the dosage schedule comprises, if the schedule comprises a plurality of schedules, providing a visual effect to at least one of the schedules so that the schedules are displayed according to priority.

7. The health care management method according to claim 1, further comprising:
displaying information on a message related to the health state of the patient; and
displaying the message related to the health state of the patient in response to user input through a user interface.

8. The health care management method according to claim 1, further comprising:
receiving a motivation message to obtain the second measurement;
determining a voice to output the motivation message based on a sender of the motivation message; and
outputting the motivation message using the determined voice.

9. The health care management method according to claim 1, further comprising:
determining a sound to notify of time to obtain the second measurement according to the health state of the patient; and
outputting the determined sound.

10. The health care management method according to claim 1, further comprising:
determining a sound to be output to the patient according to the health state or the schedule; and
outputting the determined sound.

11. A health care device comprising:
a display that displays information on a health state of a patient related to a first measurement of first bio-information and a first measurement of second bio-information of a patient and information that requests the patient to obtain a second measurement of one of the first bio-information and the second bio-information based on a schedule of the patient in which the second measurement of the first bio-information and the second measurement of the second bio-information are to be obtained;
an input unit through which an input is received to obtain the second measurement; and
a processor that controls the display to display a bio-information measurement view to acquire the second measurement through measuring the one of the first bio-information and the second bio-information in response to the user input.

12. The health care device according to claim 11, wherein the display displays an image that represents the patient decided based on at least one of the first measurement value of the first bio-information and the first measurement value of the second bio-information, and wherein the processor determines a motivation message to obtain the second measurement and controls the display to display the determined motivation message, wherein the processor determines a plurality of motivation messages and selects the motivation message from among the plurality of messages based on of at least one of header information of the motivation message, a transmission mode of the motivation message, a sender of the motivation message, and a content of the motivation message.

13. The health care device according to claim 11, wherein
the display displays a user interface connected to an external device external to the health care device, and
the processor controls the display to display information on the health state of the patient stored in the external in response to user input through the user interface.

14. The health care device according to claim 11, wherein
the display displays information on the schedule to measure at least one of the first bio-information and the second bio-information of the patient or a dosage schedule of the patient, and
the processor controls the display to display the schedule to measure the at least one of the first bio-information and the second bio-information of the patient or the dosage schedule of the patient in response to user input through a user interface.

15. The health care device according to claim 11, wherein
the display displays information on a message to care for health of the patient, and
the processor controls the display to display the message to care for health of the patient in response to user input through a user interface,
wherein the processor determines a voice to output a motivation message to obtain the second measurement and outputs the motivation message using the determined voice,
and wherein the processor determines a sound to be output to the patient according to the health state or the schedule and outputs the determined sound.
